# FASCICULE DE BREVET EUROPEEN

(11) **EP 1 730 270 B1**
(45) Date de publication et mention de la délivrance du brevet: **30.03.2016**
(21) Numéro de dépôt: 05746926.4
(22) Date de dépôt: 30.03.2005
(51) Int. Cl.: A61K 38/00

(54) **GLYCOPEPTIDES DERIVES DE STRUCTURES PANCREATIQUES, ANTICORPS ET LEURS APPLICATIONS EN DIAGNOSTIC ET THERAPEUTIQUE**
AUS PANKREASSTRUKTUREN STAMMENDE GLYCOPEPTIDE, ANTIKÖRPER UND ANWENDUNGEN DAVON IN DER DIAGNOSTIK UND THERAPEUTIK
GLYCOPEPTIDES DERIVED FROM PANCREATIC STRUCTURES, ANTIBODIES AND APPLICATIONS THEREOF IN DIAGNOSTICS AND THERAPEUTICS

(30) Priorité: 31.03.2004 FR 0403378; 16.12.2004 FR 0413428
(43) Date de publication de la demande: 13.12.2006
(73) Titulaire: Université d'Aix-Marseille, 13007 Marseille (FR); I.N.S.E.R.M. Institut National de la Santé et de la Recherche Médicale, 75654 Paris Cedex 13 (FR)
(72) Inventeur: LOMBARDO, Dominique, F-13008 Marseille (FR); MAS, Eric, F-13010 Marseille (FR); SADOULET, Marie-Odile, F-13006 Marseille (FR); PANICOT-DUBOIS, Laurence, F-66080 Canohes (FR); BERNARD, Jean-Paul, F-13001 Marseille (FR)
(74) Mandataire: Gallois, Valérie
(86) Numéro de dépôt international: PCT/FR2005/000771
(87) Numéro de publication internationale: WO 2005/095594

(56) Documents cités:
- WO-A-94/20610
- PANICOT L ET AL: "The formation of the oncofetal J28 glycotope involves core-2 beta6-N-acetylglucosaminyltransferase and alpha3/4-fucosyltransferase activities." GLYCOBIOLOGY. SEP 1999, vol. 9, no. 9, septembre 1999 (1999-09), pages 935-946, XP001024028 ISSN: 0959-6658
- DATABASE PASCAL INIST, CNRS (CENTRE NATIONAL DE LA RECHERCHE SCIENTIFIQUE), VANDOEUVRE-LES-NANCY, FR; 1994, ROUDANI SAMIRA; LOMBARDO D, DIRROUDANI SAMIRA; LOMBARDO D, DIR: "Etude des isoformes de la lipase sels-biliaire dependant du pancreas humain: expression dans le pancreas chez l'adulte normal et le fetus et dans l'adinocarlinome pancreatique" XP002302174 extrait de DIALOG Database accession no. 96-0105471 -& ROUDANI SAMIRA; LOMBARDO D, DIR: "etude des isoformes de la lipase sels-biliaire dependant du pancreas humain: expression dans le pancreas chez l'adulte normal et le fetus et dans l'adinocarlinome pancreatique" 1994, INIST , XP002302172 page 89 - page 97 page 28; figure 35B
- DATABASE PASCAL INIST, CNRS (CENTRE NATIONAL DE LA RECHERCHE SCIENTIFIQUE), VANDOEUVRE-LES-NANCY, FR; 1995, MAS ERIC; LOMBARDO D, DIR: "Glycosylation de la lipase sels biliaires dependantes du pancreas: structures, fonctions et pathologies" XP002302175 extrait de DIALOG Database accession no. 96-0113664 -& MAS ERIC; LOMBARDO D, DIR: "Glycosylation de la lipase sels biliaires dependantes du pancreas: structures, fonctions et pathologies" 1995, INIST , XP002302173 page 111 - page 114
- MAS E ET AL: "The oncofetal J28 epitope involves fucosylated O-linked oligosaccharide structures of the fetoacinar pancreatic protein." GLYCOBIOLOGY. SEP 1997, vol. 7, no. 6, septembre 1997 (1997-09), pages 745-752, XP008036319 ISSN: 0959-6658
- LOMBARDO D: "Bile salt-dependent lipase: its pathophysiological implications" BIOCHIMICA AND BIOPHYSICA ACTA. MOLECULAR AND CELL BIOLOGY OF LIPIDS, ELSEVIER, AMSTERDAM, NL, vol. 1533, no. 1, 29 août 2001 (2001-08-29), pages 1-28, XP004301092 ISSN: 1388-1981
- REUE K ET AL: "cDNA cloning of carboxyl ester lipase from human pancreas reveals a unique proline-rich repeat unit." JOURNAL OF LIPID RESEARCH. FEB 1991, vol. 32, no. 2, février 1991 (1991-02), pages 267-276, XP002302171 ISSN: 0022-2275

### SEQUENCE LISTING

<110> Université de la Méditerranée INSERM (Institut National de la santé et de la recherche médicale
<120> Glycopeptides dérivés de structures pancréatiques, anticorps et
   leurs applications en diagnostic et thérapeutique
<130> B0339wo
<150> FR 04 03378
   <151> 2004-03-31
<150> FR 04 13428
   <151> 2004-12-16
<160> 14
<170> PatentIn version 3.1
<210> 1
   <211> 36
   <212> DNA
   <213> Homo sapiens
<400> 1
   ttcgtaagct tgcgaagctg ggcgccgtgt acagaa 36
<210> 2
   <211> 42
   <212> DNA
   <213> Homo sapiens
<400> 2
   tttcgtgaat tcacgctaaa acctaatgac tgcaggcatc tg 42
<210> 3
   <211> 33
   <212> DNA
   <213> Homo sapiens
<400> 3
   cgtctaaagc tttttgatgt ctacaccgag tcc 33
<210> 4
   <211> 27
   <212> DNA
   <213> Homo sapiens
<400> 4
   aaaaagaatt cggagaaaat aatgaat 27
<210> 5
   <211> 28
   <212> DNA
   <213> Homo sapiens
<400> 5
   aaaaagggcc cacaaagtca gacattat 28
<210> 6
   <211> 2350
   <212> DNA
   <213> Homo sapiens
<220>
   <221> CDS
   <222> (17)..(2251)
   <223>
<400> 6
   ggccacccag aggctg atg ctc acc atg ggg cgc ctg caa ctg gtt gtg ttg      52
                     Met Leu Thr Met Gly Arg Leu Gln Leu Val Val Leu
                     1               5                   10
   ggc ctc acc tgc tgc tgg gca gtg gcg agt gcc gcg aag ctg ggc gcc       100
   Gly Leu Thr Cys Cys Trp Ala Val Ala Ser Ala Ala Lys Leu Gly Ala
       15                  20                  25
   gtg tac aca gaa ggt ggg ttc gtg gaa ggc gtc aat aag aag ctc ggc      148
   Val Tyr Thr Glu Gly Gly Phe Val Glu Gly Val Asn Lys Lys Leu Gly
       30                  35                  40
   ctc ctg ggt gac tct gtg gac atc ttc aag ggc atc ccc ttc gca gct      196
   Leu Leu Gly Asp Ser Val Asp Ile Phe Lys Gly Ile Pro Phe Ala Ala
   45                  50                  55                  60
   ccc acc aag gcc ctg gaa aat cct cag cca cat cct ggc tgg caa ggg      244
   Pro Thr Lys Ala Leu Glu Asn Pro Gln Pro His Pro Gly Trp Gln Gly
                   65                  70                  75
   acc ctg aag gcc aag aac ttc aag aag aga tgc ctg cag gcc acc atc      292
   Thr Leu Lys Ala Lys Asn Phe Lys Lys Arg Cys Leu Gln Ala Thr Ile
               80                  85                  90
   acc cag gac agc acc tac ggg gat gaa gac tgc ctg tac ctc aac att      340
   Thr Gln Asp Ser Thr Tyr Gly Asp Glu Asp Cys Leu Tyr Leu Asn Ile
           95                  100                 105
   tgg gtg ccc cag ggc agg aag caa gtc tcc cgg gac ctg ccc gtt atg      388
   Trp Val Pro Gln Gly Arg Lys Gln Val Ser Arg Asp Leu Pro Val Met
       110                 115                 120
   atc tgg atc tat gga ggc gcc ttc ctc atg ggg tcc ggc cat ggg gcc      436
   Ile Trp Ile Tyr Gly Gly Ala Phe Leu Met Gly Ser Gly His Gly Ala
   125                 130                 135                 140
   aac ttc ctc aac aac tac ctg tat gac ggc gag gag atc gcc aca cgc      484
   Asn Phe Leu Asn Asn Tyr Leu Tyr Asp Gly Glu Glu Ile Ala Thr Arg
                   145                 150                 155
   gga aac gtc atc gtg gtc acc ttc aac tac cgt gtc ggc ccc ctt ggg      532
   Gly Asn Val Ile Val Val Thr Phe Asn Tyr Arg Val Gly Pro Leu Gly
               160                 165                 170
   ttc ctc agc act ggg gac gcc aat ctg cca ggt aac tat ggc ctt cgg      580
   Phe Leu Ser Thr Gly Asp Ala Asn Leu Pro Gly Asn Tyr Gly Leu Arg
           175                 180                 185
   gat cag cac atg gcc att gct tgg gtg aag agg aat atc gcg gcc ttc      628
   Asp Gln His Met Ala Ile Ala Trp Val Lys Arg Asn Ile Ala Ala Phe
       190                 195                 200
   ggg ggg gac ccc aac aac atc acg ctc ttc ggg gag tct gct gga ggt      676
   Gly Gly Asp Pro Asn Asn Ile Thr Leu Phe Gly Glu Ser Ala Gly Gly
   205                 210                 215                 220
   gcc agc gtc tct ctg cag acc ctc tcc ccc tac aac aag ggc ctc atc      724
   Ala Ser Val Ser Leu Gln Thr Leu Ser Pro Tyr Asn Lys Gly Leu Ile
                   225                 230                 235
   cgg cga gcc atc agc cag agc ggc gtg gcc ctg agt ccc tgg gtc atc      772
   Arg Arg Ala Ile Ser Gln Ser Gly Val Ala Leu Ser Pro Trp Val Ile
               240                 245                 250
   cag aaa aac cca ctc ttc tgg gcc aaa aag gtg gct gag aag gtg ggt      820
   Gln Lys Asn Pro Leu Phe Trp Ala Lys Lys Val Ala Glu Lys Val Gly
           255                 260                 265
   tgc cct gtg ggt gat gcc gcc agg atg gcc cag tgt ctg aag gtt act      868
   Cys Pro Val Gly Asp Ala Ala Arg Met Ala Gln Cys Leu Lys Val Thr
       270                 275                 280
   gat ccc cga gcc ctg acg ctg gcc tat aag gtg ccg ctg gca ggc ctg      916
   Asp Pro Arg Ala Leu Thr Leu Ala Tyr Lys Val Pro Leu Ala Gly Leu
   285                 290                 295                 300
   gag tac ccc atg ctg cac tat gtg ggc ttc gtc cct gtc att gat gga      964
   Glu Tyr Pro Met Leu His Tyr Val Gly Phe Val Pro Val Ile Asp Gly
   305 310 315
   gac ttc atc ccc gct gac ccg atc aac ctg tac gcc aac gcc gcc gac 1012
   Asp Phe Ile Pro Ala Asp Pro Ile Asn Leu Tyr Ala Asn Ala Ala Asp
   320 325 330
   atc gac tat ata gca ggc acc aac aac atg gac ggc cac atc ttc gcc 1060
   Ile Asp Tyr Ile Ala Gly Thr Asn Asn Met Asp Gly His Ile Phe Ala
   335 340 345
   agc atc gac atg cct gcc atc aac aag ggc aac aag aaa gtc acg gag 1108
   Ser Ile Asp Met Pro Ala Ile Asn Lys Gly Asn Lys Lys Val Thr Glu
   350 355 360
   gag gac ttc tac aag ctg gtc agt gag ttc aca atc acc aag ggg ctc 1156
   Glu Asp Phe Tyr Lys Leu Val Ser Glu Phe Thr Ile Thr Lys Gly Leu
   365 370 375 380
   aga ggc gcc aag acg acc ttt gat gtc tac acc gag tcc tgg gcc cag 1204
   Arg Gly Ala Lys Thr Thr Phe Asp Val Tyr Thr Glu Ser Trp Ala Gln
   385 390 395
   gac cca tcc cag gag aat aag aag aag act gtg gtg gac ttt gag acc 1252
   Asp Pro Ser Gln Glu Asn Lys Lys Lys Thr Val Val Asp Phe Glu Thr
   400 405 410
   gat gtc ctc ttc ctg gtg ccc acc gag att gcc cta gcc cag cac aga 1300
   Asp Val Leu Phe Leu Val Pro Thr Glu Ile Ala Leu Ala Gln His Arg
   415 420 425
   gcc aat gcc aag agt gcc aag acc tac gcc tac ctg ttt tcc cat ccc 1348
   Ala Asn Ala Lys Ser Ala Lys Thr Tyr Ala Tyr Leu Phe Ser His Pro
   430 435 440
   tct cgg atg ccc gtc tac ccc aaa tgg gtg ggg gcc gac cat gca gat 1396
   Ser Arg Met Pro Val Tyr Pro Lys Trp Val Gly Ala Asp His Ala Asp
   445 450 455 460
   gac att cag tac gtt ttc ggg aag ccc ttc gcc acc ccc acg ggc tac 1444
   Asp Ile Gln Tyr Val Phe Gly Lys Pro Phe Ala Thr Pro Thr Gly Tyr
   465 470 475
   cgg ccc caa gac agg aca gtc tct aag gcc atg atc gcc tac tgg acc 1492
   Arg Pro Gln Asp Arg Thr Val Ser Lys Ala Met Ile Ala Tyr Trp Thr
   480 485 490
   aac ttt gcc aaa aca ggg gac ccc aac atg ggc gac tcg gct gtg ccc 1540
   Asn Phe Ala Lys Thr Gly Asp Pro Asn Met Gly Asp Ser Ala Val Pro
   495 500 505
   aca cac tgg gaa ccc tac act acg gaa aac agc ggc tac ctg gag atc 1588
   Thr His Trp Glu Pro Tyr Thr Thr Glu Asn Ser Gly Tyr Leu Glu Ile
   510 515 520
   acc aag aag atg ggc agc agc tcc atg aag cgg agc ctg aga acc aac 1636
   Thr Lys Lys Met Gly Ser Ser Ser Met Lys Arg Ser Leu Arg Thr Asn
   525 530 535 540
   ttc ctg cgc tac tgg acc ctc acc tat ctg gcg ctg ccc aca gtg acc 1684
   Phe Leu Arg Tyr Trp Thr Leu Thr Tyr Leu Ala Leu Pro Thr Val Thr
   545 550 555
   gac cag gag gcc acc cct gtg ccc ccc aca ggg gac tcc gag gcc act 1732
   Asp Gln Glu Ala Thr Pro Val Pro Pro Thr Gly Asp Ser Glu Ala Thr
   560 565 570
   ccc gtg ccc ccc acg ggt gac tcc gag acc gcc ccc gtg ccg ccc acg 1780
   Pro Val Pro Pro Thr Gly Asp Ser Glu Thr Ala Pro Val Pro Pro Thr
   575 580 585
   ggt gac tcc ggg gcc ccc ccc gtg ccg ccc acg ggt gac tcc ggg gcc 1828
   Gly Asp Ser Gly Ala Pro Pro Val Pro Pro Thr Gly Asp Ser Gly Ala
   590 595 600
   ccc ccc gtg ccg ccc acg ggt gac tcc ggg gcc ccc ccc gtg ccg ccc 1876
   Pro Pro Val Pro Pro Thr Gly Asp Ser Gly Ala Pro Pro Val Pro Pro
   605 610 615 620
   acg ggt gac tcc ggg gcc ccc ccc gtg ccg ccc acg ggt gac tcc ggg 1924
   Thr Gly Asp Ser Gly Ala Pro Pro Val Pro Pro Thr Gly Asp Ser Gly
   625 630 635
   gcc ccc ccc gtg ccg ccc acg ggt gac tcc ggg gcc ccc ccc gtg ccg 1972
   Ala Pro Pro Val Pro Pro Thr Gly Asp Ser Gly Ala Pro Pro Val Pro
   640 645 650
   ccc acg ggt gac tcc ggc gcc ccc ccc gtg ccg ccc acg ggt gac gcc 2020
   Pro Thr Gly Asp Ser Gly Ala Pro Pro Val Pro Pro Thr Gly Asp Ala
   655 660 665
   ggg ccc ccc ccc gtg ccg ccc acg ggt gac tcc ggc gcc ccc ccc gtg 2068
   Gly Pro Pro Pro Val Pro Pro Thr Gly Asp Ser Gly Ala Pro Pro Val
   670 675 680
   ccg ccc acg ggt gac tcc ggg gcc ccc ccc gtg acc ccc acg ggt gac 2116
   Pro Pro Thr Gly Asp Ser Gly Ala Pro Pro Val Thr Pro Thr Gly Asp
   685 690 695 700
   tcc gag acc gcc ccc gtg ccg ccc acg ggt gac tcc ggg gcc ccc cct 2164
   Ser Glu Thr Ala Pro Val Pro Pro Thr Gly Asp Ser Gly Ala Pro Pro
   705 710 715
   gtg ccc ccc acg ggt gac tct gag gct gcc cct gtg ccc ccc aca gat 2212
   Val Pro Pro Thr Gly Asp Ser Glu Ala Ala Pro Val Pro Pro Thr Asp
   720 725 730
   gac tcc aag gaa gct cag atg cct gca gtc att agg ttt tagcgtccca 2261
   Asp Ser Lys Glu Ala Gln Met Pro Ala Val Ile Arg Phe
   735 740 745
   tgagccttgg tatcaagagg ccacaagagt gggaccccag gggctcccct cccatcttga 2321
   gctcttcctg aataaagcct catacccct 2350
<210> 7
   <211> 745
   <212> PRT
   <213> Homo sapiens
<400> 7
   Met Leu Thr Met Gly Arg Leu Gln Leu Val Val Leu Gly Leu Thr Cys
   1 5 10 15
   Cys Trp Ala Val Ala Ser Ala Ala Lys Leu Gly Ala Val Tyr Thr Glu
   20 25 30
   Gly Gly Phe Val Glu Gly Val Asn Lys Lys Leu Gly Leu Leu Gly Asp
   35 40 45
   Ser Val Asp Ile Phe Lys Gly Ile Pro Phe Ala Ala Pro Thr Lys Ala
   50 55 60
   Leu Glu Asn Pro Gln Pro His Pro Gly Trp Gln Gly Thr Leu Lys Ala
   65 70 75 80
   Lys Asn Phe Lys Lys Arg Cys Leu Gln Ala Thr Ile Thr Gln Asp Ser
   85 90 95
   Thr Tyr Gly Asp Glu Asp Cys Leu Tyr Leu Asn Ile Trp Val Pro Gln
   100 105 110
   Gly Arg Lys Gln Val Ser Arg Asp Leu Pro Val Met Ile Trp Ile Tyr
   115 120 125
   Gly Gly Ala Phe Leu Met Gly Ser Gly His Gly Ala Asn Phe Leu Asn
   130 135 140
   Asn Tyr Leu Tyr Asp Gly Glu Glu Ile Ala Thr Arg Gly Asn Val Ile
   145 150 155 160
   Val Val Thr Phe Asn Tyr Arg Val Gly Pro Leu Gly Phe Leu Ser Thr
   165 170 175
   Gly Asp Ala Asn Leu Pro Gly Asn Tyr Gly Leu Arg Asp Gln His Met
   180 185 190
   Ala Ile Ala Trp Val Lys Arg Asn Ile Ala Ala Phe Gly Gly Asp Pro
   195 200 205
   Asn Asn Ile Thr Leu Phe Gly Glu Ser Ala Gly Gly Ala Ser Val Ser
   210 215 220
   Leu Gln Thr Leu Ser Pro Tyr Asn Lys Gly Leu Ile Arg Arg Ala Ile
   225 230 235 240
   Ser Gln Ser Gly Val Ala Leu Ser Pro Trp Val Ile Gln Lys Asn Pro
   245 250 255
   Leu Phe Trp Ala Lys Lys Val Ala Glu Lys Val Gly Cys Pro Val Gly
   260 265 270
   Asp Ala Ala Arg Met Ala Gln Cys Leu Lys Val Thr Asp Pro Arg Ala
   275 280 285
   Leu Thr Leu Ala Tyr Lys Val Pro Leu Ala Gly Leu Glu Tyr Pro Met
   290 295 300
   Leu His Tyr Val Gly Phe Val Pro Val Ile Asp Gly Asp Phe Ile Pro
   305 310 315 320
   Ala Asp Pro Ile Asn Leu Tyr Ala Asn Ala Ala Asp Ile Asp Tyr Ile
   325 330 335
   Ala Gly Thr Asn Asn Met Asp Gly His Ile Phe Ala Ser Ile Asp Met
   340 345 350
   Pro Ala Ile Asn Lys Gly Asn Lys Lys Val Thr Glu Glu Asp Phe Tyr
   355 360 365
   Lys Leu Val Ser Glu Phe Thr Ile Thr Lys Gly Leu Arg Gly Ala Lys
   370 375 380
   Thr Thr Phe Asp Val Tyr Thr Glu Ser Trp Ala Gln Asp Pro Ser Gln
   385 390 395 400
   Glu Asn Lys Lys Lys Thr Val Val Asp Phe Glu Thr Asp Val Leu Phe
   405 410 415
   Leu Val Pro Thr Glu Ile Ala Leu Ala Gln His Arg Ala Asn Ala Lys
   420 425 430
   Ser Ala Lys Thr Tyr Ala Tyr Leu Phe Ser His Pro Ser Arg Met Pro
   435 440 445
   Val Tyr Pro Lys Trp Val Gly Ala Asp His Ala Asp Asp Ile Gln Tyr
   450 455 460
   Val Phe Gly Lys Pro Phe Ala Thr Pro Thr Gly Tyr Arg Pro Gln Asp
   465 470 475 480
   Arg Thr Val Ser Lys Ala Met Ile Ala Tyr Trp Thr Asn Phe Ala Lys
   485 490 495
   Thr Gly Asp Pro Asn Met Gly Asp Ser Ala Val Pro Thr His Trp Glu
   500 505 510
   Pro Tyr Thr Thr Glu Asn Ser Gly Tyr Leu Glu Ile Thr Lys Lys Met
   515 520 525
   Gly Ser Ser Ser Met Lys Arg Ser Leu Arg Thr Asn Phe Leu Arg Tyr
   530 535 540
   Trp Thr Leu Thr Tyr Leu Ala Leu Pro Thr Val Thr Asp Gln Glu Ala
   545 550 555 560
   Thr Pro Val Pro Pro Thr Gly Asp Ser Glu Ala Thr Pro Val Pro Pro
   565 570 575
   Thr Gly Asp Ser Glu Thr Ala Pro Val Pro Pro Thr Gly Asp Ser Gly
   580 585 590
   Ala Pro Pro Val Pro Pro Thr Gly Asp Ser Gly Ala Pro Pro Val Pro
   595 600 605
   Pro Thr Gly Asp Ser Gly Ala Pro Pro Val Pro Pro Thr Gly Asp Ser
   610 615 620
   Gly Ala Pro Pro Val Pro Pro Thr Gly Asp Ser Gly Ala Pro Pro Val
   625 630 635 640
   Pro Pro Thr Gly Asp Ser Gly Ala Pro Pro Val Pro Pro Thr Gly Asp
   645 650 655
   Ser Gly Ala Pro Pro Val Pro Pro Thr Gly Asp Ala Gly Pro Pro Pro
   660 665 670
   Val Pro Pro Thr Gly Asp Ser Gly Ala Pro Pro Val Pro Pro Thr Gly
   675 680 685
   Asp Ser Gly Ala Pro Pro Val Thr Pro Thr Gly Asp Ser Glu Thr Ala
   690 695 700
   Pro Val Pro Pro Thr Gly Asp Ser Gly Ala Pro Pro Val Pro Pro Thr
   705 710 715 720
   Gly Asp Ser Glu Ala Ala Pro Val Pro Pro Thr Asp Asp Ser Lys Glu
   725 730 735
   Ala Gln Met Pro Ala Val Ile Arg Phe
   740 745
<210> 8
   <211> 1083
   <212> DNA
   <213> Homo sapiens
<220>
   <221> CDS
   <222> (1)..(1077)
   <223>
<400> 8
   ttt gat gtc tac acc gag tcc tgg gcc cag gac cca tcc cag gag aat 48
   Phe Asp Val Tyr Thr Glu Ser Trp Ala Gln Asp Pro Ser Gln Glu Asn
   1 5 10 15
   aag aag aag act gtg gtg gac ttt gag acc gat gtc ctc ttc ctg gtg 96
   Lys Lys Lys Thr Val Val Asp Phe Glu Thr Asp Val Leu Phe Leu Val
   20 25 30
   ccc acc gag att gcc cta gcc cag cac aga gcc aat gcc aag agt gcc      144
   Pro Thr Glu Ile Ala Leu Ala Gln His Arg Ala Asn Ala Lys Ser Ala
           35                  40                  45
   aag acc tac gcc tac ctg ttt tcc cat ccc tct cgg atg ccc gtc tac      192
   Lys Thr Tyr Ala Tyr Leu Phe Ser His Pro Ser Arg Met Pro Val Tyr
       50                  55                  60
   ccc aaa tgg gtg ggg gcc gac cat gca gat gac att cag tac gtt ttc      240
   Pro Lys Trp Val Gly Ala Asp His Ala Asp Asp Ile Gln Tyr Val Phe
   65                  70                  75                  80
   ggg aag ccc ttc gcc acc ccc acg ggc tac cgg ccc caa gac agg aca      288
   Gly Lys Pro Phe Ala Thr Pro Thr Gly Tyr Arg Pro Gln Asp Arg Thr
                   85                  90                  95
   gtc tct aag gcc atg atc gcc tac tgg acc aac ttt gcc aaa aca ggg      336
   Val Ser Lys Ala Met Ile Ala Tyr Trp Thr Asn Phe Ala Lys Thr Gly
               100                 105                 110
   gac ccc aac atg ggc gac tcg gct gtg ccc aca cac tgg gaa ccc tac      384
   Asp Pro Asn Met Gly Asp Ser Ala Val Pro Thr His Trp Glu Pro Tyr
           115                 120                 125
   act acg gaa aac agc ggc tac ctg gag atc acc aag aag atg ggc agc      432
   Thr Thr Glu Asn Ser Gly Tyr Leu Glu Ile Thr Lys Lys Met Gly Ser
       130                 135                 140
   agc tcc atg aag cgg agc ctg aga acc aac ttc ctg cgc tac tgg acc      480
   Ser Ser Met Lys Arg Ser Leu Arg Thr Asn Phe Leu Arg Tyr Trp Thr
   145                 150                 155                 160
   ctc acc tat ctg gcg ctg ccc aca gtg acc gac cag gag gcc acc cct      528
   Leu Thr Tyr Leu Ala Leu Pro Thr Val Thr Asp Gln Glu Ala Thr Pro
                   165                 170                 175
   gtg ccc ccc aca ggg gac tcc gag gcc act ccc gtg ccc ccc acg ggt      576
   Val Pro Pro Thr Gly Asp Ser Glu Ala Thr Pro Val Pro Pro Thr Gly
               180                 185                 190
   gac tcc gag acc gcc ccc gtg ccg ccc acg ggt gac tcc ggg gcc ccc      624
   Asp Ser Glu Thr Ala Pro Val Pro Pro Thr Gly Asp Ser Gly Ala Pro
           195                 200                 205
   ccc gtg ccg ccc acg ggt gac tcc ggg gcc ccc ccc gtg ccg ccc acg      672
   Pro Val Pro Pro Thr Gly Asp Ser Gly Ala Pro Pro Val Pro Pro Thr
       210                 215                 220
   ggt gac tcc ggg gcc ccc ccc gtg ccg ccc acg ggt gac tcc ggg gcc      720
   Gly Asp Ser Gly Ala Pro Pro Val Pro Pro Thr Gly Asp Ser Gly Ala
   225                 230                 235                 240
   ccc ccc gtg ccg ccc acg ggt gac tcc ggg gcc ccc ccc gtg ccg ccc      768
   Pro Pro Val Pro Pro Thr Gly Asp Ser Gly Ala Pro Pro Val Pro Pro
                   245                 250                 255
   acg ggt gac tcc ggg gcc ccc ccc gtg ccg ccc acg ggt gac tcc ggc      816
   Thr Gly Asp Ser Gly Ala Pro Pro Val Pro Pro Thr Gly Asp Ser Gly
               260                 265                 270
   gcc ccc ccc gtg ccg ccc acg ggt gac gcc ggg ccc ccc ccc gtg ccg      864
   Ala Pro Pro Val Pro Pro Thr Gly Asp Ala Gly Pro Pro Pro Val Pro
           275                 280                 285
   ccc acg ggt gac tcc ggc gcc ccc ccc gtg ccg ccc acg ggt gac tcc      912
   Pro Thr Gly Asp Ser Gly Ala Pro Pro Val Pro Pro Thr Gly Asp Ser
       290                 295                 300
   ggg gcc ccc ccc gtg acc ccc acg ggt gac tcc gag acc gcc ccc gtg      960
   Gly Ala Pro Pro Val Thr Pro Thr Gly Asp Ser Glu Thr Ala Pro Val
   305                 310                 315                 320
   ccg ccc acg ggt gac tcc ggg gcc ccc cct gtg ccc ccc acg ggt gac      1008
   Pro Pro Thr Gly Asp Ser Gly Ala Pro Pro Val Pro Pro Thr Gly Asp
                   325                 330                 335
   tct gag gct gcc cct gtg ccc ccc aca gat gac tcc aag gaa gct cag      1056
   Ser Glu Ala Ala Pro Val Pro Pro Thr Asp Asp Ser Lys Glu Ala Gln
               340                 345                 350
   atg cct gca gtc att agg ttt tagcgt                                   1083
   Met Pro Ala Val Ile Arg Phe
           355
<210> 9
   <211> 359
   <212> PRT
   <213> Home sapins
<400> 9
   Phe Asp Val Tyr Thr Glu Ser Trp Ala Gln Asp Pro Ser Gln Glu Asn
   1               5                   10                  15
   Lys Lys Lys Thr Val Val Asp Phe Glu Thr Asp Val Leu Phe Leu Val
               20                  25                  30
   Pro Thr Glu Ile Ala Leu Ala Gln His Arg Ala Asn Ala Lys Ser Ala
           35                  40                  45
   Lys Thr Tyr Ala Tyr Leu Phe Ser His Pro Ser Arg Met Pro Val Tyr
       50                  55                  60
   Pro Lys Trp Val Gly Ala Asp His Ala Asp Asp Ile Gln Tyr Val Phe
   65                  70                  75                  80
   Gly Lys Pro Phe Ala Thr Pro Thr Gly Tyr Arg Pro Gln Asp Arg Thr
                   85                  90                  95
   Val Ser Lys Ala Met Ile Ala Tyr Trp Thr Asn Phe Ala Lys Thr Gly
               100                 105                 110
   Asp Pro Asn Met Gly Asp Ser Ala Val Pro Thr His Trp Glu Pro Tyr
           115                 120                 125
   Thr Thr Glu Asn Ser Gly Tyr Leu Glu Ile Thr Lys Lys Met Gly Ser
       130                 135                 140
   Ser Ser Met Lys Arg Ser Leu Arg Thr Asn Phe Leu Arg Tyr Trp Thr
   145                 150                 155                 160
   Leu Thr Tyr Leu Ala Leu Pro Thr Val Thr Asp Gln Glu Ala Thr Pro
   165 170 175
   Val Pro Pro Thr Gly Asp Ser Glu Ala Thr Pro Val Pro Pro Thr Gly
   180 185 190
   Asp Ser Glu Thr Ala Pro Val Pro Pro Thr Gly Asp Ser Gly Ala Pro
   195 200 205
   Pro Val Pro Pro Thr Gly Asp Ser Gly Ala Pro Pro Val Pro Pro Thr
   210 215 220
   Gly Asp Ser Gly Ala Pro Pro Val Pro Pro Thr Gly Asp Ser Gly Ala
   225 230 235 240
   Pro Pro Val Pro Pro Thr Gly Asp Ser Gly Ala Pro Pro Val Pro Pro
   245 250 255
   Thr Gly Asp Ser Gly Ala Pro Pro Val Pro Pro Thr Gly Asp Ser Gly
   260 265 270
   Ala Pro Pro Val Pro Pro Thr Gly Asp Ala Gly Pro Pro Pro Val Pro
   275 280 285
   Pro Thr Gly Asp Ser Gly Ala Pro Pro Val Pro Pro Thr Gly Asp Ser
   290 295 300
   Gly Ala Pro Pro Val Thr Pro Thr Gly Asp Ser Glu Thr Ala Pro Val
   305 310 315 320
   Pro Pro Thr Gly Asp Ser Gly Ala Pro Pro Val Pro Pro Thr Gly Asp
   325 330 335
   Ser Glu Ala Ala Pro Val Pro Pro Thr Asp Asp Ser Lys Glu Ala Gln
   340 345 350
   Met Pro Ala Val Ile Arg Phe
   355
<210> 10
   <211> 1839
   <212> DNA
   <213> Homo sapiens
<220>
   <221> CDS
   <222> (1)..(1836)
   <223>
<400> 10
   gcg aag ctg ggc gcc gtg tac aca gaa ggt ggg ttc gtg gaa ggc gtc      48
   Ala Lys Leu Gly Ala Val Tyr Thr Glu Gly Gly Phe Val Glu Gly Val
   1               5                   10                  15
   aat aag aag ctc ggc ctc ctg ggt gac tct gtg gac atc ttc aag ggc      96
   Asn Lys Lys Leu Gly Leu Leu Gly Asp Ser Val Asp Ile Phe Lys Gly
               20                  25                  30
   atc ccc ttc gca gct ccc acc aag gcc ctg gaa aat cct cag cca cat      144
   Ile Pro Phe Ala Ala Pro Thr Lys Ala Leu Glu Asn Pro Gln Pro His
           35                  40                  45
   cct ggc tgg caa ggg acc ctg aag gcc aag aac ttc aag aag aga tgc      192
   Pro Gly Trp Gln Gly Thr Leu Lys Ala Lys Asn Phe Lys Lys Arg Cys
       50                  55                  60
   ctg cag gcc acc atc acc cag gac agc acc tac ggg gat gaa gac tgc      240
   Leu Gln Ala Thr Ile Thr Gln Asp Ser Thr Tyr Gly Asp Glu Asp Cys
   65                  70                  75                  80
   ctg tac ctc aac att tgg gtg ccc cag ggc agg aag caa gtc tcc cgg      288
   Leu Tyr Leu Asn Ile Trp Val Pro Gln Gly Arg Lys Gln Val Ser Arg
                   85                  90                  95
   gac ctg ccc gtt atg atc tgg atc tat gga ggc gcc ttc ctc atg ggg      336
   Asp Leu Pro Val Met Ile Trp Ile Tyr Gly Gly Ala Phe Leu Met Gly
               100                 105                 110
   tcc ggc cat ggg gcc aac ttc ctc aac aac tac ctg tat gac ggc gag      384
   Ser Gly His Gly Ala Asn Phe Leu Asn Asn Tyr Leu Tyr Asp Gly Glu
           115                 120                 125
   gag atc gcc aca cgc gga aac gtc atc gtg gtc acc ttc aac tac cgt      432
   Glu Ile Ala Thr Arg Gly Asn Val Ile Val Val Thr Phe Asn Tyr Arg
       130                 135                 140
   gtc ggc ccc ctt ggg ttc ctc agc act ggg gac gcc aat ctg cca ggt      480
   Val Gly Pro Leu Gly Phe Leu Ser Thr Gly Asp Ala Asn Leu Pro Gly
   145                 150                 155                 160
   aac tat ggc ctt cgg gat cag cac atg gcc att gct tgg gtg aag agg      528
   Asn Tyr Gly Leu Arg Asp Gln His Met Ala Ile Ala Trp Val Lys Arg
                   165                 170                 175
   aat atc gcg gcc ttc ggg ggg gac ccc aac aac atc acg ctc ttc ggg      576
   Asn Ile Ala Ala Phe Gly Gly Asp Pro Asn Asn Ile Thr Leu Phe Gly
               180                 185                 190
   gag tct gct gga ggt gcc agc gtc tct ctg cag act ctc tcc ccc tac      624
   Glu Ser Ala Gly Gly Ala Ser Val Ser Leu Gln Thr Leu Ser Pro Tyr
           195                 200                 205
   aac aag ggc ctc atc cgg cga gcc atc agc cag agc ggc gtg gcc ctg      672
   Asn Lys Gly Leu Ile Arg Arg Ala Ile Ser Gln Ser Gly Val Ala Leu
       210                 215                 220
   agt ccc tgg gtc atc cag aaa aac cca ctc ttc tgg gcc aaa aag gtg      720
   Ser Pro Trp Val Ile Gln Lys Asn Pro Leu Phe Trp Ala Lys Lys Val
   225                 230                 235                 240
   gct gag aag gtg ggt tgc cct gtg ggt gat gcc gcc agg atg gcc cag      768
   Ala Glu Lys Val Gly Cys Pro Val Gly Asp Ala Ala Arg Met Ala Gln
                   245                 250                 255
   tgt ctg aag gtt act gat ccc cga gcc ctg acg ctg gcc tat aag gtg      816
   Cys Leu Lys Val Thr Asp Pro Arg Ala Leu Thr Leu Ala Tyr Lys Val
                   260                     265                     270
   ccg ctg gca ggc ctg gag tac ccc atg ctg cac tat gtg ggc ttc gtc      864
   Pro Leu Ala Gly Leu Glu Tyr Pro Met Leu His Tyr Val Gly Phe Val
           275                     280                     285
   cct gtc att gat gga gac ttc atc ccc gct gac ccg atc aac ctg tac      912
   Pro Val Ile Asp Gly Asp Phe Ile Pro Ala Asp Pro Ile Asn Leu Tyr
       290                     295                     300
   gcc aac gcc gcc gac atc gac tat ata gca ggc acc aac aac atg gac      960
   Ala Asn Ala Ala Asp Ile Asp Tyr Ile Ala Gly Thr Asn Asn Met Asp
   305                     310                     315                     320
   ggc cac atc ttc gcc agc atc gac atg cct gcc atc aac aag ggc aac     1008
   Gly His Ile Phe Ala Ser Ile Asp Met Pro Ala Ile Asn Lys Gly Asn
                   325                     330                     335
   aag aaa gtc acg gag gag gac ttc tac aag ctg gtc agt gag ttc aca     1056
   Lys Lys Val Thr Glu Glu Asp Phe Tyr Lys Leu Val Ser Glu Phe Thr
               340                     345                     350
   atc acc aag ggg ctc aga ggc gcc aag acg acc ttt gat gtc tac acc     1104
   Ile Thr Lys Gly Leu Arg Gly Ala Lys Thr Thr Phe Asp Val Tyr Thr
           355                     360                     365
   gag tcc tgg gcc cag gac cca tcc cag gag aat aag aag aag act gtg     1152
   Glu Ser Trp Ala Gln Asp Pro Ser Gln Glu Asn Lys Lys Lys Thr Val
       370                     375                     380
   gtg gac ttt gag acc gat gtc ctc ttc ctg gtg ccc acc gag att gcc     1200
   Val Asp Phe Glu Thr Asp Val Leu Phe Leu Val Pro Thr Glu Ile Ala
   385                     390                     395                     400
   cta gcc cag cac aga gcc aat gcc aag agt gcc aag acc tac gcc tac     1248
   Leu Ala Gln His Arg Ala Asn Ala Lys Ser Ala Lys Thr Tyr Ala Tyr
                   405                     410                     415
   ctg ttt tcc cat ccc tct cgg atg ccc gtc tac ccc aaa tgg gtg ggg     1296
   Leu Phe Ser His Pro Ser Arg Met Pro Val Tyr Pro Lys Trp Val Gly
               420                     425                     430
   gcc gac cat gca gat gac att cag tac gtt ttc ggg aag ccc ttc gcc     1344
   Ala Asp His Ala Asp Asp Ile Gln Tyr Val Phe Gly Lys Pro Phe Ala
           435                     440                     445
   acc ccc acg ggc tac cgg ccc caa gac agg aca gtc tct aag gcc atg     1392
   Thr Pro Thr Gly Tyr Arg Pro Gln Asp Arg Thr Val Ser Lys Ala Met
       450                     455                     460
   atc gcc tac tgg acc aac ttt gcc aaa aca ggg gac ccc aac atg ggc     1440
   Ile Ala Tyr Trp Thr Asn Phe Ala Lys Thr Gly Asp Pro Asn Met Gly
   465                     470                     475                     480
   gac tcg gct gtg ccc aca cac tgg gaa ccc tac act acg gaa aac agc     1488
   Asp Ser Ala Val Pro Thr His Trp Glu Pro Tyr Thr Thr Glu Asn Ser
                   485                     490                     495
   ggc tac ctg gag atc acc aag aag atg ggc agc agc tcc atg aag cgg     1536
   Gly Tyr Leu Glu Ile Thr Lys Lys Met Gly Ser Ser Ser Met Lys Arg
               500                     505                     510
   agc ctg aga acc aac ttc ctg cgc tac tgg acc ctc acc tat ctg gcg     1584
   Ser Leu Arg Thr Asn Phe Leu Arg Tyr Trp Thr Leu Thr Tyr Leu Ala
           515                     520                     525
   ctg ccc aca gtg acc gac cag gag gcc acc cct gtg ccc ccc aca ggg     1632
   Leu Pro Thr Val Thr Asp Gln Glu Ala Thr Pro Val Pro Pro Thr Gly
       530                     535                     540
   gac tcc gag gcc act ccc gtg ccc ccc acg ggt gac tcc gag acc gcc      1680
   Asp Ser Glu Ala Thr Pro Val Pro Pro Thr Gly Asp Ser Glu Thr Ala
   545                 550                 555                 560
   ccc gtg ccg ccc acg ggc gac tcc ggg gcc ccc ccc gtg ccg ccc acg      1728
   Pro Val Pro Pro Thr Gly Asp Ser Gly Ala Pro Pro Val Pro Pro Thr
                   565                 570                 575
   ggt gac tcc ggg gcc ccc cct gtg ccc ccc acg ggt gac tct gag gct      1776
   Gly Asp Ser Gly Ala Pro Pro Val Pro Pro Thr Gly Asp Ser Glu Ala
               580                 585                 590
   gcc cct gtg ccc ccc aca ggt gac tcc aag gaa gct cag atg cct gca      1824
   Ala Pro Val Pro Pro Thr Gly Asp Ser Lys Glu Ala Gln Met Pro Ala
           595                 600                 605
   gtc att agg ttt tag                                                  1839
   Val Ile Arg Phe
       610
<210> 11
   <211> 612
   <212> PRT
   <213> Homo sapiens
<400> 11
   Ala Lys Leu Gly Ala Val Tyr Thr Glu Gly Gly Phe Val Glu Gly Val
   1               5                   10                  15
   Asn Lys Lys Leu Gly Leu Leu Gly Asp Ser Val Asp Ile Phe Lys Gly
               20                  25                  30
   Ile Pro Phe Ala Ala Pro Thr Lys Ala Leu Glu Asn Pro Gln Pro His
           35                  40                  45
   Pro Gly Trp Gln Gly Thr Leu Lys Ala Lys Asn Phe Lys Lys Arg Cys
       50                  55                  60
   Leu Gln Ala Thr Ile Thr Gln Asp Ser Thr Tyr Gly Asp Glu Asp Cys
   65                  70                  75                  80
   Leu Tyr Leu Asn Ile Trp Val Pro Gln Gly Arg Lys Gln Val Ser Arg
                   85                  90                  95
   Asp Leu Pro Val Met Ile Trp Ile Tyr Gly Gly Ala Phe Leu Met Gly
               100                 105                 110
   Ser Gly His Gly Ala Asn Phe Leu Asn Asn Tyr Leu Tyr Asp Gly Glu
           115                 120                 125
   Glu Ile Ala Thr Arg Gly Asn Val Ile Val Val Thr Phe Asn Tyr Arg
       130                 135                 140
   Val Gly Pro Leu Gly Phe Leu Ser Thr Gly Asp Ala Asn Leu Pro Gly
   145 150 155 160
   Asn Tyr Gly Leu Arg Asp Gln His Met Ala Ile Ala Trp Val Lys Arg
   165 170 175
   Asn Ile Ala Ala Phe Gly Gly Asp Pro Asn Asn Ile Thr Leu Phe Gly
   180 185 190
   Glu Ser Ala Gly Gly Ala Ser Val Ser Leu Gln Thr Leu Ser Pro Tyr
   195 200 205
   Asn Lys Gly Leu Ile Arg Arg Ala Ile Ser Gln Ser Gly Val Ala Leu
   210 215 220
   Ser Pro Trp Val Ile Gln Lys Asn Pro Leu Phe Trp Ala Lys Lys Val
   225 230 235 240
   Ala Glu Lys Val Gly Cys Pro Val Gly Asp Ala Ala Arg Met Ala Gln
   245 250 255
   Cys Leu Lys Val Thr Asp Pro Arg Ala Leu Thr Leu Ala Tyr Lys Val
   260 265 270
   Pro Leu Ala Gly Leu Glu Tyr Pro Met Leu His Tyr Val Gly Phe Val
   275 280 285
   Pro Val Ile Asp Gly Asp Phe Ile Pro Ala Asp Pro Ile Asn Leu Tyr
   290 295 300
   Ala Asn Ala Ala Asp Ile Asp Tyr Ile Ala Gly Thr Asn Asn Met Asp
   305 310 315 320
   Gly His Ile Phe Ala Ser Ile Asp Met Pro Ala Ile Asn Lys Gly Asn
   325 330 335
   Lys Lys Val Thr Glu Glu Asp Phe Tyr Lys Leu Val Ser Glu Phe Thr
   340 345 350
   Ile Thr Lys Gly Leu Arg Gly Ala Lys Thr Thr Phe Asp Val Tyr Thr
   355 360 365
   Glu Ser Trp Ala Gln Asp Pro Ser Gln Glu Asn Lys Lys Lys Thr Val
   370 375 380
   Val Asp Phe Glu Thr Asp Val Leu Phe Leu Val Pro Thr Glu Ile Ala
   385 390 395 400
   Leu Ala Gln His Arg Ala Asn Ala Lys Ser Ala Lys Thr Tyr Ala Tyr
   405 410 415
   Leu Phe Ser His Pro Ser Arg Met Pro Val Tyr Pro Lys Trp Val Gly
               420                 425                 430
   Ala Asp His Ala Asp Asp Ile Gln Tyr Val Phe Gly Lys Pro Phe Ala
           435                 440                 445
   Thr Pro Thr Gly Tyr Arg Pro Gln Asp Arg Thr Val Ser Lys Ala Met
       450                 455                 460
   Ile Ala Tyr Trp Thr Asn Phe Ala Lys Thr Gly Asp Pro Asn Met Gly
   465                 470                 475                 480
   Asp Ser Ala Val Pro Thr His Trp Glu Pro Tyr Thr Thr Glu Asn Ser
                   485                 490                 495
   Gly Tyr Leu Glu Ile Thr Lys Lys Met Gly Ser Ser Ser Met Lys Arg
               500                 505                 510
   Ser Leu Arg Thr Asn Phe Leu Arg Tyr Trp Thr Leu Thr Tyr Leu Ala
           515                 520                 525
   Leu Pro Thr Val Thr Asp Gln Glu Ala Thr Pro Val Pro Pro Thr Gly
       530                 535                 540
   Asp Ser Glu Ala Thr Pro Val Pro Pro Thr Gly Asp Ser Glu Thr Ala
   545                 550                 555                 560
   Pro Val Pro Pro Thr Gly Asp Ser Gly Ala Pro Pro Val Pro Pro Thr
                   565                 570                 575
   Gly Asp Ser Gly Ala Pro Pro Val Pro Pro Thr Gly Asp Ser Glu Ala
               580                 585                 590
   Ala Pro Val Pro Pro Thr Gly Asp Ser Lys Glu Ala Gln Met Pro Ala
           595                 600                 605
   Val Ile Arg Phe
       610
<210> 12
   <211> 750
   <212> DNA
   <213> Homo sapiens
<220>
   <221> CDS
   <222> (1)..(747)
   <223>
<400> 12
   ttt gat gtc tac acc gag tcc tgg gcc cag gac cca tcc cag gag aat      48
   Phe Asp Val Tyr Thr Glu Ser Trp Ala Gln Asp Pro Ser Gln Glu Asn
   1               5                   10                  15
   aag aag aag act gtg gtg gac ttt gag acc gat gtc ctc ttc ctg gtg      96
   Lys Lys Lys Thr Val Val Asp Phe Glu Thr Asp Val Leu Phe Leu Val
               20                  25                  30
   ccc acc gag att gcc cta gcc cag cac aga gcc aat gcc aag agt gcc      144
   Pro Thr Glu Ile Ala Leu Ala Gln His Arg Ala Asn Ala Lys Ser Ala
           35                  40                  45
   aag acc tac gcc tac ctg ttt tcc cat ccc tct cgg atg ccc gtc tac      192
   Lys Thr Tyr Ala Tyr Leu Phe Ser His Pro Ser Arg Met Pro Val Tyr
       50                  55                  60
   ccc aaa tgg gtg ggg gcc gac cat gca gat gac att cag tac gtt ttc      240
   Pro Lys Trp Val Gly Ala Asp His Ala Asp Asp Ile Gln Tyr Val Phe
   65                  70                  75                  80
   ggg aag ccc ttc gcc acc ccc acg ggc tac cgg ccc caa gac agg aca      288
   Gly Lys Pro Phe Ala Thr Pro Thr Gly Tyr Arg Pro Gln Asp Arg Thr
                   85                  90                  95
   gtc tct aag gcc atg atc gcc tac tgg acc aac ttt gcc aaa aca ggg      336
   Val Ser Lys Ala Met Ile Ala Tyr Trp Thr Asn Phe Ala Lys Thr Gly
               100                 105                 110
   gac ccc aac atg ggc gac tcg gct gtg ccc aca cac tgg gaa ccc tac      384
   Asp Pro Asn Met Gly Asp Ser Ala Val Pro Thr His Trp Glu Pro Tyr
           115                 120                 125
   act acg gaa aac agc ggc tac ctg gag atc acc aag aag atg ggc agc      432
   Thr Thr Glu Asn Ser Gly Tyr Leu Glu Ile Thr Lys Lys Met Gly Ser
       130                 135                 140
   agc tcc atg aag cgg agc ctg aga acc aac ttc ctg cgc tac tgg acc      480
   Ser Ser Met Lys Arg Ser Leu Arg Thr Asn Phe Leu Arg Tyr Trp Thr
   145                 150                 155                 160
   ctc acc tat ctg gcg ctg ccc aca gtg acc gac cag gag gcc acc cct      528
   Leu Thr Tyr Leu Ala Leu Pro Thr Val Thr Asp Gln Glu Ala Thr Pro
                   165                 170                 175
   gtg ccc ccc aca ggg gac tcc gag gcc act ccc gtg ccc ccc acg ggt      576
   Val Pro Pro Thr Gly Asp Ser Glu Ala Thr Pro Val Pro Pro Thr Gly
               180                 185                 190
   gac tcc gag acc gcc ccc gtg ccg ccc acg ggc gac tcc ggg gcc ccc      624
   Asp Ser Glu Thr Ala Pro Val Pro Pro Thr Gly Asp Ser Gly Ala Pro
           195                 200                 205
   ccc gtg ccg ccc acg ggt gac tcc ggg gcc ccc cct gtg ccc ccc acg      672
   Pro Val Pro Pro Thr Gly Asp Ser Gly Ala Pro Pro Val Pro Pro Thr
       210                 215                 220
   ggt gac tct gag gct gcc cct gtg ccc ccc aca ggt gac tcc aag gaa      720
   Gly Asp Ser Glu Ala Ala Pro Val Pro Pro Thr Gly Asp Ser Lys Glu
   225                 230                 235                 240
   gct cag atg cct gca gtc att agg ttt tag                              750
   Ala Gln Met Pro Ala Val Ile Arg Phe
                   245
<210> 13
   <211> 249
   <212> PRT
   <213> Homo sapiens
<400> 13
   Phe Asp Val Tyr Thr Glu Ser Trp Ala Gln Asp Pro Ser Gln Glu Asn
   1               5                   10                  15
   Lys Lys Lys Thr Val Val Asp Phe Glu Thr Asp Val Leu Phe Leu Val
               20                  25                  30
   Pro Thr Glu Ile Ala Leu Ala Gln His Arg Ala Asn Ala Lys Ser Ala
           35                  40                  45
   Lys Thr Tyr Ala Tyr Leu Phe Ser His Pro Ser Arg Met Pro Val Tyr
       50                  55                  60
   Pro Lys Trp Val Gly Ala Asp His Ala Asp Asp Ile Gln Tyr Val Phe
   65                  70                  75                  80
   Gly Lys Pro Phe Ala Thr Pro Thr Gly Tyr Arg Pro Gln Asp Arg Thr
                   85                  90                  95
   Val Ser Lys Ala Met Ile Ala Tyr Trp Thr Asn Phe Ala Lys Thr Gly
               100                 105                 110
   Asp Pro Asn Met Gly Asp Ser Ala Val Pro Thr His Trp Glu Pro Tyr
           115                 120                 125
   Thr Thr Glu Asn Ser Gly Tyr Leu Glu Ile Thr Lys Lys Met Gly Ser
       130                 135                 140
   Ser Ser Met Lys Arg Ser Leu Arg Thr Asn Phe Leu Arg Tyr Trp Thr
   145                 150                 155                 160
   Leu Thr Tyr Leu Ala Leu Pro Thr Val Thr Asp Gln Glu Ala Thr Pro
                   165                 170                 175
   Val Pro Pro Thr Gly Asp Ser Glu Ala Thr Pro Val Pro Pro Thr Gly
               180                 185                 190
   Asp Ser Glu Thr Ala Pro Val Pro Pro Thr Gly Asp Ser Gly Ala Pro
           195                 200                 205
   Pro Val Pro Pro Thr Gly Asp Ser Gly Ala Pro Pro Val Pro Pro Thr
       210                 215                 220
   Gly Asp Ser Glu Ala Ala Pro Val Pro Pro Thr Gly Asp Ser Lys Glu
   225                 230                 235                 240
   Ala Gln Met Pro Ala Val Ile Arg Phe
                   245
<210> 14
   <211> 11
   <212> PRT
   <213> Homo sapiens
<220>
   <221> MISC_FEATURE
   <222> (3)..(3)
   <223> Xaa is Glu or Gly
<220>
   <221> MISC_FEATURE
   <222> (11)..(11)
   <223> xaa is Glu or Gly
<400> 14
   Asp Ser Xaa Ala Pro Pro Val Pro Pro Thr Xaa
   1               5                   10

## Description

La présente invention concerne des glycopeptides dérivés de structures pancréatiques, des anticorps et leurs applications en diagnostic et thérapeutique.

La description décrit plus particulièrement l'obtention de glycopeptides C-terminaux naturels de la BSDL et/ou de la FAPP portant divers épitopes glycosylés (seul ou combinés) reconnus par des anticorps induits chez un patient atteint d'un diabète de type I, et purifiés à partir de fluides biologiques d'origine humaine ou animale et desdits glycopeptides recombinants produits par génie biologique à partir de lignées cellulaires équipées avec les ADNc codant pour les peptides C-terminaux de la FAPP et/ou de la BSDL et avec les ADNc codant pour les différentes glycosyltransférases nécessaires à la structuration desdits glycopeptides. L'invention est relative à l'obtention d'anticorps monoclonaux reconnaissant les glycopeptides C-terminaux recombinants et/ou naturels de la BSDL et/ou de la FAPP ainsi que l'utilisation desdits glycopeptides et desdits anticorps monoclonaux comme agents immunogènes, agents de diagnostic et agents thérapeutiques utilisables pour le diagnostic ou le traitement curatif ou préventif des pathologies pancréatiques telles que le diabète et les cancers du pancréas exocrine ou de toutes autres pathologies ainsi que dans des protocoles curatifs et préventifs desdites pathologies.

Le cancer du pancréas exocrine, qui représente plus de 20% des cancers du tractus digestif, est l'un des plus agressifs. En France par exemple, 4000 nouveaux cas sont diagnostiqués par an. Sa fréquence est d'ailleurs en nette augmentation dans de nombreuses régions du monde. Le taux de survie ne dépasse pas 20% à 1 an et 3% à 5 ans avec une moyenne de survie de 3 à 4 mois après le diagnostic. Il fait l'objet d'un diagnostic tardif et présente une évolution très rapide principalement due à la formation de métastases péritonéales et hépatiques. De plus, la localisation anatomique profonde de ce cancer, l'absence de marqueurs biologiques précoces sensibles et spécifiques ainsi que son caractère asymptomatique ont pour conséquence un diagnostic toujours tardif. Il n'existe actuellement aucune thérapie efficace des cancers du pancréas exocrine qui demeure insensible à la chimiothérapie et à la radiothérapie.

Il serait donc souhaitable de disposer de marqueurs spécifiques du cancer pancréatique voire d'autres pathologies pancréatiques pour en réaliser un diagnostic et de disposer de produits pour constituer des traitements efficaces.

Il a été précédemment démontré que la formation du glycotope J28 exprimé par les tissus pancréatiques tumoraux, impliquait des activités α3/4-fucosyltransférase et core-2 β6-N-acétylglucosaminyltransférase (Panicot et al., glycobiology, vol. 9 no. 9, 1999, 935-946). Il a également été montré que la lipase sels biliaires-dépendante (Bile Salt Dependent Lipase : BSDL) pouvait être impliquée dans des pathologies pancréatiques (Lombardo, Biochimica et Biophysica Acta 1533, 2001, 1-28).

Après de longues recherches, les demandeurs ont découvert avec étonnement des marqueurs spécifiques des pathologies pancréatiques, et notamment du cancer pancréatique, exprimés à la surface de la cellule tumorale pancréatique.

Ils ont découvert des anticorps circulants dirigés contre des structures antigéniques glycosylées des peptides C-terminaux de la lipase sels biliaires-dépendante (Bile Salt Dependent Lipase : BSDL), chez des patients diabétiques.

Ils ont aussi découvert que des anticorps mono et polyclonaux anti-épitopes glycosylés exprimés dans la partie C-terminale de la protéine foeto-acineuse du pancréas humain (FAPP) reconnaissaient spécifiquement des lignées cellulaires tumorales du pancréas humain, mais ne reconnaissaient pas des lignées cellulaires tumorales d'origine non-pancréatiques.

Ils ont en outre découvert que des anticorps mono et polyclonaux anti-épitopes glycosylés exprimés dans la partie C-terminale de la protéine foeto-acineuse du pancréas humain (FAPP) reconnaissaient spécifiquement le tissu tumoral du pancréas humain, mais ne reconnaissaient pas le tissu pancréatique normal.

Ils ont également découvert que les anticorps anti-épitopes glycosylés exprimés dans la partie C-terminale de la BSDL ou la FAPP peuvent détecter dans les urines des glycopeptides dérivées de la BSDL et de la FAPP. En particulier, les anticorps anti-épitopes glycosylés exprimés dans la partie C-terminale de la FAPP étaient capables de détecter dans les urines des glycopeptides permettant d'identifier un sujet atteint d'une pathologie du pancréas, en particulier du cancer du pancréas.

Ils ont donc plus particulièrement découvert que des composés de structure glycopeptidique dont la partie peptidique est basée sur les séquences répétées C-terminales de la BSDL, une enzyme lipolytique digestive présente dans les sécrétions du pancréas normal, ou sur les séquences répétées C-terminales de la FAPP (une forme oncofoetale de la BSDL) constituaient de tels marqueurs spécifiques de pathologies pancréatiques.

La BSDL et la FAPP comprennent en effet des séquences peptidiques répétées C-terminales de 11 acides aminés, comprenant une partie généralement invariante de 7 acides aminés de séquence Ala Pro Pro Val Pro Pro Thr et un site de glycosylation. Cette partie généralement invariante est encadrée de chaque côté par une Glycine souvent remplacée par un acide glutamique et comporte du côté N-terminal les acides aminés Asp et Ser.

Ils ont aussi découvert que des composés de structure glycopeptidique pouvant être préparés par expression et sécrétion par une cellule hôte, par exemple d'ovaire de Hamsters chinois (CHO), comprenant une construction génique incluant une molécule d'ADN codant pour une ou plusieurs séquences répétées du peptide C-terminal notamment recombinant de la BSDL, par exemple tout ou partie des 16 séquences répétées et comprenant aussi une construction génique telle une molécule d'ADN codant pour au moins une enzyme à activité transférase d'oses, notamment choisie parmi la Core 2 β(1-6) N-acétylglucosaminyltransférase, la fucosyltransférase FUT3 qui possède une activité α(1-3) et α(1-4) fucosyltransférase, ou la fucosyltransférase FUT7 qui possède seulement une activité α(1-3) fucosyltransférase, constituaient de tels marqueurs spécifiques du cancer pancréatique.

La présente invention concerne l'anticorps monoclonal selon les revendications 1 à 4, l'utilisation de cet anticorps selon les revendications 5 et 6, les méthodes de détection selon les revendications 7 à 10 et 12 à 15, le kit selon la revendication 11 et les compositions selon les revendications 16 à 33.

Toute information dans la présente description qui n'entre pas dans la portée des revendications est fournie uniquement à titre d'information.

La présente demande décrit un glycopeptide notamment recombinant, éventuellement isolé ou purifié, comprenant de 1 à 40 polypeptides répétés C-terminaux, composés de 11 acides aminés, de la BSDL ou de la FAPP, lesdits polypeptides étant glycosylés et portant des épitopes glycosylés donnant lieu à une réaction immunologique spécifique avec des anticorps induits chez un patient atteint par un diabète de type I et
- ou bien purifié à partir de fluides biologiques d'origine humaine ou animale
- ou bien recombinant et pouvant être produit par expression dans une cellule-hôte conventionnelle comprenant un matériel enzymatique nécessaire à l'amorçage d'une glycosylation, ladite cellule-hôte étant génétiquement modifiée de manière à comporter un gène codant pour ledit polypeptide et un gène codant pour une ou plusieurs enzymes choisies parmi les glycosyltransférases et notamment parmi la Core2 β(1-6) N-acétylglucosaminyltransférase (en abrégé C2GnT), l'α(1-3) galactosyltransférase, la fucosyltransférase 3 (en abrégé FUT3) et la fucosyltransférase 7 (en abrégé FUT7).

Dans d'autres conditions préférentielles, le glycopeptide ci-dessus est essentiellement constitué de 1 à 40 peptides répétés C-terminaux de 11 acides aminés de la BSDL ou de la FAPP glycosylés et notamment exclusivement constitué de tels peptides glycosylés. Ainsi, la présente description décrit donc des glycopeptides isolés, purifiés ou recombinants comprenant, ou consistant essentiellement en, des répétitions de la séquence peptidique répétée C-terminale de 11 acides aminés, de préférence: D-S-G/E-A-P-P-V-P-P-T-G/E (SEQ ID No 14). Ces glycopeptides peuvent comporter 1 à 40 répétitions. Selon un aspect préféré, ces glycopeptides comprennent 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 ou 40 répétitions. De préférence, les glycopeptides comprennent 1, 2, 3, 4, 5, 6, 7, 8, 9, 10, 11, 12, 13, 14, 15, 17, 18, 19, 20, 21, 22, 23, 24, 25, 26, 27, 28, 29, 30, 31, 32, 33, 34, 35, 36, 37, 38, 39 ou 40 répétitions. Selon un aspect particulier, les glycopeptides comprennent 1, 2, 3, 4, 5, 7, 8, 9, 10, 11, 12, 13, 14, 15, 17, 18, 19, 20, 21, 22, 23, 24, 25, 26, 27, 28, 29, 30, 31, 32, 33, 34, 35, 36, 37, 38, 39 ou 40 répétitions. Selon un aspect alternatif, les glycopeptides comprennent entre 1 et 15 répétitions (par ex., 1, 2, 3, 4, 5, 6, 7, 8, 9, 10, 11, 12, 13, 14, ou 15 répétitions), de préférence entre 2 et 10 répétitions (par ex., 2, 3, 4, 5, 6, 7, 8, 9, ou 10 répétitions). Selon un aspect additionnel, les glycopeptides comprennent entre 17 et 40 répétitions (par ex., 17, 18, 19, 20, 21, 22, 23, 24, 25, 26, 27, 28, 29, 30, 31, 32, 33, 34, 35, 36, 37, 38, 39 ou 40 répétitions). Dans des conditions préférentielles, le glycopeptide ci-dessus comprend de 1 à 40, de préférence de 4 à 25 et tout particulièrement de 6 à 16 polypeptides répétés C- terminaux. Selon un aspect particulier, le glycopeptide comprend, ou consiste essentiellement en, 6 répétitions.

De préférence, les glycopeptides décrits ici sont glycosylés par une ou plusieurs enzymes à activité transférase d'oses sélectionnées parmi le groupe constitué de la Core 2 P(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède seulement une activité α(1-3) fucosyltransférase. Selon un aspect particulier, les glycopeptides décrits ici ont été glycosylés par les enzymes C2GnT et FUT3. Selon un aspect alternatif, les glycopeptides décrits ici ont été glycosylés par les enzymes C2GnT et FUT7. La description considère également des glycopeptides qui ont été glycosylés par les enzymes C2GnT, FUT3 et FUT7. Selon un aspect préféré, les glycopeptides décrits ici ont en outre été glycosylés par l'α(1-3)galactosyltransférase (GT). Ainsi, lorsque le glycopeptide décrit ici est recombinant, la cellule hôte qui le produit comprend un gène codant pour ledit polypeptide et un gène codant pour une ou plusieurs enzymes choisies parmi les glycosyltransférases et notamment parmi C2GnT, FUT3 et FUT7. Selon un aspect préféré, cette cellule hôte comprend en outre un gène codant pour l'α(1-3)galactosyltransférase (GT).

Selon un aspect particulièrement préféré, la présente description décrit un glycopeptide recombinant, isolé ou purifié comprenant 1 à 40 répétitions de la séquence peptidique décrite dans SEQ ID No 14 et glycosylé par une ou plusieurs enzymes à activité transférase d'oses sélectionnées parmi le groupe constitué de la Core 2 β(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède une activité α(1-3) fucosyltransférase, ledit glycopeptide étant en outre glycosylé par l'α(1-3)galactosyltransférase (GT).

Par « glycopeptides isolés », on entend que ces composés sont séparés de leur environnement naturel. Ainsi, ces composés sont séparés de certains ou de tous les autres composants de leur environnement naturel.

Par « glycopeptides purifiés », on entend que ces composés sont enrichis dans un mélange d'au moins un ordre de grandeur, de préférence par au moins 2, 3, 4 ou 5 ordres de grandeur. Le terme « purifié » ne signifie pas forcément que les composés sont absolument purs. Ainsi, ces composés peuvent présenter 30%, 40%, 50%, 60%, 70%, 80%, 90% ou 100% de pureté. De préférence, cependant, ces composés ne conduisent qu'à des bandes uniques en électrophorèse sur gel de polyacrylamide.

Tout procédé de séparation et/ou de purification approprié pour les obtenir peut être utilisé. Selon un aspect particulier, les glycopeptides sont purifiées à partir d'un fluide biologique d'un sujet ou un animal. Le fluide biologique peut être sélectionné parmi le groupe constitué du sérum, de l'urine, des sucs pancréatiques et des sécrétions lactées. De préférence, le fluide biologique est l'urine. Selon un aspect préféré, le sujet est un patient susceptible d'être atteint ou atteint par une maladie pancréatique, de préférence d'un cancer du pancréas. Selon un autre aspect, les glycopeptides sont purifiés à partir d'une culture cellulaire. Selon un aspect additionnel, les glycopeptides sont purifiés à partir d'un prélèvement de tissu, de préférence un tissu pancréatique et notamment une tumeur du tissu pancréatique.

Dans la présente demande, le terme « glycotype » fait référence à des épitopes glycosylés. Les épitopes glycosylés peuvent mettre en jeu une ou plusieurs chaînes glycosylées. Les termes « mAb » et « Acm » font référence à un anticorps monoclonal. Le terme « Acp » fait référence à un anticorps polyclonal.

Selon un aspect particulier, la présente description décrit un glycopeptide tel que décrit ici et capable de donner lieu à une réaction immunologique spécifique avec un anticorps selon la présente invention. De préférence, le glycopeptide est capable de donner lieu, à une réaction immunologique spécifique avec un ou plusieurs anticorps choisis parmi J28 et 16D10. Facultativement, le glycopeptide sera capable de donner lieu à une réaction immunologique spécifique avec J28. Alternativement, il sera capable de donner lieu à une réaction immunologique spécifique avec 16D10.

Les cellules-hôtes classiquement utilisées en génie génétique sont naturellement dotées d'enzymes, par exemple l'α-3 N-acétylgalacto-saminyltransférase fixant une N-acétylgalactosamine généralement sur un résidu sérine ou thréonine d'un peptide. C'est ainsi que par exemple les cellules-hôtes largement utilisées comme C127, COS, CHO ou CHO-K1 sont munies de telles enzymes.

Dans la présente demande et dans ce qui suit, le terme « cellule hôte conventionnelle » désigne une cellule habituellement utilisée dans la production de glycopeptides, de préférence une cellule humaine ou une cellule animale telles que les cellules C127, COS, ou les cellules d'insecte telles les cellules d'ovaire de *Spodoptera frugiperda* et particulièrement une cellule CHO ou CHO-K1. Une telle cellule hôte conventionnelle acceptable peut notamment être choisie en fonction de son équipement génétique et de ses capacités à générer les divers épitopes glycosylés. Pour opérer ce choix, on peut opérer un dosage enzymatique pour déceler la présence notamment de l'α-3 N-acétylglucosaminyltransférase dans la cellule hôte que l'on souhaite utiliser.

Les glycosyltransférases utilisables peuvent être d'origine animale ou de préférence humaine.

Dans des conditions préférentielles, la cellule hôte conventionnelle génétiquement modifiée comprend au moins un gène codant pour ledit polypeptide et au moins un gène codant pour la Core2 β(1-6) N-acétylglucosaminyltransférase (C2GnT) et la fucosyltransférase 3 (FUT3).

Dans d'autres conditions préférentielles, notamment pour les applications dans le cancer du pancréas, la cellule hôte conventionnelle génétiquement modifiée comprend au moins un gène codant pour ledit polypeptide et au moins un gène codant pour la Core2 β(1-6) N-acétylglucosaminyltransférase (C2GnT), la fucosyltransférase 3 (FUT3) et l'α (1-3) galactosyltransférase (GT).

Dans encore d'autres conditions préférentielles, notamment pour les applications dans le diabète de type I, la cellule hôte conventionnelle génétiquement modifiée comprend au moins un gène codant pour ledit polypeptide et au moins un gène codant pour la glycosyltransférase Core2 β(1-6) N-acétylglucosaminyltransférase (C2GnT) et la fucosyltransférase 7 (FUT7).

Tout ou partie des peptides répétés C-terminaux peuvent être glycosylés. De préférence, la totalité des séquences répétées sera glycosylée, généralement 16 séquences, plus spécifiquement 6 séquences et notamment 2 séquences seront glycosylées.

Les glycosylations peuvent être dues à un ou plusieurs N-acétylglucosamine, N-acétylgalactosamine, acide sialique, glucose, galactose, fucose.

Dans encore d'autres conditions préférentielles, la taille d'un glycopeptide ci-dessus estimé par gel de polyacrylamide (SDS-PAGE) est avantageusement compris entre 20 et 120 kDa, de préférence compris entre 20 et 85 kDa, tout particulièrement compris entre 45 et 83 kDa.

On retient particulièrement les glycopeptides dénommés C2-F3-16R, C2-F3-GT-6R et tout particulièrement C2-F3-6R et C2-F7-16R. C2-F3-16R désigne un glycopeptide présentant 16 répétitions glycosylé par C2GnT et FUT3. C2-F3-GT-6R désigne un glycopeptide présentant 6 répétitions glycosylé par C2GnT, FUT3 et GT. C2-F3-6R désigne un glycopeptide présentant 6 répétitions glycosylé par C2GnT et FUT3. C2-F7-16R désigne un glycopeptide présentant 16 répétitions glycosylé par C2GnT et FUT7. De préférence, les glycopeptides sont sélectionnés parmi C2-F3-6R, C2-F3-GT-6R, C2-F7-6R, C2-F7-GT-6R, C2-F3-F7-6R, et C2-F3-F7-GT-6R.

Une lignée cellulaire produisant le glycopeptide C2-F7-16R a été déposée à la Collection Nationale de Culture de Microorganismes (CNCM) à Paris le 16 Mars 2004 sous le N° I-3189.

Comme le sait l'homme de l'art, un peptide peut subir quelques modifications structurales sans changer de fonction.

Il peut comporter une ou plusieurs modifications dans ses acides aminés telles que des délétions, substitutions, additions, ou fonctionnalisations telles qu'acylation d'acides aminés, dans la mesure où ces modifications n'en affectent pas les caractères immunologiques. Notamment, la ou les modifications peuvent être des remplacements par un acide aminé conservatif (c'est-à-dire présentant des caractéristiques physico-chimiques similaires). Par exemple, en général le remplacement d'un résidu leucine par un résidu isoleucine ne modifie pas de telles propriétés. Les modifications doivent généralement concerner moins de 40%, notamment moins de 30% de préférence moins de 20% et tout particulièrement moins de 10% des acides aminés du peptide. Il est important que le peptide modifié ne soit pas dénaturé comme on peut le faire par exemple par un traitement physique comme la chaleur afin de préserver ses sites conformationnels pour que les anticorps induits par les dérivés modifiés soient actifs vis à vis de la structure native.

De manière générale, en ce qui concerne les modifications, l'homologie ou la similitude entre le glycopeptide ci-dessus modifié et le glycopeptide ci-dessus natif, de même que les modalités d'utilisation, ou de couplage du composé immunogène tel que décrit ici, à une protéine immunogène telle que le toxoïde tétanique, on peut en particulier se référer à WO-A-86/06 414 ou à EP-A-0.220.273 ou encore à PCT/US.86/00831, équivalents.

Les glycopeptides tels que décrits ici peuvent être préparés à partir notamment de sucs pancréatiques, de sérum, de lait, d'urine ou de liquide amniotique d'origine animale ou humaine. Dans un mode de réalisation préféré, les glycopeptides tels que décrits ici sont isolés et/ou purifiés à partir d'urine d'origine animale ou humaine. De préférence, l'urine est d'origine humaine. Selon un aspect préféré, l'urine provient d'un patient susceptible de souffrir ou souffrant d'une pathologie pancréatique. De préférence, la pathologie pancréatique est un cancer du pancréas. L'homme du métier connaît les techniques pouvant être utilisées pour purifier et/ou isoler les glycopeptides selon la présente invention. Notamment, les glycopeptides peuvent être purifiés en utilisant par exemple, sans se limiter à ces techniques, des centrifugations, des ultrafiltrations, des étapes de concentration, des chromatographies liquides sur colonnes d'exclusions, d'affinités, des chromatographies en phase inverse ou par échange de cations ou d'anions, des électrophorèses ou des combinaisons de ces techniques. En particulier, les anticorps spécifiques des glycopeptides selon l'invention comme J28 et 16D10 peuvent être utilisés pour une purification par affinité. Un exemple de protocole de purification est détaillé dans l'Expérimentation 7. Ainsi, la présente description décrit une méthode de préparation d'un glycopeptide tel que décrit ici, comprenant la récupération de l'urine d'un sujet et la purification dudit glycopeptide à partir de l'urine.

La présente description décrit tout particulièrement les glycopeptides tels que décrits ici, purifiés et/ou isolés à partir d'urine d'un patient susceptible de souffrir ou souffrant d'une pathologie pancréatique, en particulier un cancer du pancréas. Comme cela sera détaillé plus loin, ces glycopeptides purifiés peuvent être utilisés pour un traitement préventif ou thérapeutique par immunothérapie. Ainsi, ils peuvent être utilisés pour la préparation de composition pharmaceutique et/ou vaccinale.

Les glycopeptides tels que décrits ici peuvent être recombinants et préparés comme suit.

On transfecte une lignée convenable d'une cellule hôte conventionnelle comme CHO-K1 à l'aide d'un plasmide comprenant l'ADN complémentaire de la transférase désirée ou par les plasmides comprenant l'ADN complémentaire des transférases désirées, par exemple la glycosyltransférase Core2 β(1-6) N-acétylglucosaminyltransférase (C2GnT), la fucosyltransférase 3 (FUT3), la fucosyltransférase 7 (FUT7) ou l'α(1-3)galactosyltransférase (GT), en utilisant par exemple de la lipofectamine.

On transfecte la lignée cellulaire obtenue par un plasmide permettant l'expression et de préférence la sécrétion des peptides recombinants, par exemple un plasmide pSecTag dans lequel a été introduit l'ADNc codant pour un glycopeptide tel que décrit ici, par exemple le domaine C-terminal de la BSDL ou de la FAPP. Des exemples de séquences codant pour un glycopeptide tel que décrit ici sont SEQ ID Nos 6, 8, 10 et 12.

Le glycopeptide recombinant obtenu par culture de la lignée cellulaire munie des ADNc désirés est alors avantageusement purifié par exemple sur gel de polyacrylamide ou par chromatographie liquide et/ou d'affinité et si désiré analysé en SDS-Page pour son identification.

On peut évidemment transférer dans la cellule hôte les gènes nécessaires pour préparer les glycopeptides tels que décrits ici par tout autre moyen approprié.

Dans des conditions préférentielles de mise en oeuvre du procédé ci-dessus décrit, on transfecte une lignée convenable comme CHO-K1 à l'aide d'un plasmide comprenant l'ADN complémentaire de la glycosyltransférase Core2 β(1-6) N-acétylglucosaminyltransférase (C2GnT), de la fucosyltransférase 3 (FUT3), et d'un glycopeptide tel que décrit ici, par exemple le domaine polypeptidique C-terminal de la FAPP ou notamment de la BSDL, de préférence dans cet ordre.

On utilise avantageusement comme cellule hôte la lignée CHO et notamment la lignée CHO-K1.

On utilise avantageusement comme plasmide les plasmides pSecTag, pcDNA-3 ou pBK-CMV.

Plus généralement, la présente description décrit aussi un procédé de préparation d'un glycopeptide ci-dessus caractérisé en ce que l'on transfère dans une cellule-hôte conventionnelle comprenant un matériel enzymatique nécessaire à l'amorçage d'une glycosylation, un gène codant pour le polypeptide tel que décrit ici et au moins un gène codant pour une ou plusieurs enzymes choisies parmi les glycosyltransférases, dans des conditions permettant l'expression et optionnellement la sécrétion du glycopeptide désiré, puis on isole le glycopeptide attendu.

Des glycopeptides tels que décrits ici, et notamment des glycopeptides naturels ou dérivés de la FAPP ou de la BSDL, peuvent être préparés à partir des protéines isolées depuis les tissus ou fluides biologiques de quelque origine humaine ou animale que ce soit notamment sérums, urines, sucs pancréatiques, sécrétions lactées, homogénats tissulaires ou cellulaires etc. par des méthodes chromatographiques comme décrit par Lombardo et coll. 1978, Biochim. Biophys. Acta, 527 : 142-149, ou par Abouakil et coll. 1988, Biochim. Biophys. Acta, 961 : 299-308, ou par Wang et Johnson, 1983, Anal. Biochem., 133 : 457-461 ou encore par Blackberg et Hernell, 1981, Eur J. Biochem. 116 : 221-225. Les protéines homogènes sont ensuite hydrolysées soit par des méthodes enzymatiques impliquant une quelconque enzyme notamment protéolytique soit par des méthodes chimiques.

Les glycopeptides naturels peuvent alors être isolés par des méthodes chromatogaphiques (tamisage moléculaire, affinité et immunoaffinité, échange ionique....) comme décrit notamment par Mas et coll. 1997, Glycobiology, 7 : 745-752, ou Wang et coll. 1995, Biochemistry, 34 : 10639-10644, ou par toute autre méthode.

Lesdits glycopeptides peuvent aussi être purifiés, isolés soit directement à partir des tissus ou fluides biologiques comme mentionnés ci-dessus soit après traitements enzymatiques ou chimiques exposés ci-dessus de ces tissus ou fluides biologiques.

La glycosylation desdits glycopeptides naturels de la BSDL ou de la FAPP peut être modifiée par toutes voies chimiques ou enzymatiques (utilisation de formes natives, recombinantes solubles, membranaires notamment, de glycosidases ou de glycosyltransférases) afin d'obtenir les structures glycanniques ci-après utilisées notamment pour le diagnostic des pathologies mentionnées ci-dessus et ci-dessous ou pour la vaccination contre lesdites pathologies, ou pour l'obtention ou la fabrication d'anticorps faisant l'objet de la présente invention.

En outre, il est possible de modifier la séquence peptidique des glycopeptides naturels ou recombinants tels que décrits ici.

Les glycopeptides naturels et notamment recombinants, isolés ou purifiés tels que décrits ici, possèdent de très intéressantes propriétés. Ils sont notamment doués de remarquables propriétés immunogènes, spécifiques des pathologies pancréatiques.

Ces propriétés sont illustrées ci-après dans la partie expérimentale. Elles justifient l'utilisation des glycopeptides ci-dessus décrits, à titre de médicament.

C'est pourquoi la présente invention décrit aussi les glycopeptides ci-dessus décrits, pour leur utilisation dans une méthode de traitement thérapeutique du corps humain ou animal, c'est-à-dire à titre de médicament.

Les médicaments décrits ici trouvent leur emploi par exemple dans une thérapie immunitaire (immunisation, vaccination) préventive et/ou curative des cancers du pancréas, des cancers du sein, pour une immunothérapie cellulaire : vaccination autologue par activation du système immunitaire du patient (cellules dendritiques ou autres...), pour le diagnostic de certaines pathologies comme le diabète de type I par détection d'anticorps circulants dirigés contre ces structures chez les patients atteints d'une telle pathologie diabétique et plus généralement de tout autre pathologie nécessitant l'utilisation de l'un ou plusieurs de ces glycopeptides.

Ils trouvent aussi leur emploi dans d'autres systèmes biologiques comme l'inflammation, la formation de métastases et l'inhibition de l'invasion par des agents pathogènes.

Ainsi, la présente description décrit une composition pharmaceutique ou vaccinale comprenant un glycopeptide tel que décrit ici. En particulier, la présente description décrit une composition pharmaceutique ou vaccinale comprenant un glycopeptide comprenant 1 à 40 répétitions de la séquence peptidique décrite dans SEQ ID No 14 et glycosylé par une ou plusieurs enzymes à activité transférase d'oses sélectionnées parmi le groupe constitué de la Core 2 β(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède une activité α(1-3) fucosyltransférase. Facultativement, ledit glycopeptide est glycosylé par les enzymes C2GnT et FUT3. Facultativement, ledit glycopeptide est glycosylé par les enzymes C2GnT et FUT7. Facultativement, ledit glycopeptide est glycosylé par les enzymes C2GnT, FUT3 et FUT7. De préférence, ledit glycopeptide est en outre glycosylé par l'α(1-3)galactosyltransférase (GT). Selon un aspect préféré, ledit glycopeptide comprend entre 1 et 15 desdites répétitions. Ledit glycopeptide peut être recombinant ou purifié à partir d'un fluide biologique. De préférence, ledit fluide biologique est l'urine, en particulier l'urine provenant d'un sujet souffrant d'une pathologie pancréatique, notamment le cancer du pancréas. Selon un aspect particulier, ledit glycopeptide est chargé sur des cellules présentatrices d'antigène, de préférence des cellules dendritiques.

La présente description décrit également l'utilisation d'un glycopeptide ou d'une composition pharmaceutique ou vaccinale tel que décrit ici, comme médicament. En particulier, elle décrit l'utilisation d'un glycopeptide ou d'une composition pharmaceutique ou vaccinale tel que décrit ici, pour la préparation d'un médicament destiné au traitement préventif ou curatif d'une maladie sélectionnée parmi un cancer, une maladie inflammatoire, une pathologie vasculaire ou une infection par un pathogène. De préférence, la maladie est un cancer du sein ou du pancréas. La présente invention concerne l'utilisation d'un glycopeptide ou d'une composition pharmaceutique ou vaccinale tel que décrit ici pour la préparation d'un médicament destiné au traitement ou à la prévention d'un cancer du pancréas. Dans un mode de réalisation préféré, le glycopeptide est obtenu par purification à partir de l'urine d'un sujet souffrant d'une pathologie pancréatique, de préférence d'un cancer du pancréas.

Au sein d'un conditionnement adjuvant, l'immunogène, en l'occurrence un glycopeptide tel que décrit ici, pourra notamment être inclus dans une émulsion eau dans huile, en utilisant par exemple l'ISA 51.

Une préparation vaccinale contenant le glycopeptide immunogène pourra être administrée sous une forme galénique appropriée à induire une réponse immunitaire de type systémique par voie Intra Musculaire (IM), Sous Cutanée (SC), Intra Dermique (ID) ou de type mucosal par voies intra nasale, orale, vaginale ou rectale.

Une préparation vaccinale contenant le glycopeptide immunogène pourra également contenir d'autres immunogènes.

Une préparation galénique à visée systémique, administrée par voie SC, IM, ID, pourra être une émulsion eau renfermant le glycopeptide immunogène, dans huile, ou une suspension de phosphate de calcium enchâssant l'immunogène, ou de l'hydroxyde d'aluminium adsorbant l'immunogène.

Les préparations vaccinales pourront être conditionnées pour la voie intra nasale sous forme de gel avec comme excipient le carbopol, de gouttes nasales ou de spray et pour la voie orale sous forme de capsules gastrorésistantes, de dragées ou de granules gastrorésistants.

La dose usuelle, variable selon le sujet traité et l'affection en cause, peut être, par exemple, de 1 à 100 mg d'un glycopeptide tel que décrit ici, notamment du glycopeptide de l'exemple 4 ci-dessous, administré par voie sanguine chez l'homme, par quinzaine de jours pendant 10 à 20 semaines.

La présente description décrit aussi les compositions pharmaceutiques qui renferment au moins un glycopeptide ci-dessus décrit, à titre de principe actif.

Dans ces compositions, le principe actif est avantageusement présent à des doses physiologiquement efficaces ; les compositions précitées renferment notamment une dose vaccinale efficace d'au moins un principe actif ci-dessus.

A titre de médicaments, les glycopeptides ci-dessus décrits peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés simples ou dragéifiés, les gélules, les granulés, les caramels, les suppositoires, notamment les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La présente description décrit encore un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

L'invention a encore pour objet l'utilisation d'un glycopeptide ci-dessus décrit, pour l'obtention d'un médicament destiné au traitement curatif ou préventif des cancers du pancréas.

La présente description décrit également une méthode de traitement préventif ou curatif d'une maladie sélectionnée parmi un cancer, une maladie inflammatoire, une pathologie vasculaire ou une infection par un pathogène chez un sujet, comprenant l'administration d'une dose efficace d'un glycopeptide tel que décrit ici.

Dans un mode de réalisation préféré du traitement du cancer, le glycopeptide peut être chargé sur une cellule présentatrice d'antigène allogénique. De préférence, la cellule présentatrice d'antigène est une cellule dendritique. D'une manière préférée, la cellule présentatrice d'antigène est autologue, c'est-à-dire qu'elle a été préalablement prélevée sur le sujet receveur ou qu'elle est dérivée de cellules souches provenant du sujet receveur. Dans une alternative, la cellule présentatrice est issue d'un prélèvement sur un sujet allogénique, c'est-à-dire compatible avec le receveur. Ainsi, dans ce mode de réalisation, la composition pharmaceutique ou vaccinale telle que décrite ici comprend des cellules présentatrices d'antigène chargées ou « pulsées » avec un glycopeptide tel que décrit ici. La présente invention concerne donc l'utilisation d'une composition pharmaceutique ou vaccinale comprenant des cellules présentatrices d'antigène chargées ou « pulsées » avec un glycopeptide tel que décrit ici comme médicament pour le traitement ou la prévention d'un cancer du pancréas. Dans un mode de réalisation préféré, le glycopeptide est obtenu par purification à partir de l'urine d'un sujet souffrant d'une pathologie pancréatique, de préférence d'un cancer du pancréas. Dans un mode de réalisation le plus souhaitable, le glycopeptide est obtenu par purification à partir de l'urine du sujet traité. De préférence, le sujet est un humain. La présente invention concerne en outre une méthode de traitement curatif ou préventif d'un cancer du pancréas, comprenant :la fourniture de glycopeptides tels que décrits ici ; et l'administration desdits glycopeptides, éventuellement chargés sur des cellules présentatrices d'antigène, ladite administration permettant de traiter, de diminuer ou de prévenir un cancer du pancréas. Lesdits glycopeptides peuvent être recombinants ou isolés d'un échantillon biologique, de préférence un fluide biologique. Dans un mode de réalisation préféré, lesdits glycopeptides sont isolés et/ou purifiés à partir purification à partir de l'urine d'un sujet souffrant d'une pathologie pancréatique, de préférence d'un cancer du pancréas, ou d'un cancer du sein. De préférence, lesdits glycopeptides sont isolés et/ou purifiés à partir purification à partir de l'urine du sujet traité. Ainsi, l'étape de fourniture des glycopeptides peut comprendre le prélèvement du fluide biologique, de préférence l'urine, et la purification des dits glycopeptides.

Les propriétés des glycopeptides ci-dessus décrits justifient aussi leur utilisation dans le diagnostic, notamment dans des méthodes immunoenzymatiques.

C'est pourquoi la présente demande décrit aussi une composition pour le diagnostic *in vitro* de la présence d'anticorps contre un glycopeptide ci-dessus décrit, dans un prélèvement biologique humain, caractérisée en ce qu'elle contient au moins un glycopeptide ci-dessus décrit.

La présente demande décrit aussi une méthode pour la détection *in vitro* de la présence d'anticorps induits chez l'homme affecté d'une pathologie ci-dessus, dans un échantillon biologique humain tel qu'un sérum et, plus particulièrement pour le diagnostic *in vitro* d'un cancer du pancréas ou d'une pathologie diabétique et provenant de la personne faisant l'objet du diagnostic, caractérisée en ce que l'on met en contact cet échantillon biologique avec un antigène reconnu par un anticorps induit chez le malade tel que défini ci-dessus ou ci-après, dans des conditions autorisant la formation d'un composé immunologique entre cet antigène et ledit anticorps et en ce que l'on détecte le conjugué immunologique éventuellement formé entre cet anticorps et l'antigène mis en oeuvre.

La présente demande décrit aussi un nécessaire ou kit pour la détection d'anticorps induits chez l'homme affecté d'une pathologie ci-dessus, dans un échantillon biologique, notamment d'un porteur éventuel de ces anticorps, caractérisé en ce qu'il comprend :
- au moins un glycopeptide ci-dessus décrit
- des moyens pour détecter le conjugué immunologique résultant de la réaction immunologique entre l'antigène et ledit échantillon biologique.

Les glycopeptides notamment recombinants, isolés ou purifiés tels que décrits ici trouvent aussi leur emploi dans le diagnostic de certaines pathologies comme le diabète de type I par détection d'anticorps circulants dirigés contre ces structures chez les patients atteints d'une telle pathologie diabétique.

On utilise particulièrement les glycopeptides dénommés C2-F7-16R pour le diagnostic du diabète de type I.

Comme les glycopeptides notamment recombinants, isolés ou purifiés tels que décrits ici possèdent de très intéressantes propriétés antigéniques, ils permettent aussi l'élaboration d'anticorps notamment monoclonaux.

C'est pourquoi la présente demande décrit aussi un anticorps notamment monoclonal donnant lieu à une réaction immunologique spécifique avec un glycopeptide ci-dessus décrit, à l'exception de l'anticorps J28 et de tout autre anticorps répondant à la définition ci-dessus et pouvant être décrit dans la littérature. Les anticorps monoclonaux peuvent être de toute classe, notamment des IgM, IgD, IgE ou IgA. De préférence, les anticorps monoclonaux sont des IgG. Un anticorps tel que décrit ici peut être modifié, quelle que soit sa classe initiale, en un anticorps d'une autre classe d'immunoglobuline, par exemple par des techniques connues de biologie moléculaire (Lewis et al., 1992, Hum. Antibodies Hybridomas, 3, 146-52; Lewis et al., 1993, J. Immunol., 151, 2829-38; Hall et al., 1994, Cancer Res., 54, 5178-85 ; Shepherd et Dean, Monoclonal antibodies , Oxford university press, 2000,479 pages). Ces mêmes anticorps monoclonaux pourront être humanisés par recombinaison génétique ou par tout autre procédé pour conserver leur site de reconnaissance desdits glycopeptides recombinants ou naturels afin de les utiliser dans les conditions citées ci-dessus.

On retient particulièrement un anticorps obtenu par immunisation d'un mammifère à l'aide d'un glycopeptide ci-dessus décrit.

La présente invention concerne tout particulièrement les anticorps décrits dans les exemples et notamment les anticorps 16D10 de type IgM pouvant être produits par l'hybridome déposé à la Collection Nationale de Culture de Microorganismes (CNCM) à Paris le 16 mars 2004 sous le N° I-3188 et 8H8 de type IgG.

De préférence, la présente invention concerne un anticorps monoclonal sélectionné parmi l'anticorps monoclonal 16D10, un fragment ou un dérivé de celui-ci, et un anticorps qui se lie essentiellement au même épitope que l'anticorps monoclonal 16D10. Facultativement, ledit anticorps est humanisé, chimérique ou humain. De préférence, l'anticorps est de type IgG. Facultativement, l'anticorps est un anticorps simple chaine.

Par fragment ou dérivé d'un anticorps est entendu une entité conservant substantiellement la même spécificité que l'anticorps considéré. Un fragment de celui-ci peut être un fragment Fab, Fab', F(ab)2 et sFv (Blazar et al., 1997, Journal of Immunology 159 : 5821-5833 et Bird et al., 1988, Science 242 : 423-426). Des fragments Fv, Fab, ou F(ab)2 conformes à l'invention peuvent être obtenus par les techniques classiques de digestion enzymatique. Un dérivé de celui-ci peut être un anticorps chimérique, humanisé ou simple chaîne scFv. Ces dérivés peuvent être obtenus par des techniques classiques de génie génétique. Des polynucléotides codant les régions variables de l'anticorps 16D10 peuvent par exemple être obtenus par clonage desdites régions variables à partir d'une banque d'ADNc de l'hybridome produisant l'anticorps 16D10. Ils peuvent également être préparés totalement ou partiellement, par synthèse d'acides nucléiques, à partir des séquences nucléotidiques desdites régions variables. On peut par exemple synthétiser des polynucléotides codant les CDRs de 16D10, et les incorporer dans les régions adéquates d'un autre anticorps, notamment d'un anticorps d'origine humaine et/ou de type IgG, par les techniques connues de greffe de CDRs, telles que celles décrites par Routledge et al., ("Reshaping antibodies for therapy", in Protein Engineering of antibody molecules for prophylactic and therapeutic applications in man, 13-44, Academic Titles, Nottingham, England (1993)] ou par Roguska et al., Protein Engineering, 9 (10), 895-904, (1996)).

La présente demande décrit également toute molécule d'acide nucléique codant une protéine comprenant les CDRs de l'anticorps 16D10, ainsi que tout vecteur recombinant, notamment tout vecteur d'expression, comprenant ladite molécule d'acide nucléique, toute cellule hôte comprenant cet acide nucléique ou ce vecteur.

La présente demande décrit encore un procédé de préparation d'un anticorps anti-glycopeptide ci-dessus décrit, caractérisé en ce que l'on immunise un mammifère à l'aide dudit glycopeptide ou à l'aide d'un mélange de FAPP et de BSDL isolé à partir d'un quelconque fluide ou tissu biologique humain ou animal et notamment de sucs pancréatiques humains normaux et pathologiques puis récupère les anticorps attendus reconnaissant le domaine C-terminal de ces protéines et les purifie si désiré.

La présente demande décrit tout autant un procédé de préparation d'un anticorps anti-glycopeptide ci-dessus décrit caractérisé en ce que l'on procède au clonage de lymphocytes B provenant d'un sujet immunisé à l'aide d'un glycopeptide naturel ou recombinant décrit ci-dessus ou à l'aide d'un mélange de FAPP et de BSDL ci-dessus décrit et transformés par le virus Epstein-Barr (EBV), puis récupère les anticorps attendus sécrétés par lesdits lymphocytes B transformés.

Les anticorps monoclonaux objets de la présente invention sont notamment capables de donner lieu à une réaction immunologique spécifique avec un glycopeptide ci-dessus décrit.

Dans un mode de réalisation particulier de la présente invention, l'anticorps se lie essentiellement au même épitope que l'anticorps monoclonal 16D10 (produit respectivement par l'hybridome 16D10). De préférence, ledit anticorps est un anticorps monoclonal. De préférence, l'invention concerne l'anticorps monoclonal 16D10 (produit par l'hybridome 16D10), mais il sera apprécié que l'invention concerne également les anticorps monoclonaux capables de déplacer spécifiquement l'anticorps 16D10. Selon un autre aspect particulier, les anticorps monoclonaux décrits ici peuvent déplacer spécifiquement l'anticorps J28.

Le terme « se lie essentiellement au même épitope ou déterminant que » un anticorps d'intérêt signifie que l'anticorps « entre en compétition » avec ledit anticorps d'intérêt. Le terme « se lie essentiellement au même épitope ou déterminant que » un anticorps d'intérêt ne signifie pas que les deux anticorps ont exactement le même épitope. Cependant, dans un mode de réalisation particulier, les deux anticorps peuvent avoir le même épitope. Le terme « se lie essentiellement au même épitope ou déterminant que » un anticorps monoclonal 16D10 signifie que l'anticorps « entre en compétition » avec l'anticorps 16D10. De manière générale, un anticorps qui « se lie essentiellement au même épitope ou déterminant que » un anticorps d'intérêt (par ex., l'anticorps 16D10) signifie que l'anticorps « entre en compétition » avec ledit anticorps d'intérêt pour un glycopeptide selon la présente invention, notamment une ou plusieurs protéines parmi BSDL ou FAPP ou un fragment de celles-ci, de préférence pour une partie C-terminale de BSDL ou FAPP comme décrit dans la présente demande, de manière encore plus préférée une partie comprenant une ou plusieurs répétitions de 11 acides aminés de BSDL ou FAPP, ou une partie ou un fragment de celle-ci. Dans d'autres exemples, un anticorps se lie essentiellement au même épitope ou déterminant de BSDL ou FAPP lorsque l'anticorps « entre en compétition » avec l'anticorps d'intérêt pour la liaison à BSDL ou FAPP.

Le terme « se lie essentiellement au même épitope ou déterminant que » un anticorps d'intérêt signifie l'anticorps « entre en compétition » avec ledit anticorps d'intérêt pour toute molécule BSDL et/ou FAPP à laquelle ledit anticorps d'intérêt se lie spécifiquement. Le terme « se lie essentiellement au même épitope ou déterminant que » un anticorps monoclonal 16D10 signifie l'anticorps « entre en compétition » avec ledit anticorps monoclonal 16D10 pour toute molécule BSDL et/ou FAPP à laquelle ledit anticorps monoclonal 16D10 se lie spécifiquement. Par exemple, un anticorps qui se lie essentiellement au même épitope ou déterminant que les anticorps monoclonaux 16D10 ou J28 « entre en compétition avec ledit anticorps 16D10 ou J28 pour la liaison de BSDL ou FAPP, respectivement.

L'identification d'un ou plusieurs anticorps qui se lie(nt) essentiellement au même épitope que les anticorps monoclonaux décrits dans la présente demande peut être réalisée en utilisant une quelconque des méthodes de test de criblage immunologique dans lesquelles une compétition entre anticorps peut être évaluée. De nombreux tests sont effectués en routine et sont bien connus de l'homme du métier (voir, par ex., U.S. 5,660,827, délivré le 26 Août 1997, qui est spécifiquement incorporé ici par référence). La détermination de l'épitope auquel se lie un anticorps n'est pas nécessaire pour identifier un anticorps qui se lie, ou qui se lie essentiellement, au même épitope que l'anticorps monoclonal décrit dans la présente demande.

Par exemple, lorsque les anticorps candidats à étudier ont été obtenus de différentes sources animales, ou sont éventuellement d'isotypes d'Ig différents, un simple test de compétition peut être employé dans lequel l'anticorps contrôle (l'anticorps 16D10, par exemple) et l'anticorps candidat sont mélangés (ou pré-adsorbés) et mis en contact avec un échantillon contenant soit BSDL soit FAPP, les deux étant connus pour se lier avec l'anticorps 16D10. Des protocoles basés sur des ELISAs, des dosages radioimmunologiques, des analyses par Western Blot, ou l'utilisation d'une analyse par BIACORE (comme décrite, par exemple, dans les Exemples) sont adaptés pour une utilisation dans des études simples de compétition.

Dans des modes de réalisation particuliers, on pourrait préalablement préparer des mélanges d'anticorps contrôle (par exemple, l'anticorps 16D10) avec des quantités variables des anticorps candidats (par exemple, environ 1 :10 ou environ 1 :100) avant la mise en contact avec l'échantillon d'antigène BSDL ou FAPP. Dans un autre mode de réalisation, l'anticorps contrôle et les quantités variables des anticorps candidats peuvent simplement être mélangés lors de la mise en contact avec l'échantillon d'antigène BSDL ou FAPP. Tant que l'on peut distinguer les anticorps liés des anticorps libres (par ex., en utilisation des techniques de séparation ou de lavage pour éliminer les anticorps non-liés) et distinguer l'anticorps 16D10 des anticorps candidats (par ex., en utilisant des anticorps secondaires spécifiques de l'espèce ou de l'isotype ou en marquant l'anticorps 16D10 par un marqueur détectable), on sera capable de déterminer si les anticorps candidats réduisent la liaison de l'anticorps 16D10 à l'antigène BSDL ou FAPP, indiquant ainsi si l'anticorps candidat reconnaît essentiellement le même épitope que l'anticorps 16D10. La liaison de l'anticorps contrôle en présence d'un anticorps complètement non-pertinent peut servir de valeur haute de contrôle. La valeur basse de contrôle peut être obtenue en incubant des anticorps de contrôle (16D10) marqués avec des anticorps non-marqués reconnaissant exactement le même épitope (16D10), provoquant ainsi une compétition qui diminue la liaison des anticorps marqués. Dans un test, une réduction significative de la réactivité d'un anticorps marqué en présence d'un anticorps candidat indique que l'anticorps candidat reconnaît essentiellement le même épitope, c'est-à-dire qu'il réagit de manière croisée avec l'anticorps marqué (16D10). Tout anticorps candidat qui réduit la liaison de l'anticorps 16D10 à chacun des antigènes BSDL ou FAPP d'au moins environ 20%, 30%, 40%, 50%, de préférence d'au moins environ 60%, ou de manière encore plus préférée d'au moins environ 70% (par ex., 65-100%), à un ratio de l'anticorps 16D10 : anticorps candidat entre environ 1 :10 et environ 1 :100, est considéré comme un anticorps qui se lie essentiellement au même épitope ou déterminant que l'anticorps 16D10. De préférence, de tels anticorps candidats réduisent la liaison de l'anticorps 16D10 à l'antigène BSDL ou FAPP d'au moins environ 90% (par ex., environ 95%). De préférence, l'anticorps 16D10 diminue également la liaison de l'anticorps candidat à l'antigène BSDL ou FAPP lorsque l'évaluation de la liaison est faite de la même manière, bien que le degré de diminution de la liaison puisse être différent.

La compétition peut être évaluée, par exemple, dans un dosage en cytométrie de flux. Dans un tel dosage, les cellules portant un antigène BSDL ou FAPP, par exemple des cellules transfectés avec BSDL ou FAPP, sont d'abord incubées avec l'anticorps 16D10, par exemple, puis avec l'anticorps candidat marqué avec un fluorochrome ou la biotine. On considère que l'anticorps entre en compétition avec l'anticorps 16D10 si la liaison obtenue après préincubation avec une quantité saturante de 16D10 est d'environ 30%, de préférence d'environ 40%, d'environ 50%, d'environ 80%, ou plus (par ex., environ 90 %) de liaison (telle que mesurée par fluorescence) obtenue par l'anticorps sans préincubation avec 16D10. De manière alternative, un anticorps est considéré comme entrant en compétition avec l'anticorps 16D10 lorsque la liaison obtenue avec un anticorps 16D10 marqué (par un fluorochrome ou une biotine) sur des cellules préincubées avec une quantité saturant d'anticorps candidat est d'environ d'environ 80%, de préférence d'environ 50%, d'environ 40%, d'environ 30%, ou moins (par ex., environ 20 %) de liaison obtenue sans préincubation avec l'anticorps.

Un dosage simple de compétition dans lequel un anticorps candidat est pré-adsorbé et appliqué à une concentration saturante à une surface sur laquelle a été immobilisé un antigène BSDL ou FAPP peut également être utilisé avantageusement. La surface dans le dosage simple de compétition est de préférence une puce BIACORE (ou un autre support compatible avec l'analyse de résonance des plasmons de surface). L'anticorps contrôle (par ex., 16D10) est alors mis en contact avec une surface à une concentration saturante de BSDL ou FAPP et la liaison à la surface portant BSDL ou FAPP de l'anticorps contrôle est mesurée. Cette liaison de l'anticorps contrôle est comparée à la liaison de l'anticorps contrôle à la surface portant BSDL ou FAPP en absence de l'anticorps candidat. Dans un test de dosage, une réduction significative de la liaison à la surface portant BSDL ou FAPP par l'anticorps contrôle en présence de l'anticorps candidat indique que l'anticorps candidat reconnait essentiellement le même épitope que l'anticorps contrôle de telle manière que l'anticorps candidat réagit de façon croisée avec l'anticorps contrôle. Tout anticorps candidat qui diminue la liaison de l'anticorps contrôle (tel que l'anticorps 16D10) à l'antigène BSDL ou FAPP d'au moins environ 30% ou de préférence environ 40% peut être considéré comme un anticorps qui se lie essentiellement au même épitope que l'anticorps contrôle (par ex., 16D10). De préférence, un tel anticorps candidat réduit la liaison de l'anticorps contrôle (par ex., 16D10) à l'antigène BSDL ou FAPP d'au moins environ 50% (par ex., au moins environ 60%, au moins environ 70%, ou plus). Il sera apprécié que l'ordre entre les anticorps contrôle et candidat peut être inversé, c'est-à-dire que l'anticorps contrôle peut être le premier à se lier à la surface et que l'anticorps candidat est mis en contact avec la surface ensuite dans un test de compétition. De préférence, l'anticorps présentant l'affinité la plus élevée pour BSDL ou FAPP est lié à la surface portant BSDL ou FAPP en premier, puisqu'il est espéré que la diminution de liaison observée pour le deuxième anticorps (en supposant que les anticorps réagissent de manière croisée) soit de plus grande amplitude. D'autres exemples de tels tests sont fournis dans les exemples et dans Saunal and Regenmortel, (1995) J. Immunol. Methods 183: 33-41, le contenu de celui-ci étant incorporé ici par référence.

Ces propriétés sont illustrées ci-après dans la partie expérimentale. Elles justifient l'utilisation des anticorps monoclonaux ci-dessus décrits et notamment des anticorps monoclonaux 8H8, 16D10, 14H10 et autres anticorps monoclonaux dirigés contre la partie C-terminale de la FAPP et/ou la BSDL, pour le diagnostic, le pronostic et/ou la prédiction de plusieurs pathologies pancréatiques dont le cancer du pancréas, les pancréatites et les diabètes de type I, mais aussi le cancer du sein ou des pathologies cardiovasculaires. Les éléments diagnostiques pourront être obtenus lors de dosages sériques et/ou urinaires.

L'anticorps 16D10 présente des propriétés particulièrement intéressantes. Enfin, il est spécifique du cancer du pancréas. Il ne reconnait ni les tissus normaux, ni les tissus tumoraux d'autres origines. L'épitope reconnu par l'anticorps 16D10 est distinct de celui reconnu par l'anticorps J28 puisque ces deux anticorps ne sont pas en compétition. Ces résultats sont détaillés dans l'exemple 4.

Ainsi, la présente invention concerne une méthode permettant de détecter, de préférence *in vitro,* une pathologie pancréatique, en particulier un cancer du pancréas, comprenant la mise en contact d'un échantillon biologique avec un anticorps tel que décrit ici et la détection de la formation de complexes immunologiques résultant de la réaction immunologique entre ledit anticorps et ledit échantillon biologique. De préférence, l'anticorps est l'anticorps 16D10, un fragment ou un dérivé de celui-ci, ou un anticorps qui se lie essentiellement au même épitope ou déterminant que celui-ci. De préférence, l'échantillon biologique est un prélèvement de tissu pancréatique (biopsie) ou un fluide biologique. L'échantillon biologique peut être une coupe de tissu (immuno-histochimie) ou des cellules contenues dans l'échantillon ou dérivées par culture de l'échantillon (immuno-cytochimie). Le fluide biologique peut être sélectionné parmi le groupe constitué du sérum, de l'urine, des sucs pancréatiques et des sécrétions lactées. De préférence, le fluide biologique est l'urine. La révélation du complexe peut être directe en marquant l'anticorps selon la présente invention ou indirecte en ajoutant une molécule révélant la présence de l'anticorps selon la présente invention (anticorps secondaire, couple streptavidine/biotine, etc...). Par exemple, le marquage peut être effectué en couplant l'anticorps à des marqueurs radioactifs ou fluorescents. Ces techniques sont bien connues de l'homme du métier.

La présente invention concerne également l'utilisation d'un anticorps selon la présente invention pour la préparation d'une composition diagnostic utile pour la détection d'une pathologie pancréatique *in vivo* ou *in vitro*. De préférence, l'anticorps est l'anticorps 16D10, un fragment ou un dérivé de celui-ci, ou un anticorps qui se lie essentiellement au même épitope ou déterminant que celui-ci. De préférence, la pathologie pancréatique est un cancer du pancréas.

Dans un mode de réalisation préféré, la présente invention concerne une méthode permettant de détecter une pathologie pancréatique chez un sujet, en particulier un cancer du pancréas, comprenant la récupération de l'urine du sujet, la mise en contact de ladite urine avec un anticorps selon la présente invention, et la détection de la formation de complexes immunologiques résultant de la réaction immunologique entre ledit anticorps et ladite urine. De préférence, l'anticorps est l'anticorps 16D10, un fragment ou un dérivé de celui-ci, ou un anticorps qui se lie essentiellement au même épitope ou déterminant que celui-ci. L'anticorps peut également être l'anticorps J28, un fragment ou un dérivé de celui-ci, ou un anticorps qui se lie essentiellement au même épitope ou déterminant que celui-ci. De préférence, la pathologie pancréatique est le cancer du pancréas. La révélation du complexe peut être directe en marquant l'anticorps selon la présente invention ou indirecte en ajoutant une molécule révélant la présence de l'anticorps selon la présente invention (anticorps secondaire, couple streptavidine/biotine, etc...). Ces techniques sont bien connues de l'homme du métier. Facultativement, la méthode peut comprendre des étapes intermédiaires de traitement de l'échantillon d'urine avant incubation avec ledit anticorps. Ces étapes peuvent être par exemple une concentration des urines, des étapes de purification ou d'enrichissement en glycopeptide, etc...

La présente invention concerne aussi l'utilisation d'un anticorps selon la présente invention pour le diagnostic d'une pathologie pancréatique, en particulier le cancer du pancréas chez un sujet. De préférence, l'anticorps est l'anticorps 16D10, un fragment ou un dérivé de celui-ci, ou un anticorps qui se lie essentiellement au même épitope ou déterminant que celui-ci. L'anticorps peut également être l'anticorps J28, un fragment ou un dérivé de celui-ci, ou un anticorps qui se lie essentiellement au même épitope ou déterminant que celui-ci.

La présente invention concerne un kit de diagnostic d'une pathologie pancréatique, en particulier du cancer du pancréas, comprenant un anticorps selon la présente invention. Ce kit peut comprendre en outre des moyens pour détecter le conjugué immunologique résultant de la réaction immunologique entre l'échantillon biologique et ledit anticorps, notamment des réactifs permettant la révélation dudit anticorps. Le kit comprend l'anticorps 16D10, un fragment ou un dérivé de celui-ci, ou un anticorps qui se lie essentiellement au même épitope ou déterminant que celui-ci.

Ces propriétés justifient l'utilisation des anticorps monoclonaux ci-dessus décrits pour développer de nouveaux protocoles thérapeutiques des cancers du pancréas exocrine comme le ciblage des cellules tumorales avec ces anticorps couplés à des drogues (chimiothérapie) ou à des éléments radioactifs (radiothérapie et radiodiagnostic) ou encore à des gènes modifiant le devenir ou le comportement des cellules néoplasiques (thérapie génique).

Les anticorps couplés à des éléments radioactifs pourront être utilisés en radiolocalisation (immunoscintigraphie) des tumeurs primaires et des métastases (tumeurs secondaires).

Ces éléments diagnostiques pourront être acquis lors de dosages sériques et/ou urinaires.

Notamment, la présente invention concerne des anticorps tels que décrits ici, en particulier monoclonaux, couplés à une substance active antitumorale, qui peuvent être utilisés dans le traitement des cancers pancréatiques pour cibler et détruire les cellules tumorales pancréatiques.

De même des éléments radioactifs (ou autres) peuvent être couplés à ces anticorps et permettre une localisation précise des tumeurs primaires et des métastases. Lesdits anticorps peuvent en outre permettre une distinction claire et nette du tissu normal versus le tissu tumoral.

Ces mêmes propriétés justifient l'utilisation desdits anticorps monoclonaux seuls, combinés ou couplés à une molécule à visée diagnostique ou thérapeutique pour développer de nouveaux protocoles d'immunothérapies passives des pathologies cités ci-dessus.

C'est pourquoi la présente demande décrit aussi une composition pour le diagnostic in vitro d'une pathologie ci-dessus décrite, dans un prélèvement biologique humain, caractérisée en ce qu'elle contient au moins un anticorps monoclonal ci-dessus décrit, notamment couplé à au moins un élément radioactif. Selon la nature de l'élément radioactif, une telle composition pourra être utilisée à titre curatif notamment pour irradier une région du pancréas.

A titre de composition pour le diagnostic, les anticorps ci-dessus peuvent être mélangés avec des excipients acceptables.

La présente demande a encore pour objet un nécessaire pour la détection de la présence d'un glycopeptide ci-dessus décrit apparaissant chez l'homme affecté d'une pathologie diabétique ou néoplasique dans un échantillon biologique humain provenant de la personne faisant l'objet du diagnostic, caractérisé en ce qu'il comprend :
- au moins un anticorps tel que défini ci-dessus,
- des moyens pour détecter le conjugué immunologique résultant de la réaction immunologique entre l'anticorps et l'échantillon biologique.

Les conditions préférentielles de mise en oeuvre des glycopeptides et anticorps ci-dessus décrites s'appliquent également aux autres objets de l'invention visés ci-dessus, notamment aux méthodes de diagnostic.

La présente invention concerne également une composition pharmaceutique comprenant un anticorps selon la présente invention. De préférence, l'anticorps est l'anticorps 16D10, un fragment ou un dérivé de celui-ci, ou un anticorps qui se lie essentiellement au même épitope ou déterminant que celui-ci. L'anticorps peut également être l'anticorps J28, un fragment ou un dérivé de celui-ci, ou un anticorps qui se lie essentiellement au même épitope ou déterminant que celui-ci. La composition peut également comprendre un support pharmaceutiquement acceptable. Dans un mode de réalisation particulier, ledit anticorps est couplé à une substance anti-tumorale.

La présente demande décrit également l'utilisation d'un anticorps selon la présente invention ou d'une composition pharmaceutique comprenant un tel anticorps comme médicament. En particulier, la présente invention concerne l'utilisation d'un anticorps selon la présente invention ou d'une composition pharmaceutique comprenant un tel anticorps pour la préparation d'un médicament destiné au traitement préventif ou curatif d'un cancer du pancréas. De préférence, l'anticorps est l'anticorps 16D10, un fragment ou un dérivé de celui-ci, ou un anticorps qui se lie essentiellement au même épitope ou déterminant que celui-ci. L'anticorps peut également être l'anticorps J28, un fragment ou un dérivé de celui-ci, ou un anticorps qui se lie essentiellement au même épitope ou déterminant que celui-ci. Dans un mode de réalisation particulier, ledit anticorps est couplé à une substance anti-tumorale.

La présente description décrit une méthode de traitement préventif ou curatif d'un sujet souffrant d'une pathologie pancréatique, en particulier le cancer du pancréas, ou d'un cancer du sein, comprenant l'administration d'une quantité efficace d'un anticorps tel que décrit ici audit sujet, cette administration résultant en une diminution ou disparition de la pathologie pancréatique, en particulier le cancer du pancréas, ou du cancer du sein chez le sujet. De préférence, l'anticorps est l'anticorps 16D10, un fragment ou un dérivé de celui-ci, ou un anticorps qui se lie essentiellement au même épitope ou déterminant que celui-ci. L'anticorps peut également être l'anticorps J28, un fragment ou un dérivé de celui-ci, ou un anticorps qui se lie essentiellement au même épitope ou déterminant que celui-ci. Dans un mode de réalisation particulier, ledit anticorps est couplé à une substance anti-tumorale.

Dans un autre protocole la dose usuelle, variable selon le sujet traité et l'affection en cause, peut être, par exemple, de 1 à 10 mg de l'anticorps monoclonal de l'exemple 18 ci-dessous par kilo corporel administré par voie sanguine chez l'homme, un fois par semaine pendant 2 semaines.

L'invention a pour objet tout autant la mise à disposition de nouveaux produits (glycopeptides, anticorps, etc.) que d'autres produits, meilleurs, plus efficaces ou plus purs que ceux pouvant avoir été décrits dans l'état de la technique.

Les exemples qui suivent illustrent la présente demande.

### Description des figures

**Figure 1****.** Examen comparant la réactivité du mAbJ28 et du mAb16D10 sur deux tissus pancréatiques tumoraux (PDAC = ADK1 et PDAC4 = ADK4).
**Figure 2****.** Test de compétition entre les anticorps mAb J28 et mAb 16D10 pour le glycopeptide J28.
**Figure 3****.** Test de compétition entre les anticorps mAb J28 et mAb 16D10 sur des cellules SOJ-6.
**Figure 4****.** Analyse comparative par immunodétection d'urines de personnes saines (échantillons 29 à 36) et de personnes atteintes d'un cancer du pancréas (échantillons 1 à 5) à l'aide de l'anticorps polyclonal pAb L64, de l'anticorps monoclonal mAb 8H8 et de l'anticorps monoclonal mAb J28. Les protéines urinaires sont séparées sur un gel de polyacrylamide (10 %) puis électrotransférées sur une membrane de nitrocellulose. Les membranes sont incubées en présence de l'AcpL64, de l'Acm 8H8 ou de l'Acm J28. Les pistes de gauche (Std) correspondent au marqueur de poids moléculaires.

### EXEMPLES

### Préparations de glycopeptides recombinants

Des glycopeptides recombinants de la BSDL (SEQ ID No 9) et de la FAPP (SEQ ID No 13), ont été exprimés dans différentes souches issues de la lignée cellulaire bien connue CHO-K1, une lignée cellulaire provenant d'une tumeur ovarienne prélevée sur un hamster chinois et présentant une morphologie de type fibroblastique, qui peut être obtenue notamment auprès de *l'American Type Culture Collection* ATCC sous le n° CRL61, Buck et coll., 1958, J. Exp.Med ; 108, 945-955).

La lignée cellulaire CHO-K1 a été choisie car elle ne présente pas d'activité C2GnT glycosyltransférase, α(1-2)fucosyltransférase, α(1-3) fucosyl-transférase, α(1-4) fucosyltransférase ni α(1-3)galactosyltransférase détectable dans les conditions de dosage standard.

### Préparations des plasmides (ADNc clonés)

Les ADNc clonés ayant permis l'expression des différentes composantes des glycopeptides ont été obtenus par transcription inverse, réalisée à l'aide de sonde polynucléotidique polydT (18 bases) s'hybridant au niveau de la queue polyA d'ARN messagers extraits à partir d'échantillons tissulaires de pancréas humains sains ou de lignée tumorale pancréatique humaine comme par exemple la lignées SOJ-6 [Fujii et coll., 1990, Hum. Cell., 3, 31-36] provenant d'un adénocarcinome du corps pancréatique humain, suivant la méthode décrite par Chirgwin et coll. [1979, Biochemistry, 18, 5294-5299].

### Préparation 1 : Plasmide pSec-FAPP

L'amplification de l'ADNc de la protéine foetoacineuse pancréatique (FAPP) (SEQ ID No 10) a été réalisée par transcription inverse à partir de 5 µg d'ARN totaux extraits de la lignée tumorale pancréatique humaine SOJ-6 citée ci-dessus (45 minutes à 55°C, trousse de transcription inverse, Sigma, St Louis, MO, USA) suivi d'une PCR grâce aux amorces nucléotidiques C-ter/FAPP-BSDL et N-ter/FAPP, définies à partir de la séquence de l'ADNc codant pour la BSDL humaine (SEQ ID No 6) dans les conditions décrites ci-après. La réaction de polymérisation en chaîne a été réalisée avec une trousse « Advantage-GC cDNA PCR » (Clontech) permettant l'amplification de séquences nucléiques particulièrement riches en guanine et cytosine. Le programme appliqué a été : un cycle de 2 minutes à 94°C suivi de trente cinq cycles d'1 minute à 94°C (dénaturation), 1 minute à 52°C (hybridation des amorces), et 4 minutes à 68°C (élongation), puis un cycle de 10 minutes à 68°C, effectués dans un thermocycleur Robocycler Gradient 96 (Stratagène).

Les séquences des amorces utilisées sont les suivantes :
N-ter/FAPP (seq id N° 1) :
   5'- TTCGTaagcttGCGAAGCTGGGCGCCGTGTACAGAA-3';
C-ter/FAPP-BSDL (seq id N° 2) :
   5'-TTTCGTgaattcACGCTAAAACCTAATGACTGCAGGCATCTG-3'.

L'amorce C-ter/FAPP-BSDL (seq id N° 2) utilisée comprend un codon de terminaison de sorte à éliminer la traduction de l'épitope c-myc ainsi que celle du peptide de six histidines que porte le vecteur commercial.

L'ADNc (nucléotides 1 à 2169) ainsi amplifié (SEQ ID No 12) ne comprend pas de peptide signal.

L'ADNc obtenu est ensuite inséré dans le plasmide pSec-Tag (Invitrogen, Leek, Pays Bas).

### Préparation 2 : Plasmide pSec-16R

L'amplification de l'ADNc de la partie C-terminale de la BSDL (du nucléotide 1089 : Phe364 au nucléotide 2169 : codon stop ; SEQ ID Nos 8 et 9) issu des ARN extraits d'un pancréas normal, a été réalisée par PCR à l'aide des amorces C-ter/FAPP-BSDL (amorce seq id N° 2, cf. Préparation 1 ) et N-ter-Ct.

La séquence de l'amorce N-ter-Ct est la suivante :
N-ter-Ct (seq id N° 3) :
   5'- CGTCTAaagcttTTTGATGTCTACACCGAGTCC-3'.

La réaction de polymérisation en chaîne a été réalisée dans les conditions déjà décrites ci-dessus (cf. Préparation 1 ).

L'ADNc obtenu a été inséré dans le plasmide pSec-Tag.

### Préparation 3 : Plasmide pSec-6R

L'amplification de l'ADNc de la partie C-terminale de la FAPP (du nucléotide 1089 : Phe364 au nucléotide 1839 : codon stop ; SEQ ID Nos 12 et 13) présent sur le plasmide pSec-FAPP a été réalisée par PCR grâce aux amorces nucléotidiques N° 2 (C-ter/FAPP-BSDL) et N° 3 (N-ter-Ct) décrites ci-dessus.

La réaction de polymérisation en chaîne a été réalisée dans les conditions de la préparation 1.

L'ADNc obtenu a été inséré dans le plasmide pSec-Tag.

### Préparation 4 : Plasmide pBK-αGT

L'amplification de l'ADNc codant pour l'α(1-3) galactosyltransférase (du nucléotide -10 au nucléotide +1122 : codon stop) issu des ARN extraits de coeur de souris (souche Balb/c) a été réalisée par PCR à l'aide des amorces Cter/αGal et Nter/αGal définies à partir de la séquence de l'ADNc codant pour l'α(1-3) galactosyltransférase de souris. La séquence de ces amorces est la suivante :
Nter/αGal (seq id N° 4) : 5'-AAAAA*gaattc*GGAGAAAATAATGAAT-3'.
Cter/αGal (seq id N° 5) : 5'-AAAAA*gggccc*ACAAAGTCAGACATTAT-3'.

La réaction de polymérisation en chaîne a été réalisée à l'aide de la trousse « Taq PCR Master Mix » (Qiagen, Courtaboeuf, France) et selon le programme suivant : un cycle de 2 minutes à 94°C suivi de trente cinq cycles d'1 minute à 94°C (dénaturation), 1 minute à 54°C (hybridation des amorces), et 2 minutes à 74°C (élongation), puis un cycle de 10 minutes à 74°C, effectués dans un thermocycleur Gene Amp PCR System 2400 (Perkin Elmer).

L'ADNc obtenu a été inséré dans le plasmide pBK-CMV (Stratagene, La Jolla CA, USA).

### Préparation 5 : Plasmide pcDNA3-C2β6GnT-flag

L'ADN complémentaire de la glycosyltransférase Core2 β(1-6) N-acétylglucosaminyltransférase décrit par Panicot et coll. dans Glycobiology, (1999) 9 ; 935-946 a été cloné dans le vecteur pCDNA-3 qui comprend à son extrémité 3' une séquence codant pour l'épitope flag. L'expression de l'épitope flag permet de détecter l'expression de la C2GnT dans les cellules transformées.

Les étapes communes aux préparations de plasmides sont les suivantes :

### a) Liguation

Les différents produits de PCR sont analysés sur un gel d'agarose à 1%. Après purification grâce à la trousse « Geneclean » (Bio 101), les produits de PCR sont sous-clonés dans le plasmide navette pCR2.1 TOPO et séquencés (Euro Séquences Gènes Service, Paris, France) grâce aux amorces universelles M13 et M13 inverses. Les produits de PCR sont alors excisés de ce plasmide navette par l'action des enzymes de restriction *Hind*III et *Eco*RI. Ces inserts sont introduits dans les plasmides de sécrétion et d'expression eucaryotes pSec-Tag afin d'obtenir les plasmides pSec-FAPP et pSec-16R (16R pour domaine C-terminal de la BSDL) (SEQ ID No 8). L'ADN complémentaire codant la partie C-terminale de la FAPP, obtenu par PCR, a été inséré directement après purification et action des enzymes de restriction *Hind*III et *Eco*RI dans le vecteur pSec-Tag pour former le plasmide pSec-6R (6R pour domaine C-terminal de la FAPP) (SEQ ID No 12). Les séquencages sont effectués avec l'amorce universelle T7 et l'amorce BGH inverse synthétisée à cet effet par Euro Séquences Gènes Service.

Le produit de PCR codant pour l'α(1-3) galactosyltransférase est inséré directement, après excision par les enzymes de restriction *Eco*RI et *Apa*I suivi d'une purification, par ligation dans le vecteur pBK-CMV préalablement ouvert par les enzymes de restriction *Eco*RI et *Apa*I pour former le plasmide pBK-αGT.

### b) Transformations bactériennes

Une aliquote du produit de ligation (5 µl) est mise en contact de 50 µl d'une suspension de cellules bactériennes (*Escherichia coli*, souche TOP10F') rendues compétentes selon le protocole établi par Hanahan [Hanahan, 1983, J. Mol. Biol., 166, 557-580]. Après l'addition de 2 µl d'une solution de β-mercaptoéthanol 0,5 M, l'échantillon est maintenu dans la glace durant 30 minutes, puis incubé à 42°C durant 30 secondes, et immédiatement replacé dans la glace. Après 2 minutes, il est dilué dans 450 µl de milieu SOC (Life Technologies). La suspension bactérienne est incubée 1 heure à 37°C sous agitation. Les bactéries sont ensuite étalées, au moyen de billes de verre, sur un milieu de Luria-Bertani agar additionné de 50 µg/ml d'ampicilline, coulé dans des boites de Pétri.

### c) Purifications plasmidiques

Les colonies bactériennes, apparues après 18 heures de culture sur un milieu gélosé sélectif (50 µg/ml d'ampicilline), sont prélevées. Chaque colonie est réensemencée dans 2 ml de milieu de Luria-Bertagni liquide, supplémenté en ampicilline (50 µg/ml). Les cultures sont incubées 8 heures à 37°C sous agitation. 1,5 ml de suspension bactérienne est alors centrifugé à 5 000 g pendant 5 minutes. Le culot bactérien est repris dans 100 µl d'un tampon déstabilisateur des membranes bactériennes (Tris-HCl 50 mM pH 8, EDTA 10 mM, Ribonucléase A 100 µg/ml). La suspension bactérienne est lysée par ajout de 200 µl d'un tampon de lyse alcaline (NaOH 200 mM, SDS 1%) et le pH de la préparation est neutralisé par 150 µl de tampon acétate de potassium 3 M, pH 5,5. Les étapes de lyse alcaline et de neutralisation nécessitent 5 minutes d'incubation dans de la glace. Les débris cellulaires, les protéines dénaturées et l'ADN chromosomique sont éliminés par centrifugation à 10 000 g pendant 10 minutes à 4°C. Le surnageant subit alors une extraction au phénol-chloroforme par dilution au 1/2 dans une solution de phénol/chloroforme/alcool isoamylique (v/v/v : 25/24/1) stabilisée à pH 8 par une solution de Tris-HCl 100 mM. Les phases aqueuse et organique sont séparées par une centrifugation à 10 000 g pendant 2 minutes à température ambiante. La phase aqueuse supérieure est récupérée et diluée au 1/3 dans de l'éthanol absolu (-20°C). L'ADN plasmidique est précipité par centrifugation à 12 000 g pendant 15 minutes à 4°C, puis lavé deux fois par une solution d'éthanol à 70%. L'ADN plasmidique est ensuite suspendu à nouveau dans 20 µl d'eau ultra-pure et conservé à -20°C.

### Préparations de Clones cellulaires

### Préparation 6 : Clone cellulaire CHO-C2 et généralités sur l'obtention des clones cellulaires intermédiaires

On a transfecté une lignée CHO-K1 par le plasmide pcDNA3-C2β6GnT-flag. Ce plasmide comprend l'ADN complémentaire de la glycosyltransférase Core2 β(1-6) N-acétylglucosaminyltransférase (C2GnT), lequel comprend à son extrémité 3' une séquence codant pour l'épitope flag. L'expression de l'épitope flag permet de détecter l'expression de la C2GnT dans les cellules transformées.

Pour cela, on a cultivé des cellules CHO-K1 sur du milieu HAM-F12. Lorsque les cellules CHO-K1 ont atteint 60 à 80% de confluence, le milieu de culture a été retiré, la surface cellulaire lavée à trois reprises avec du milieu Opti-MEM puis recouverte par 200 µl de solution de transfection diluée dans le milieu Opti-MEM sans sérum de veau foetal. La solution de transfection contient le plasmide cité ci-dessus et une suspension de liposomes appelée lipofectamine (Life Technologies).

Les cellules ont été maintenues ainsi pendant 16 heures. Le surnageant de transfection a alors été remplacé par 2 ml de milieu HAM-F12 complet additionné de l'antibiotique de sélection approprié (néomycine, zéocine ou hygromycine) durant une période d'au moins 4 semaines. Les clones résistants à l'action de l'antibiotique ont été isolés puis amplifiés.

Le clone cellulaire CHO-K1 ainsi sélectionné par la néomycine (250 µg/ml) a été nommé CHO-C2.

Ce clone CHO-C2 a ensuite été transfecté par le plasmide pSec-16R comme décrit ci-dessous à la préparation 7. Le clone résultant de la transformation de la lignée CHO-C2 par le plasmide pSEC-16R a été sélectionné par la zéocine (1 mg/ml) et nommé CHO-C2-16R.

On a de même co-transfecté la lignée CHO-K1 par le plasmide pCDM7-FUT3 codant pour la fucosyltransférase 3 décrit par Kukowska-Lallo et coll. (1990, Genes Dev. 4 ; 1288-1303), et le plasmide pMamNeo selon la technique décrite ci-dessus. Le clone CHO-F3 résultant de la transformation de la lignée CHO-K1 par le plasmide pCDM7-FUT3 a été sélectionné d'une part par la néomycine (250 µg/ml) et d'autre part pour sa forte expression en des motifs sialyl Lewis x. Cette expression a été mise en évidence par immunofluorescence indirecte à l'aide de l'anticorps CSLEX-1 (Becton-Dickinson). Le clone CHO-F3 est ainsi obtenu.

Ce clone CHO-F3 a ensuite été transfecté par le plasmide pSec-16R comme décrit ci-dessous à la préparation 7. Le clone résultant de la transformation de la lignée CHO-F3 par le plasmide pSec-16R après sélection par la zéocine (1 mg/ml) a été nommé, CHO-F3-16R.

On a de même transfecté la lignée CHO-K1 en utilisant le plasmide pCDM8-FUT7 codant pour la fucosyltransférase 7 décrit par Lowe et coll. (1991) J. Biol. Chem. 266 ; 17467-17477) au lieu du plasmide pCDM7-FUT3, puis par le plasmide pSec16R. Le clone résultant a été nommé CHO-F7-16R, après sélection par la zéocine et la néomycine dans les conditions décrites ci-dessus.

Pour la préparation des clones intermédiaires exprimant le peptide C-terminal de la FAPP (6R), on a opéré comme ci-dessus en transfectant les lignées ou clones cellulaires CHO-K1, CHO-C2 et CHO-F3 par le plasmide pSec-6R au lieu du plasmide pSec16R (voir la technique de la préparation 7). Mais seules les glycosyltransférases C2GnT et fucosyltransférase 3 essentielles pour l'élaboration de l'épitope glycosylé J28 ont été utilisées lors de la préparation des lignées cellulaires.

Trois clones cellulaires intermédiaires CHO-6R, CHO-C2-6R et CHO-F3-6R ont été ainsi obtenus après sélection par les antibiotiques dans les conditions ci-dessus décrites.

### Préparation 7 : Clone cellulaire CHO-16R

On a transfecté une lignée CHO-K1 par le plasmide pSec-16R, c'est à dire le plasmide pSec-Tag (Invitrogen, Leek, Pays Bas) dans lequel a été introduit l'ADNc codant pour le domaine C-terminal de la BSDL (voir plus loin à la Préparation 2). Pour cela, on a procédé comme pour la préparation du clone CHO-C2 de la préparation 6.

Le plasmide pSec-Tag possède une séquence codant pour la région V-J2C de la chaîne légère k des immunoglobulines de souris. Cette séquence permet de diriger la protéine codée par l'ADN complémentaire inclus dans le plasmide vers la voie de sécrétion de la cellule eucaryote dans laquelle il aura été préalablement transfecté.

Le clone résultant de la transformation de la lignée CHO-K1 par le plasmide pSec-16R a été nommé CHO-16R et a été sélectionné pour l'expression du (glyco)peptide afférant à ce clone ((glyco)peptide 16R).

### Préparation 8 : Clone cellulaire CHO-C2-F3-16R

Le clone CHO-C2 de la préparation 6 a été transfecté par le plasmide pCDM7-FUT3 ainsi que par le plasmide pLSVHg (R & D Systems) qui confère une résistance à l'hygromycine, ce qui a donné lieu au clone cellulaire CHO-C2-F3.

On a transfecté la lignée cellulaire CHO-C2-F3 par le plasmide pSec-16R comme dans la préparation 7. Le clone résultant de la transformation de la lignée CHO-C2-F3 par le plasmide pSec-16R a été nommé CHO-C2-F3-16R.

Le clone CHO-C2-F3-16R exprime les motifs sialyl Lewis x et présente une réactivité à l'anticorps pAbL64.

### Préparation 9 : Clone cellulaire CHO-C2-F7-16R

On a opéré comme à la préparation 8, mais en utilisant le plasmide pCDM8-FUT7 au lieu du plasmide pCDM7-FUT3.

Le clone résultant de la transformation de la lignée CHO-C2-F7 par le plasmide pSec-16R a été nommé CHO-C2-F7-16R et la lignée cellulaire correspondante a été déposée à la Collection Nationale de Culture de Microorganismes (CNCM) à Paris le le 16 mars 2004 sous le N° I-3189.

Les clones CHO-C2-F3-16R et CHO-C2-F7-16R des préparations 8 et 9 expriment les motifs sialyl Lewis x et présentent une réactivité à l'anticorps pAbL64.

### Préparation 10 : Clone cellulaire CHO-C2-F3-6R

Le clone CHO-C2-F3 de la préparation 8 a été transfecté par le plasmide pSec-6R (Voir la préparation 3) comme décrit pour les préparations 6 et 7. Le clone CHO-C2-F3-6R est ainsi obtenu. Le peptide exprimé par ce clone porte entre autre l'épitope glycosylé reconnu par l'anticorps monoclonal J28.

### Préparation 11 : Clone cellulaire CHO-C2-F3-GT-6R

Le clone CHO-C2-F3-6R de la préparation 10 a été transfecté par le plasmide pBK-αGT (Voir la préparation 4) comme décrit ci-dessus. Le clone CHO-C2-F3-GT-6R ainsi obtenu porte l'épitope glycosylé reconnu par l'anticorps monoclonal J28 ainsi que l'épitope glycosylé αGal reconnu par les anticorps naturels présents dans la circulation sanguine humaine.

### Exemple 1 : Glycopeptide recombinant avant la taille et les caractéristiques de ceux correspondant à l'extrémité C-terminale de la BSDL (CHO-16R)

Le glycopeptide recombinant a été produit en cultivant durant 16 heures à 37°C sous atmosphère humide et en présence de 5 % de CO₂ le clone cellulaire CHO-16R (Préparation 7) de façon routinière dans du milieu Opti-MEM (Invitrogen), avec ajout de L-glutamine (2 mM) pénicilline (100 U/ml) streptomycine (100 mg/ml) et fongizone (0,1 %) additionnés des antibiotiques de sélection adéquats. Les cellules ont été ensemencées à la densité de 2x10 ⁴ cellules/cm².

Le milieu de culture a été renouvelé toutes les 48 heures. Arrivées à confluence, les cellules ont été lavées avec un tampon phosphate (PBS) sans CaCl₂ et sans MgCl₂, puis décollées par une solution de trypsine/EDTA (0,05 %), sédimentées par centrifugation à basse vitesse (400 g pendant 3 min) et suspendues à nouveau dans 5 ml de milieu de culture.

L'expression du glycopeptide recombinant d'intérêt par les clones cellulaires a été vérifiée par immunofluorescence intracellulaire et cytométrie de flux.

Le glycopeptide recombinant obtenu a été purifié par chromatographie liquide suivie d'une chromatographie d'affinité et analysé en SDS-Page.

On observe sur le gel que le peptide sécrété se répartit en deux bandes de poids moléculaires distincts soit environ 78 KDa et 83KDa.

La forme à 83 KDa correspond probablement à la forme glycosylée du peptide tandis que la forme à 78 KDa correspondrait soit à une forme non glycosylée soit à une forme dont la glycosylation serait moins importante.

Il apparaît donc que le clone ci-dessus exprime un glycopeptide ayant la taille et les caractéristiques de ceux correspondant à l'extrémité C-terminale de la BSDL.

### Exemple 2 : Glycopeptide recombinant avant la taille est les caractéristiques de ceux correspondant à l'extrémité C-terminale de la BSDL (CHO-C2-16R)

On a opéré comme à l'exemple 1, mais en cultivant le clone cellulaire CHO-C2-16R (Préparation 6).

Le glycopeptide recombinant obtenu a été purifié par chromatographie liquide suivie d'une chromatographie d'affinité et analysé en SDS-Page.

On observe sur le gel que le peptide sécrété se répartit en deux bandes de poids moléculaires distincts soit environ 78 KDa et 83KDa.

Il apparaît donc que le clone ci-dessus exprime un glycopeptide ayant la taille et les caractéristiques de ceux correspondant à l'extrémité C-terminale de la BSDL.

### Exemple 3 : Glycopeptide recombinant ayant la taille et les caractéristiques de ceux correspondant à l'extrémité C-terminale de la BSDL (CHO-F3-16R)

On a opéré comme à l'exemple 1, mais en cultivant le clone cellulaire CHO-F3-16R (Préparation 6).

Le glycopeptide recombinant obtenu a été purifié par chromatographie liquide suivie d'une chromatographie d'affinité et analysé en SDS-Page.

On observe sur le gel que le peptide sécrété se répartit en deux bandes de poids moléculaires distincts soit environ 78 KDa et 83KDa.

Il apparaît donc que le clone ci-dessus exprime un glycopeptide ayant la taille et les caractéristiques de ceux correspondant à l'extrémité C-terminale de la BSDL.

### Exemple 4 : Glycopeptide recombinant avant la taille et les caractéristiques de ceux correspondant à l'extrémité C-terminale de la BSDL (CHO-C2-F3-16R)

On a opéré comme à l'exemple 1, mais en cultivant le clone cellulaire CHO-C2-F3-16R (Préparation 8).

Le glycopeptide recombinant obtenu a été purifié par chromatographie liquide suivie d'une chromatographie d'affinité et analysé en SDS-Page.

On observe sur le gel que le peptide sécrété se répartit en deux bandes de poids moléculaires distincts soit environ 78 KDa et 83KDa.

Il apparaît donc que le clone ci-dessus exprime un glycopeptide ayant la taille et les caractéristiques de ceux correspondant à l'extrémité C-terminale de la BSDL.

### Exemple 5 : Glycopeptide recombinant avant la taille et les caractéristiques de ceux correspondant à l'extrémité C-terminale de la BSDL (CHO-F7-16R)

On a opéré comme à l'exemple 1, mais en cultivant le clone cellulaire CHO-F7-16R (Préparation 6).

Le glycopeptide recombinant obtenu a été purifié par chromatographie liquide suivie d'une chromatographie d'affinité et analysé en SDS-Page.

On n'observe sur le gel qu'une bande de poids moléculaire 83 KDa.

### Exemple 6 : Glycopeptide recombinant avant la taille et les caractéristiques de ceux correspondant à l'extrémité C-terminale de la BSDL (CHO-C2-F7-16R)

On a opéré comme à l'exemple 1, mais en cultivant le clone cellulaire CHO-C2-F7-16R (Préparation 9).

Le glycopeptide recombinant obtenu a été purifié par chromatographie liquide suivie d'une chromatographie d'affinité et analysé en SDS-Page.

On observe sur le gel que le peptide sécrété se répartit en deux bandes de poids moléculaires distincts soit environ 78 KDa et 83KDa.

Il apparaît donc que le clone ci-dessus exprime un glycopeptide ayant la taille et les caractéristiques de ceux correspondant à l'extrémité C-terminale de la BSDL.

Il apparaît donc que tous les clones (16R) ci-dessus expriment des glycopeptides ayant la taille et les caractéristiques de ceux correspondant à l'extrémité C-terminale de la BSDL.

### Exemples 7 à 13 : Glycopeptides recombinants avant la taille et les caractéristiques de ceux correspondant à l'extrémité C-terminale de la FAPP

Les polypeptides recombinants produits par les clones CHO-K1 (témoin), CHO-6R (Ex 7), CHO-C2-6R (Ex 8), CHO-F3-6R (Ex 9), CHO-C2-F3-6R (Ex 10), CHO-GT-6R (Ex 11), CHO-C2-GT-6R (Ex 12) et CHO-C2-F3-GT-6R (Ex 13), ont été purifiés sur gel de polyacrylamide ou bien par chromatographie liquide et/ou d'affinité et ont été analysés par immuno détection en utilisant les anticorps pAbL64 décrit par Abouakil et coll. 1988, Biochim. Biophys. Acta, 961 : 299-308, et mAbJ28. L'anticorps monoclonal de souris mAbJ28, spécifique du glycopeptide fucosylé J28 porté par la O-glycosylation de la forme oncofoetale de FAPP, a été décrit par Panicot et coll. dans Glycobiology (1999), 9, 935-946.

L'anticorps polyclonal pAbL64 dirigé contre la BSDL a été obtenu par immunisation de lapins après purification de l'antigène à partir du suc pancréatique humain. Cet anticorps reconnaît parfaitement la forme oncofoetale de la BSDL, appelée FAPP.

L'anticorps polyclonal spécifique du noyau protéique de la BSDL nommé antipeptide a été obtenu par immunisation de lapins avec un peptide synthétique correspondant à la séquence peptidique de la BSDL comprise entre les résidus de sérine en position 346 et glutamine en position 370 couplé à l'hémocyanine.

Ces deux anticorps ont été purifiés sur une colonne de protéine A couplée à du Sepharose 4B (Pharmacia). L'antipeptide a subi une seconde étape de purification par chromatographie d'affinité sur une colonne de Keyhole lampey hemocyanin (KLH) greffée sur un gel de Sepharose pour éliminer les anticorps dirigés contre la molécule KLH.

Les peptides recombinants produits par les différents clones de type 6R ont été disposés à raison de 100 µg par puit sur un gel de polyacrylamide à 10%, séparés puis électrotransférés sur membrane de nitrocellulose. Les membranes ont été ensuite incubées en présence d'une part, de l'anticorps pAbL64 et d'autre part, de l'anticorps mAbJ28 puis révélées.

Dans les deux cas, les anticorps utilisés se lient spécifiquement avec des polypeptides de taille 50 KDa, correspondant à la taille attendue des glycopeptides C-terminaux de la FAPP, sauf dans le cas de la lignée CHO-K1 non transfectée, servant de témoin négatif.

### Exemples 14 à 19 : Anticorps monoclonaux anti-glycopeptides

On a préparé des anticorps anti-glycopeptides recombinants ayant la taille et les caractéristiques de ceux correspondant à l'extrémité C-terminale de la FAPP comme suit :

### Stade A - Immunisation des souris.

Des souris Balb/c mâles, âgées de 6 semaines sont immunisées selon le programme suivant :
Jour 0 : Injection par voie intrapéritonéale de 25 µg d'un mélange de FAPP et de BSDL purifiées à partir de sucs pancréatiques humains normaux et pathologiques (appelé ci-après antigène BSDL-FAPP) dans une émulsion 50/50 (150 µl NaCl / 150 µl adjuvant de Freund complet).
Jour 15 : Rappel par voie intrapéritonéale avec 25 µg d'antigène BSDL-FAPP dans une émulsion 50/50 (150 µl NaCl / 150 µl adjuvant de Freund incomplet).
   - Jour 30 : Rappel par voie intrapéritonéale avec 25 µg d'antigène BSDL-FAPP dans une émulsion 50/50 (150 µl NaCl / 150 µl adjuvant de Freund incomplet).
   - Jour 110 : Rappel par voie intrapéritonéale avec 20 µg d'antigène BSDL-FAPP dans une émulsion 50/50 (150 µl NaCl / 150 µl adjuvant de Freund incomplet).
   - Jour 140 : Rappel par voie intrapéritonéale avec 20 µg d'antigène BSDL-FAPP dans une émulsion 50/50 (150 µl NaCl / 150 µl adjuvant de Freund incomplet).
   - Jour 215 : Rappel par voie intrapéritonéale avec 20 µg d'antigène BSDL-FAPP dans une émulsion 50/50 (150 µl NaCl / 150 µl adjuvant de Freund incomplet).
   - Jour 244 : Injection par voie intraveineuse de 10 µg d'antigène BSDL-FAPP dans 100
      µl de NaCl stérile.
   - Jour 247 : Fusion cellulaire.

### Stade B - Fusion cellulaire selon le protocole de Köhler et Milstein.

a) Au jour 247, la souris sélectionnée est sacrifiée et sa rate prélevée et dilacérée. Les cellules spléniques sont lavées dans du milieu RPMI 1640. Les cellules de myélome P3.X63.Ag8 653, préalablement cultivées en RPMI 20% sérum de veau foetal (SVF), 1% glutamine, 1% acides aminés non essentiels et 1% pyruvate de sodium sont aussi lavées dans le même milieu.
   Parallèlement, des macrophages péritonéaux sont obtenus par lavage du péritoine de souris Balb/c non immunisées avec du RPMI.
   Pour obtenir la formation d'hybridomes, les splénocytes et les cellules de myélome sont mélangées dans un tube à raison de 5 cellules de rate pour 1 cellule de myélome. Après centrifugation, le culot cellulaire est remis en suspension dans 800 µl de polyéthylène glycol 1500 à 50% en tampon Hepes 75 mM pH 7,5. Après un contact de 1 minute à 37°C, 20 ml de milieu RPMI 1640 sont rajoutés lentement aux cellules fusionnées.
b) La culture initiale est réalisée sur plaques de microculture 96 puits en présence de milieu RPMI contenant 20% de sérum de veau foetal (SVF) et les additifs suivants : hypoxanthine 5x10⁻³ M, aminoptérine 2x10⁻⁵ M et thymidine 8x10⁻⁴ M. Dans chaque puits sont ajoutés 5x10³ macrophages péritonéaux puis 10⁵ cellules fusionnées.

### Stade C - Clonages et sous-clonages.

Chaque hybridome sélectionné suivant la technique citée au stade D ci-après, résulte d'un clonage par une technique en dilution limite dans laquelle 10, 5, 2, 1 et 0,5 cellules sont réparties statistiquement dans des micropuits contenant des macrophages péritonéaux. Deux sous-clonages sont ainsi réalisés, chaque clone et sous-clone ayant été répliqué puis congelé dans 90% de SVF et 10% de diméthylsulfoxyde (DMSO). Les sous-clones de dernière génération font enfin l'objet d'une expansion *in vivo* pour l'obtention de liquide d'ascite dans des souris Balb/c, suivie d'une purification des immunoglobulines sur protéine A.

### Stade D - Technique de sélection des cellules hybrides.

La sélection est effectuée par méthode ELISA en phase liquide à partir du surnageant de culture. 5 µg d'antigène BSDL-FAPP dans 100 µl d'un tampon bicarbonate pH 8,5 sont déposés dans les puits d'une plaque ELISA 96 puits et la plaque est activée pendant 12 h à 4 °C et saturée pendant deux heures avec 300 µl d'albumine bovine à 1 mg/ml. Puis les plaques sont lavées et incubées avec 100 µl de surnageants de culture cellulaire contenant éventuellement des anticorps dirigés contre l'antigène BSDL-FAPP. Après deux heures d'incubation, les plaques sont lavées et incubées pendant deux heures avec des anticorps secondaires marqués à la phosphatase alcaline. A la fin de l'incubation, les plaques sont de nouveau lavées et incubées avec du para-nitrophényl phosphate (100 µl, 1 mg/ml dans du tampon Tris/HCl 0,2 M pH 8,2 et CaCl2 1 mM). Après une heure à 37 °C, les plaques sont lues avec un lecteur de microplaques à 410 nm.

Une quinzaine d'anticorps a été sélectionnée pour leur réponse en ELISA. Des analyses complémentaires réalisées par Western-blot (immuno-empreinte) en utilisant la FAPP comme protéine immunogène ont permis la sélection des hybridomes 7B4 (Exemple 14), 11D7 (Exemple 15), 14H9 (Exemple 16), 14H10 (Exemple 17), 16D10 (Exemple 18), et 8H8 (Exemple 19).

Des cellules produisant l'anticorps 16D10 de type IgM ont été déposées à la Collection Nationale de Culture de Microorganismes (CNCM) à Paris le 16 mars 2004 sous le N° I-3188.

### Exemple 20. Préparation de glycopeptides membranaires d'origine naturelle

On a préparé des glycopeptides membranaires portant les épitopes reconnus par les anticorps monoclonaux des exemples 14 à 19 de la manière suivante : des cellules pancréatiques tumorales sont cultivées sur un support en matière plastique puis décollées du support par une solution de dissociation non enzymatique (Sigma). Le culot cellulaire obtenu par centrifugation (2 min, 1000 tours par min) est alors soumis à une sonification (2X15 sec) puis centrifugé de nouveau (20 min, 14 000 tours par min, 4°C). Le culot solubilisé dans du tampon phosphate correspond aux glycopeptides membranaires portant les épitopes reconnus par les anticorps monoclonaux des exemples 14 à 19.

### Exemple 21.

On a préparé un vaccin ayant la composition suivante :
- glycopeptides membranaires de l'exemple 20 isolés à partir de cellules pancréatiques HaPT-1, 20 µg
- Adjuvant ALU-gel-ser (laboratoires Serva) 150 µl
- Excipient dont eau pour préparations injectables 150 µl

La vaccination est pratiquée par injection intra-péritonéale 2 semaines puis une semaine avant transplantation de cellules tumorales dans le flanc des animaux. Le résultat de cette vaccination est obtenu par comparaison avec un placébo (solution isotonique injectable).

### Exemple 22.

On a préparé une solution isotonique injectable d'anticorps de l'exemple 18 (16D10) purifiés de 0,5 mg/ml.

### Exemple Témoin 1 : Glycopeptide recombinant avant la taille et les caractéristiques de ceux correspondant à l'extrémité C-terminale de la BSDL

On a opéré comme à l'exemple 1, mais en cultivant le clone cellulaire CHO-K1.

On n'observe sur le gel aucune bande de poids moléculaire de 78 KDa ou 83 KDa.

### ETUDE PHARMACOLOGIQUE

### Expérimentation 1 : Utilisation des glycopeptides recombinants C-terminaux de la BSDL et de la FAPP en immuno-thérapie cellulaire des cancers du pancréas exocrine

### Protocole opératoire :

Plusieurs groupes de hamsters (90-100 g) ont été constitués, puis vaccinés avec des cellules HaPT-1 inactivées par 30 minutes d'exposition aux UV. Les glycopeptides de l'exemple 20 et les cellules inactivées mélangés à l'adjuvant ALU-gel-ser, sont inoculés aux hamsters par injection intra péritonéale, une fois par semaine pendant deux semaines. Deux semaines après la dernière inoculation, les hamsters sont transplantés avec des cellules HaPT-1 de manière ectotopique (sous-cutanée, sur le flanc de l'animal).

L'évolution des tumeurs est suivie hebdomadairement, leurs volumes sont comparés au groupe contrôle vacciné avec un placebo (PBS seul).

### Résultats :

On observe que la courbe de croissance des tumeurs est significativement ralentie chez les hamsters immunisés avec les glycopeptides de l'exemple 20 (antigènes représentés en grande partie par des structures reconnues par les anticorps monoclonaux J28 et 16D10 (Exemple 18), dirigés contre le domaine C-terminal de la BSDL et/ou de la FAPP).

A la fin de l'expérimentation, les volumes tumoraux du groupe vacciné avec les cellules inactivées sont réduits de 80 % par rapport au groupe contrôle.

Les hamsters ont été euthanasiés, des prélèvements de tissus tumoraux et pancréatiques ont été réalisés et analysés par immunohistochimie. L'expression des épitopes 16D10 et J28 (reconnus par les anticorps monoclonaux J28 et 16D10, respectivement) ainsi que du core protéique de la FAPP est recherchée en utilisant les anticorps monoclonaux J28, 16D10 et polyclonaux L64.

L'analyse des coupes tissulaires des tumeurs révèle une diminution de l'expression des épitopes 16D10 et J28 (diminution plus faible pour ce dernier), entre le groupe vacciné avec les cellules inactivées et le groupe contrôle.

En conséquence, les glycopeptides C-terminaux de la BSDL et de la FAPP tels que décrits ici peuvent être utilisés en immunothérapie cellulaire des cancers du pancréas exocrine.

### Expérimentation 2 : Utilisation des glycopeptides tels que décrits ici dans le diagnostic des pathologies diabétiques

### Protocole opératoire :

Une série d'expériences réalisée à l'aide de la technique ELISA sur des sérums de patients atteints de diverses pathologies pancréatiques et autres, a permis de montrer que la BSDL utilisée comme antigène pour activer les plaques ELISA était spécifiquement reconnue par des anticorps présents dans environ 80 % des sérums provenant de patients affectés d'un diabète de type I alors que moins de 10 % des sérums des patients témoins sont réactifs. Les sérums des patients atteints d'autres pathologies telles que pancréatites, cancers du pancréas ou maladie de Graves ne sont pas réactifs (Panicot et coll. 1999, Diabetes, 48 : 2316-2323).

On a testé les glycopeptides recombinants des exemples 2, 5 et 6 par la technique du Western-Blot (ou immuno-empreinte).

### Résultats :

La BSDL, et notamment son domaine C-terminal, est reconnu par les sérums des patients diabétiques alors que la BSDL n'est pas reconnue par les sérums des patients normaux contrôles.

Les glycopeptides recombinants des exemples 2 et 6 sont reconnus par les sérums des patients diabétiques (type I).

Les sérums contrôles (prélevés chez des patients sains) ne reconnaissent aucun de ces trois glycopeptides.

En conséquence, les glycopeptides naturels et recombinants C-terminaux décrits dans les exemples donnés peuvent être utilisés pour le diagnostic des pathologies diabétiques.

### Expérimentation 3 : Utilisation des anticorps monoclonaux dirigés contre le domaine C-terminal de la FAPP et/ou de la BSDL dans le diagnostic des cancers du pancréas

### Protocole opératoire :

Des lignées cellulaires tumorales pancréatiques humaines SOJ-6 et Bx-PC-3 et tumorales humaines non pancréatiques Caco-2 et Hep-G2 ont été traitées avec les anticorps monoclonaux J28, 16D10 (Exemple 18), et 8H8 (Exemple 19) ainsi que l'anticorps polyclonal (pAb) L64 spécifiques du domaine C-terminal de la BSDL et de la FAPP puis les complexes antigènes-anticorps ont été détectés à l'aide d'un anticorps fluorescent anti-IgG de lapin et de souris respectivement couplés au N-isothiocyanate de fluorescéïne. Le tout est ensuite analysé par FACS afin de détecter la présence des antigènes associés au domaine C-terminal de la FAPP et de la BSDL à leur surface.

### Résultats :

Les anticorps monoclonaux J28 et 16D10 (Exemple 18), ainsi que l'anticorps polyclonal (pAb) L64 reconnaissent spécifiquement la surface des lignées cellulaires, tumorales pancréatiques humaines SOJ-6 et Bx-PC-3 et ne reconnaissent pas la surface des lignées tumorales humaines non pancréatiques testées telles les cellules Caco-2 et Hep-G2.

Les anticorps monoclonaux J28 et 16D10 dirigés contre des épitbpes glycosylés exprimés spécifiquement sur le domaine C-terminal de la FAPP et de la BSDL sont donc capables de distinguer les cellules tumorales pancréatiques des cellules tumorales non-pancréatiques.

En conséquence les anticorps monoclonaux dirigés contre des épitopes glycosylés exprimés spécifiquement sur le domaine C-terminal de la FAPP et de la BSDL peuvent être utilisés dans le diagnostic des cancers du pancréas.

En particulier ces anticorps peuvent définir si un foyer tumoral secondaire (métastase) est d'origine pancréatique ou issu d'un foyer primaire de toute autre origine.

### Expérimentation 4 : Ciblage des cellules néoplasiques pancréatiques par les anticorps monoclonaux de l'invention

### A- Etude sur coupes de tissus pancréatiques humains.

### Protocole opératoire:

L'expression tissulaire des épitopes glycosylés reconnus par les anticorps monoclonaux dirigés contre le domaine C-terminal de la FAPP et/ou de la BSDL a été testée par des expériences d'immunohistochimie réalisées à l'aide de divers anticorps monoclonaux de l'invention sur des coupes de tissus pancréatiques humain, cancéreux et normaux.

Dans un premier temps, on a prélevé et réalisé des coupes de tissu pancréatique de 4 malades atteints d'un cancer du pancréas ainsi que 5 tissus pancréatiques normaux. Les coupes sont alors mises à incuber avec les anticorps des exemples 18 (16D10) et 19 (8H8).

Dans un deuxième temps, sept tissus pancréatiques humains tumoraux et cinq tissus non tumoraux (diagnostic effectué par un médecin anatomo-pathologiste) ont été coupés en sections de 5 µm d'épaisseur, séchés dans l'acétone à 4°C et réhydratés dans un tampon Tris/HCl à pH 7.6. Les sections ont ensuite été incubées soit 1 h à 25°C (mAbJ28 et mAb8H8, exemple 19) soit une nuit à 4°C (mAb16D10, exemple 18), les anticorps étant dilués au 1/50 dans le diluant Dako (EnVision System, Dako, Coppenhage, Danemark). Les coupes ont ensuite été incubées avec un anticorps secondaire adéquat marqué à la phosphatase alcaline. Le complexe antigène-anticorps a ensuite été détecté par le substrat chromogène Fast-Red. Dans le cas d'une analyse quantitative confocale « Confocal Laser Scanning Microscopy », l'anticorps secondaire adéquat était marqué à la Streptavidiné-fluorosceine. Les coupes ont finalement été examinées soit avec un microscope soit avec un microscope confocal à fluorescence lasser.

### Résultats:

Dans la première expérience, on a observé que l'anticorps monoclonal de l'exemple 18 (16D10) reconnaît spécifiquement les cellules néoplasiques issues des 4 tissus pancréatiques cancéreux.

Aucune réaction n'avait été observée avec les cinq tissus pancréatiques normaux.

L'anticorps monoclonal de l'exemple 19 (8H8) reconnaîssait préférentiellement les tissus pancréatiques normaux.

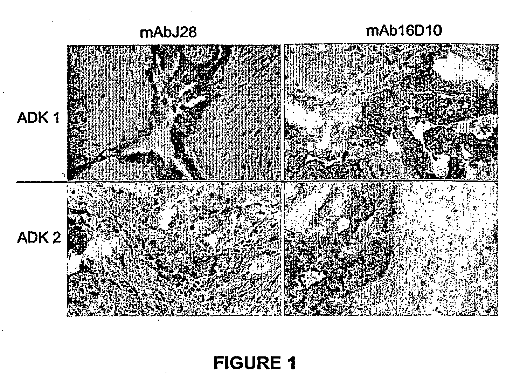

Dans la deuxième expérience, on a observé que, sur l'ensemble des sept tissus cancéreux étudiés, 7 ont été reconnus par le mAb16D10 (exemple 18) et cinq ont été reconnus par le mAbJ28. Les résultats d'immunohistochimie sont présentés sur la Figure 1 et sur les Tableaux 1 et 2. Le mAb16D10 (exemple 18) reconnaît de façon très spécifique les tissus tumoraux tandis que le mAb8H8 (exemple 19) ne reconnaît que les tissus pancréatiques normaux. Le mAbJ28 a une position intermédiaire puisqu'il reconnaît préférentiellement les tissus tumoraux, mais aussi certains tissus normaux. Le tableau 2 donne les résultats obtenus par microscopie confocale laser définissant l'intensité de la fluorescence sur les coupes après marquage par les anticorps. A noter qu'aucun de ces anticorps ne reconnaît les autres tissus tumoraux testés : foie, poumon, estomac, colon, oesophage et thyroïde.

Les anticorps monoclonaux de l'invention couplés à une substance active anti-tumorale peuvent être utilisés dans le traitement des cancers pancréatiques pour cibler et détruire les cellules tumorales pancréatiques.

De même des éléments radioactifs (ou autres) peuvent être couplés à ces anticorps et permettre une localisation précise des tumeurs primaires et des métastases. Lesdits anticorps peuvent en outre permettre une distinction claire et nette du tissu normal versus le tissu tumoral.

**Table 1. Etudes immunohistochimique semi-quantitative. Résultats obtenus avec les anticorps mAb8H8, mAbJ28 et mAb16D10**

| Marquage | | | |
|---|---|---|---|
| | mAb8H8 | mAbJ28 | mAb16D10 |
| Contrôle1 | | | |
| Acini | 4 | 1 | 0 |
| Canaux | 0 | 0 | 0 |
| | | | |
| Contrôle 2 | | | |
| Acini | 4 | 1 | 0 |
| Canaux | 0 | 0 | 0 |
| | | | |
| Contrôle 3 | | | |
| Acini | 4 | 1 | 0 |
| Canaux | 0 | 0 | 0 |
| | | | |
| Contrôle 4 | | | |
| Acini | 4 | 1 | 0 |
| Canaux | 0 | 0 | 0 |
| | | | |
| Contrôle 5 | | | |
| Acini | 4 | 1 | 0 |
| Canaux | 0 | 0 | 0 |
| | | | |
| PDAC1 | 0 | | 4 |
| PDAC2 | 0 | 3 | 4 |
| PDAC3 | 0 | 2 | 4 |
| PDAC4 | 0 | 0 | 4 |
| PDAC5 | 1 | 3 | 4 |
| PDAC6 | 0 | 2 | 4 |
| PDAC7* | 0 | 1 | 3 |
| | | | |
| CCA* | 0 | 0 | 0 |
| ECA* | 0 | 0 | 0 |
| | | | |
| LiCa* | 0 | 0 | 0 |
| LuCa | 0 | 0 | 0 |
| SCA* | 0 | 0 | 0 |
| TCA* | 0 | 0 | 0 |

| | | | |
|---|---|---|---|
| C, contrôle; PDAC, adenocarcinome pancréatique; LuCA, carcinome des poumons CCA, carcinome colique; ECA, carcinome de l'oesophage; LiCa, carcinome hépatique; SCA, carcinome stomacal; TCA, carcinome thyroïdien. Tissus obtenus auprès du BioChain Institute (USA). Le nombre de cellules marquées est exprimé de façon semi-quantitative comme suit : 0, aucun marquage; 1 de 1 à 10% de cellules marquées; 2 de 10 à 30% de cellules marquées; 3 de 30 à 40% de cellules marquées et 4 plus de 40% de cellules marquées. | | | |

**Table 2. Intensité de la fluorescence sur coupes marquées avec les anticorps de l'invention.**

| Cas | Intensité de la fluorescence | | |
|---|---|---|---|
| | mAb 8H8 | mAb J28 | mAb 16D10 |
| Contrôle 1 | 92.33 ± 2.80 | 19.11 ± 8.75 | 31.41 ± 1.19 |
| Contrôle 2 | 138.72 ± 3.36 | 14.54 ± 6.57 | 8.16 ± 5.16 |
| Contrôle 3 | 110.95 ± 2.71 | 17.41 ± 8.09 | 0 |
| Contrôle 4 | 109.04 ± 4.58 | 26.49 ± 12.01 | 0 |
| Contrôle 5 | 114.14 ± 1.26 | 35.56 ± 16.30 | 0 |
| PDAC1 | 0 | 53.12 ± 2.12 | 124.02 ± 2.43 |
| PDAC 2 | 36.47 ± 0.35 | 87.88 ± 4.28 | 129.84 ± 6.35 |
| PDAC 3 | 0 | 89.60 ± 2.84 | 123.27 ± 6.16 |
| PDAC 4 | 17.06 ±7.64 | 50.61 ± 2.36 | 143.95 ± 2.71 |
| PDAC15 | 40.42 ± 0.22 | 88.92 ± 5.48 | 135.06 ± 4.03 |
| PDAC 6 | 0 | 59.97 ± 1.64 | 129.30 ± 1.91 |
| PDAC 7* | 18-65 ± 8.38 | 72.74 ± 4.84 | 132.34 ± 2.75 |

| | | | |
|---|---|---|---|
| C, contrôle; PDAC, adenocarcinome pancréatique. Tissus obtenus auprès du BioChain Institute (USA). Pour chaque cas la moyenne ± l'erreur standard des marquages de six aires différentes sur chaque coupe de tissus est utilisée. Les différences sont significatives entre les contrôles et les cancers du pancréas avec le mAb16D10 (*P*<0,001) le mAb 8H8 (*P* < 0.001) et le mAb J28 (*P* < 0.05). | | | |

### B- Etudes par compétition entre le mAbJ28 et le mAb16D10 (exemple 18) sur le glycopeptide recombinant portant le glycotope J28 par ELISA.

### Protocole opératoire :

Les glycosyltransférases impliquées dans l'élaboration du glycotope oncofoetal J28 ont été caractérisée et ce glycotope a été reproduit *ex vivo* par génie génétique. Celui-ci a été reconstitué dans les cellules CHO à partir du peptide C-terminal recombinant de la FAPP (constitués de 6 séquences répétées) et de deux glycosyltransférases, l'α(1-3/4) fucosyltransférase FUT3 et la Core 2 β(1-6) N-acétylglucosaminyltransférase nécessaires à l'établissement de cette structure. Le glycopeptide C-terminal recombinant de la FAPP portant le glycotope J28 (exemple 10) est sécrété dans le surnageant de culture de cette lignée cellulaire.

Des expériences de compétitions ont été réalisées à-l'aide d'un test ELISA en présence de surnageant de culture contenant le glycopeptide C-terminal recombinant de la FAPP portant le glycotope J28 (exemple 10), du mAbJ28 biotinylé et de concentrations croissantes de mAb16D10 (exemple 18) compétiteur dans le but de mettre en évidence la spécificité de ces anticorps.

Le principe du protocole est le suivant :
1- Le surnageant de culture concentré contenant le glycopeptide J28 recombinant dilué au 1/10, 1/20,1/50, 1/100ème est fixé au fond des puits.
2- Le mAbJ28-biotinylé est ajouté à 10ng/puits (Exp.1) ou 5ng/puits (Exp.2) associé à des concentrations croissantes de mAb16D10 purifié.
3- La reconnaissance du mAbJ28-biotinylé est réalisée à l'aide d'un anticorps Ab anti-biotine couplé à la phosphatase alcaline (contrôle Ab anti-biotine sur mAb16D10 =0).

### Résultats :

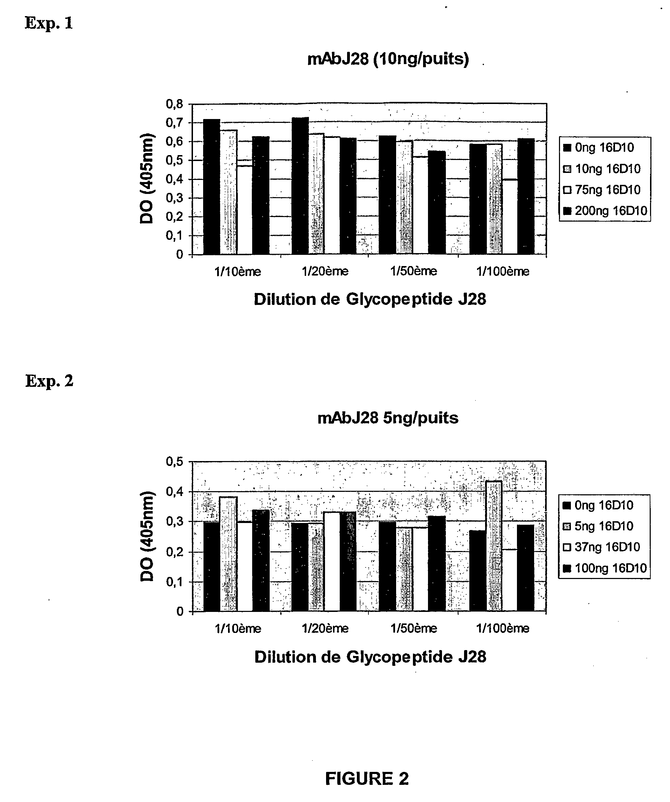

La figure 2, représentant le résultat des 2 expériences, ne montre pas une perte significative de la réactivité du mAbJ28 pour son glycotope fixé au fond des puits en présence de concentrations croissantes de mAb16D10 (exemple 18) compétiteur (même pour un excès de 4 fois).

### C- Compétition entre l'anticorps mAbJ28 et l'anticorps mAb16D10 sur la lignée cellulaire pancréatique humaine cancéreuse SOJ-6 par FACS

### Protocole opératoire :

Les anticorps monoclonaux mAb J28 et 16D10 reconnaissent spécifiquement la surface des lignées cellulaires tumorales pancréatiques humaines SOJ-6, Bx-PC-3 et ne reconnaissent pas la surface des lignées tumorales humaines non pancréatiques testées.

Des expériences préliminaires de compétitions ont été réalisées sur la lignée cellulaire pancréatique humaine cancéreuse SOJ-6 par FACS en présence d'anticorps monoclonal mAbJ28 couplé à un fluorochrome (Alexa 488) et de concentrations croissantes d'anticorps monoclonal mAb 16D10 (exemple 18) compétiteur sous forme de liquide d'ascite.

Le principe du protocole est le suivant :
1- Les cellules pancréatiques humaines cancéreuses SOJ-6 sont décollées, fixées, et saturées en BSA à 4°C.
2- L'anticorps mAb J28-fluorescent est ajouté à 2ng/échantillons en présence de concentrations croissantes de liquide d'ascite contenant l'anticorps mAb16D10 au 1/100^{ème}, 1/40^{ème}, 1/20^{ème}, 1/20^{ème}, 1/10^{ème}, 1/4 et 1/2.
3- Les cellules sont analysées par FACS.

### Résultats :

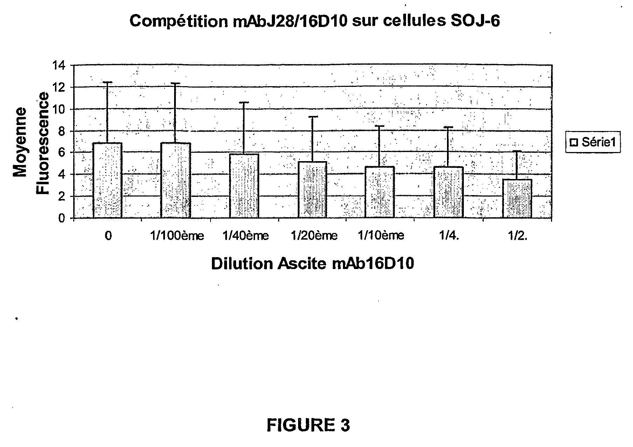

Les résultats sont représentés dans la figure 3. Malgré un marquage très faible des cellules (due aux 1^{er} essais avec le mAb J28 marqué avec l'Alexa 488), l'expérience ne montre pas une perte significative de la réactivité de l'anticorps mAbJ28 pour son glycotope en présence de concentrations croissantes de l'anticorps mAb16D10 compétiteur.

### Expérimentation 5 : Utilisation des anticorps monoclonaux de l'invention dans le traitement des cancers du pancréas exocrine

### Protocole expérimental :

Deux groupes de souris nude (souris NMRI) âgées de six semaines ont été inoculées par voie intra-péritonéale au Jour - 1 soit avec l'anticorps monoclonal 16D10 de l'exemple 18 (à raison de 150 µl d'une solution isotonique d'anticorps à 0,5 mg/ml) soit avec une solution isotonique placebo.

Au jour 0, 2 x10⁶ cellules tumorales de la lignée pancréatique humaine SOJ-6 ont été injectées par voie sous-cutanée dans le flanc droit de l'animal. Les inoculations de l'anticorps monoclonal 16D10 ou de solution isotonique ont été ensuite effectuées aux jours +1, +3, +6, +8 et +10 (selon les quantités données ci-dessus).

Le volume des tumeurs sous-cutanées palpables a été mesuré pour chaque animal aux semaines 1, 2, 3, 4 ,5 ,6 ,7 ,8, 9 et 10.

### Résultats :

On a observé que la vitesse de croissance des tumeurs chez les animaux traités avec l'anticorps monoclonal de l'exemple 18 était 10 fois inférieure à celle des animaux traités avec le placebo.

A la fin de l'expérience (10 semaines post-inoculation), la taille médiane des tumeurs prélevées sur les animaux témoins était de 1,25 cm³ tandis que la taille des tumeurs prélevées sur les animaux traités était nettement inférieure et s'établissait autour d'une médiane de 0,15 cm³.

Les anticorps monoclonaux dirigés contre le domaine C-terminal de la FAPP et/ou de la BSDL et en particulier l'anticorps monoclonal de l'exemple 18, peuvent donc être utilisés en thérapie contre les cancers du pancréas.

### Expérimentation 6 : Utilisation des anticorps monoclonaux dirigés contre le domaine C-terminal de la FAPP et/ou de la BSDL dans le diagnostic urinaire des cancers du pancréas

### Protocole opératoire :

Une série d'expériences réalisée à l'aide des techniques de Western-Blot, de chromatographie liquide et de caractérisation par spectrométrie de masse ont permis d'identifier la présence de la BSDL intacte dans les urines de patients sains. Au vu de ces informations, les différents épitopes glycosylés J28, 16D10 portés par le domaine C-terminal de la BSDUFAPP et qui apparaissent lors des adénocarcinomes pancréatiques, pourraient être aussi présents dans les urines des patients atteints de ce cancer.

### Résultats :

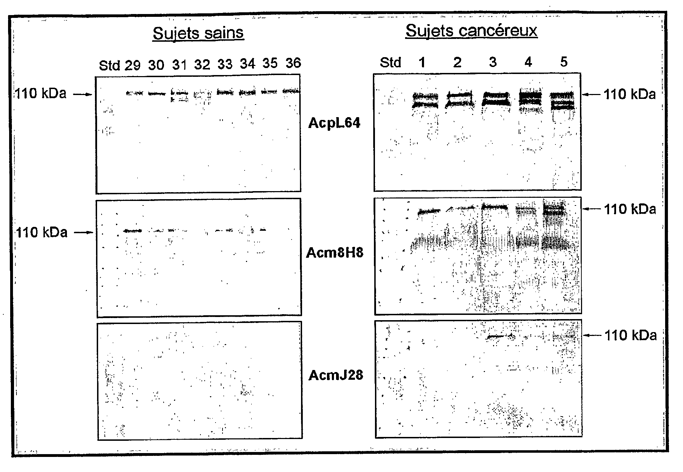

Les premiers résultats représentés dans la Figure 4, obtenus dans une étude préliminaire par les techniques de Westen-blot, montre la présence de l'épitope glycosylé J28 porté par la BSDL/FAPP dans les échantillons urinaires de patients atteints d'adénocarcinomes pancréatiques, alors qu'il est absent dans les urines prélevées chez les personnes saines.

Dans la Figure 4, on peut remarquer qu'à la fois les sujets sains et les sujets cancéreux présentent une ou deux bandes immunoréactives à l'AcpL64 et à l'Acm8H8 (exemple 19) localisées à 110 kDa. En revanche, il existe une différence de réactivité à l'Acm J28, entre les deux groupes. Les sujets cancéreux présentent une bande immunoréactive à l'Acm J28 (110 kDa) alors que ce n'est pas le cas chez les personnes saines.

### Expérimentation 7 : Utilisation des glycopeptides C-terminaux de la BSDL et de la FAPP urinaire en immuno-thérapie cellulaire des cancers du pancréas exocrine

### Protocole opératoire :

Des résultats préliminaires montre la présence de l'épitope glycosylé J28 porté par la BSDL/FAPP dans les échantillons urinaires de patients atteints d'adénocarcinomes pancréatiques testés à l'heure actuelle alors qu'il est absent dans les urines prélevées chez les personnes saines. La présence d'un tel marqueur tumoral dans les urines, qui peuvent être obtenues en grande quantité sans l'emploi de techniques invasives, peut alors servir de source antigénique pour développer différents protocoles d'immunothérapies actives comme décrit par Ramanathan et coll, 2004, Cancer Immunol Immunoth, 54 :254-64 ; O'Neill et coll, 2004, Blood, 104 :2235-46 ; Vlad et coll, 2002, J Exp Med, 2002, 196 : 1435-46 ; Schmidt et coll, Cancer Res, 2003, 63 : 8962-67. Une de ces voies est le développement de protocoles d'immunothérapie cellulaire autologue (comme décrit par Yamanaka et coll Br J Cancer, 2003, 89 :1172-9 ; Svane et coll Cancer Immunol Immunother. 2004, 53 :633-41 ; Yu et coll, Cancer Res. 2004, 64 :4973-9) en utilisant les cellules dendritiques des patients qui auront subi une résection de la tumeur pancréatique. Les cellules dendritiques du patient seront alors chargées *ex vivo* par ces propres antigènes, c'est à dire par le(s) glycopeptide(s) C-terminal de la BSDL/FAPP portant le(s) glycotope(s) J28 (et peut être aussi le glycotope 16D10) obtenus à partir de ces urines, avant de les lui réinjecter pour promouvoir une réponse immunitaire contre les foyers tumoraux résiduels qui expriment ces même antigènes.

Cette stratégie d'activation des cellules dendritiques humaines, actuellement en cours d'évaluation en utilisant comme antigène tumoral, le glycopeptide C-terminal de la FAPP recombinant portant l'épitope glycosylé J28 (exemple 10), peut être aussi réalisée en utilisant le glycopeptide C-terminal de la BSDUFAPP urinaire portant ces épitope glycosylés. Les protocoles de purification (différentes étapes de centrifugations, d'ultrafiltrations et de concentrations, associés à des étapes de chromatographies liquides sur colonnes d'exclusions et d'affinités) élaborés pour purifier et caractériser la BSDL dans les urines de personnes saines sont utilisés pour purifier la BSDL/FAPP qui porte le glycotope J28 (et également celui de 16D10) à partir d'échantillons urinaires de patients atteints d'adénocarcinomes pancréatiques. Le protocole est le suivant : Les urines natives sont centrifugées pour éliminer les débris cellulaires puis concentrées et partiellement dessalées par ultrafiltration sur centripreps® YM-10. Elles sont ensuite totalement dessalées grâce à une colonne de Sephadex® G-25. Les fractions récupérées sont lyophilisées puis reprises dans du tampon d'élution Tris-HCl 10 mM, pH=7,8, NaCl 400 mM, et déposées sur gel Sephacryl™ S-200 permettant la séparation des protéines en fonction de leur masse moléculaire. Les fractions contenant la BSDL/FAPP qui porte le glycotope J28 (et/ou le glycotype 16D10) sont récupérées, dialysées et lyophilisées. Les lyophilisats obtenus après le tamisage moléculaire sont réhydratés dans du PBS, et la BSDL/FAPP qui porte le glycotope J28 (et peut être aussi le glycotope 16010test purifiée soit par chromatographie d'immunoaffinité sur colonne d'Agarose-Acm J28 soit sur une colonne d'héparine Sépharose. La BSDL/FAPP purifiée qui porte le glycotope J28 (et/ou le glycotype 16D10) sera fragmenté par la trypsine ou le bromure de cyanogène pour obtenir le glycopeptide C-terminal de la BSDL/FAPP portant ces glycotopes, comme décrit par ailleurs pour isoler le domaine C-terminal de la BSDUFAPP (Mas et coll. 1997, Glycobiology, 7 : 745-752). Les cellules dendritiques de patients atteints d'adénocarcinomes pancréatiques seront cultivées en présence de ce glycopeptide porteur du glycotope J28 (et/ou le glycotype 16D10) purifié à partir de ces propres urines. La présence des glycopeptides à la surface des cellules dendritiques matures pulsées sera vérifiée en microscopie confocale avant de les lui réinjecter pour déclencher une réponse anti-tumorale.

## Revendications

1. Anticorps monoclonal sélectionné parmi l'anticorps monoclonal 16D10 produit par l'hybridome déposé à la Collection Nationale de Culture de Microorganismes (CNCM) à Paris le 16 mars 2004 sous le N° I-3188, un fragment de celui-ci, un anticorps chimérique, humanisé ou simple chaîne dérivé de celui-ci qui conservent la même spécificité que l'anticorps, et un anticorps qui entre en compétition avec l'anticorps monoclonal 16D10 pour toute molécule BSDL et/ou FAPP à laquelle ledit anticorps monoclonal 16D10 se lie spécifiquement.

2. Anticorps selon la revendication 1, dans lequel ledit anticorps est humanisé, chimérique ou humain.

3. Anticorps selon la revendication 1 ou 2, dans lequel l'anticorps est de type IgG.

4. Anticorps selon la revendication 1 ou 2, dans lequel l'anticorps est un anticorps simple chaine.

5. Utilisation d'un anticorps selon l'une des revendications 1 à 4 pour la préparation d'une composition diagnostic utile pour la détection d'un cancer du pancréas ou d'un diabète *in vivo* ou *in vitro.*

6. Utilisation selon la revendication 5, dans laquelle la pathologie pancréatique est le cancer du pancréas.

7. Méthode de détection *in vitro* d'un sujet souffrant d'un cancer du pancréas ou d'un diabète, comprenant la mise en contact d'un échantillon biologique du sujet avec un anticorps selon l'une des revendications 1 à 4 et la détection de la formation de complexes immunologiques résultant de la réaction immunologique entre ledit anticorps et ledit échantillon biologique.

8. Méthode selon la revendication 7, dans laquelle ledit échantillon biologique est un prélèvement de tissu pancréatique.

9. Méthode selon la revendication 7, dans laquelle ledit échantillon biologique est un fluide biologique sélectionné de préférence parmi le suc pancréatique, le sérum et l'urine.

10. Méthode selon l'une des revendications 7 à 9, dans laquelle la méthode permet la détection d'un sujet souffrant d'un cancer du pancréas.

11. Kit de diagnostic d'une pathologie pancréatique, comprenant un anticorps selon l'une des revendications 1 à 4, et éventuellement des moyens pour détecter le conjugué immunologique résultant de la réaction immunologique entre l'échantillon biologique et ledit anticorps.

12. Méthode de détection *in vitro* d'un sujet souffrant d'un cancer du pancréas ou d'un diabète, comprenant la mise en contact de l'urine du sujet avec un anticorps capable de reconnaître spécifiquement un glycopeptide comprenant 1 à 40 répétitions de la séquence peptidique décrite dans SEQ ID No 14 et glycosylé par une ou plusieurs enzymes à activité transférase d'oses sélectionnées parmi le groupe constitué de la Core 2 β(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède une activité α(1-3) fucosyltransférase, et la détection de la formation de complexes immunologiques résultant de la réaction immunologique entre ledit anticorps et ladite urine.

13. Méthode selon la revendication 12, dans laquelle ledit anticorps est l'anticorps J28, un fragment de celui-ci, un anticorps chimérique, humanisé ou simple chaîne dérivé de celui-ci qui conservent la même spécificité que l'anticorps, ou un anticorps qui entre en compétition avec l'anticorps monoclonal J28 pour toute molécule BSDL et/ou FAPP à laquelle ledit anticorps monoclonal J28 se lie spécifiquement.

14. Méthode selon la revendication 12, dans laquelle ledit anticorps est l'anticorps 16D10 produit par l'hybridome déposé à la Collection Nationale de Culture de Microorganismes (CNCM) à Paris le 16 mars 2004 sous le N° 1-3188, un fragment de celui-ci, un anticorps chimérique, humanisé ou simple chaîne dérivé de celui-ci qui conservent la même spécificité que l'anticorps, ou un anticorps qui entre en compétition avec l'anticorps monoclonal 16D10 pour toute molécule BSDL et/ou FAPP à laquelle ledit anticorps monoclonal 16D10 se lie spécifiquement.

15. Méthode selon l'une des revendications 12 à 14, dans laquelle la méthode permet la détection d'un sujet souffrant d'un cancer du pancréas.

16. Composition pharmaceutique ou vaccinale comprenant un glycopeptide comprenant 1 à 40 répétitions de la séquence peptidique décrite dans SEQ ID No 14 et glycosylé par une ou plusieurs enzymes à activité transférase d'oses sélectionnées parmi le groupe constitué de la Core 2 β(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède une activité α(1-3) fucosyltransférase, pour une utilisation dans le traitement préventif ou curatif d'un cancer du pancréas.

17. Composition pharmaceutique ou vaccinale selon la revendication 16, dans laquelle ledit glycopeptide est glycosylé par les enzymes C2GnT et FUT3.

18. Composition pharmaceutique ou vaccinale selon la revendication 16, dans laquelle ledit glycopeptide est glycosylé par les enzymes C2GnT et FUT7.

19. Composition pharmaceutique ou vaccinale selon la revendication 16, dans laquelle ledit glycopeptide est glycosylé par les enzymes C2GnT, FUT3 et FUT7.

20. Composition pharmaceutique ou vaccinale selon l'une quelconque des revendications 16 à 19, dans laquelle ledit glycopeptide est en outre glycosylé par l'α(1-3)galactosyltransférase (GT).

21. Composition pharmaceutique ou vaccinale selon l'une quelconque des revendications 16 à 20, dans laquelle ledit glycopeptide comprend entre 1 et 15 desdites répétitions.

22. Composition pharmaceutique ou vaccinale selon l'une quelconque des revendications 16 à 21, dans laquelle ledit glycopeptide est recombinant.

23. Composition pharmaceutique ou vaccinale selon l'une quelconque des revendications 16 à 22, dans laquelle ledit glycopeptide est purifié à partir d'un fluide biologique.

24. Composition pharmaceutique ou vaccinale selon la revendication 23, dans laquelle ledit fluide biologique est l'urine.

25. Composition pharmaceutique ou vaccinale selon la revendication 24, dans laquelle ladite urine provient d'un sujet souffrant d'un cancer du pancréas.

26. Composition pharmaceutique ou vaccinale selon l'une quelconque des revendications 16 à 25, dans laquelle ledit glycopeptide est chargé sur des cellules présentatrices d'antigène, de préférence des cellules dendritiques.

27. Composition pharmaceutique comprenant un anticorps capable de reconnaître spécifiquement un glycopeptide comprenant 1 à 40 répétitions de la séquence peptidique décrite dans SEQ ID No 14 et glycosylé par une ou plusieurs enzymes à activité transférase sélectionnées parmi le groupe constitué de la Core 2 β(1-6) N-acétylglucosaminyltransférase (C2GnT), la fucosyltransférase FUT3 qui possède une activité α(1-3) et α(1-4) fucosyltransférase, ou la fucosyltransférase FUT7 qui possède une activité α(1-3) fucosyltransférase, pour une utilisation dans le traitement préventif ou curatif d'un cancer du pancréas.

28. Composition pharmaceutique selon la revendication 27, dans laquelle ledit glycopeptide est glycosylé par les enzymes C2GnT et FUT3.

29. Composition pharmaceutique selon la revendication 27, dans laquelle ledit glycopeptide est glycosylé par les enzymes C2GnT et FUT7.

30. Composition pharmaceutique selon la revendication 27, dans laquelle ledit glycopeptide est glycosylé par les enzymes C2GnT, FUT3 et FUT7.

31. Composition pharmaceutique selon l'une des revendications 27 à 30, dans laquelle ledit glycopeptide comprend entre 1 et 15 desdites répétitions.

32. Composition pharmaceutique selon l'une des revendications 27 à 31, dans laquelle ledit anticorps est l'anticorps J28, un fragment de celui-ci, un anticorps chimérique, humanisé ou simple chaîne un dérivé de celui-ci qui conservent la même spécificité que l'anticorps, ou un anticorps qui entre en compétition avec l'anticorps monoclonal J28 pour toute molécule BSDL et/ou FAPP à laquelle ledit anticorps monoclonal J28 se lie spécifiquement.

33. Composition pharmaceutique selon l'une des revendications 27 à 31, dans laquelle ledit anticorps est l'anticorps 16D10 produit par l'hybridome déposé à la Collection Nationale de Culture de Microorganismes (CNCM) à Paris le 16 mars 2004 sous le N° I-3188, un fragment de celui-ci, un anticorps chimérique, humanisé ou simple chaîne dérivé de celui-ci qui conservent la même spécificité que l'anticorps, ou un anticorps qui entre en compétition avec l'anticorps monoclonal 16D10 pour toute molécule BSDL et/ou FAPP à laquelle ledit anticorps monoclonal 16D10 se lie spécifiquement.

## Patentansprüche

1. Monoklonaler Antikörper, ausgewählt aus dem monoklonalen 16D10-Antikörper, der von dem bei der Collection Nationale de Culture de Microorganismes (CNCM) in Paris am 16. März 2004 unter der Nr. I-3188 hinterlegten Hybridom produziert wird, einem Fragment davon, einem davon abstammenden chimären, humanisierten oder einzelkettigen Antikörper, welche die gleiche Spezifität beibehalten wie der Antikörper, und einem Antikörper, der mit dem monoklonalen 16D10-Antikörper um das BSDL- und/oder FAPP-Molekül konkurriert, an das besagter monoklonaler 16D10-Antikörper spezifisch bindet.

2. Antikörper gemäß Anspruch 1, wobei besagter Antikörper ein humanisierter, chimärer oder humaner Antikörper ist.

3. Antikörper gemäß Anspruch 1 oder 2, wobei besagter Antikörper vom Typ IgG ist.

4. Antikörper gemäß Anspruch 1 oder 2, wobei der Antikörper ein einzelkettiger Antikörper ist.

5. Verwendung eines Antikörpers gemäß einem der Ansprüche 1 bis 4 zur Herstellung einer diagnostischen Zusammensetzung, die zur Detektion von Pankreaskrebs oder von Diabetes *in vivo* oder *in vitro* zweckdienlich ist.

6. Verwendung gemäß Anspruch 5, wobei die Pankreaserkrankung Pankreaskrebs ist.

7. *In vitro* Verfahren zur Detektion einer Person, die an Pankreaskrebs oder an Diabetes leidet, umfassend das Inkontaktbringen einer biologischen Probe der Person mit einem Antikörper gemäß einem der Ansprüche 1 bis 4 und die Detektion der Bildung von Immunkomplexen, die aus der Immunreaktion zwischen besagtem Antikörper und besagter biologischer Probe resultieren.

8. Verfahren gemäß Anspruch 7, wobei besagte biologische Probe eine Pankreasgewebsprobe ist.

9. Verfahren gemäß Anspruch 7, wobei besagte biologische Probe eine biologische Flüssigkeit ist, vorzugsweise ausgewählt aus Pankreassaft, Serum und Urin.

10. Verfahren gemäß einem der Ansprüche 7 bis 9, wobei das Verfahren die Detektion eines Patienten erlaubt, der an Pankreaskrebs leidet.

11. Kit zur Diagnose einer Pankreaserkrankung, umfassend einen Antikörper gemäß einem der Ansprüche 1 bis 4 und eventuell Mittel zur Detektion des Immunkonjugats, das aus der Immunreaktion zwischen der biologischen Probe und besagtem Antikörper resultiert.

12. *In vitro* Verfahren zur Detektion einer Person, die an Pankreaskrebs oder an Diabetes leidet, umfassend das Inkontaktbringen von Urin der Person mit einem Antikörper, der in der Lage ist, ein Glycopeptid spezifisch zu erkennen, umfassend 1 bis 40 Wiederholungen der Peptidsequenz, die in SEQ ID NR: 14 beschrieben ist und von einem oder mehreren Enzymen mit Zucker-Transferaseaktivität glycosyliert ist, die aus der Gruppe ausgewählt sind, bestehend aus der Core 2 β(1-6) N-Acetylglucosaminyltransferase (C2GnT), der Fucosyltransferase FUT3, die eine α(1-3) und α(1-4) Fucosyltransferaseaktivität besitzt, oder der Fucosyltransferase FUT7, die eine α(1-3) Fucosyltransferaseaktivität besitzt, und die Detektion der Bildung von Immunkomplexen, die aus der Immunreaktion zwischen besagtem Antikörper und besagtem Urin resultieren.

13. Verfahren gemäß Anspruch 12, wobei besagter Antikörper der J28-Antikörper, ein Fragment davon, ein davon abstammender einzelkettiger, humanisierter oder chimärer Antikörper, welche die gleiche Spezifität beibehalten wie der Antikörper, oder ein Antikörper ist, der mit dem monoklonalen J28-Antikörper um das BSDL- und/oder FAPP-Molekül konkurriert, an das besagter monoklonaler J28-Antikörper spezifisch bindet.

14. Verfahren gemäß Anspruch 12, wobei besagter Antikörper der 16D10-Antikörper, der von dem bei der Collection Nationale de Culture de Microorganismes (CNCM) in Paris am 16. März 2004 unter der Nr. I-3188 hinterlegten Hybridom produziert wird, ein Fragment davon, ein davon abstammender chimärer, humanisierter oder einzelkettiger Antikörper, welche die gleiche Spezifität beibehalten wie der Antikörper, oder ein Antikörper ist, der mit dem monoklonalen 16D10-Antikörper um das BSDL- und/oder FAPP-Molekül konkurriert, an das besagter monoklonaler 16D10-Antikörper spezifisch bindet.

15. Verfahren gemäß einem der Ansprüche 12 bis 14, wobei das Verfahren die Detektion einer Person erlaubt, die an Pankreaskrebs leidet.

16. Pharmazeutische Zusammensetzung oder Impfstoffzusammensetzung, umfassend ein Glycopeptid, umfassend 1 bis 40 Wiederholungen der Peptidsequenz, die in SEQ ID NR: 14 beschrieben ist und von einem oder mehreren Enzymen mit Zucker-Transferaseaktivität glycosyliert ist, die aus der Gruppe ausgewählt sind, bestehend aus der Core 2 β(1-6) N-Acetylglucosaminyltransferase (C2GnT), der Fucosyltransferase FUT3, die eine α(1-3) und α(1-4) Fucosyltransferaseaktivität besitzt, oder der Fucosyltransferase FUT7, die eine α(1-3) Fucosyltransferaseaktivität besitzt, zur Verwendung in der präventiven oder kurativen Behandlung eines Pankreaskrebses.

17. Pharmazeutische Zusammensetzung oder Impfstoffzusammensetzung gemäß Anspruch 16, wobei besagtes Glycopeptid von den Enzymen C2GnT und FUT3 glycosyliert ist.

18. Pharmazeutische Zusammensetzung oder Impfstoffzusammensetzung gemäß Anspruch 16, wobei besagtes Glycopeptid von den Enzymen C2GnT und FUT7 glycosyliert ist.

19. Pharmazeutische Zusammensetzung oder Impfstoffzusammensetzung gemäß Anspruch 16, wobei besagtes Glycopeptid von den Enzymen C2GnT, FUT3 und FUT7 glycosyliert ist.

20. Pharmazeutische Zusammensetzung oder Impfstoffzusammensetzung gemäß einem der Ansprüche 16 bis 19, wobei besagtes Glycopeptid außerdem von der α(1-3) Galactosyltransferase (GT) glycosyliert ist.

21. Pharmazeutische Zusammensetzung oder Impfstoffzusammensetzung gemäß einem der Ansprüche 16 bis 20, wobei besagtes Glycopeptid zwischen 1 und 15 besagter Wiederholungen umfasst.

22. Pharmazeutische Zusammensetzung oder Impfstoffzusammensetzung gemäß einem der Ansprüche 16 bis 21, wobei besagtes Glycopeptid rekombinant ist.

23. Pharmazeutische Zusammensetzung oder Impfstoffzusammensetzung gemäß einem der Ansprüche 16 bis 22, wobei besagtes Glycopeptid aus einer biologischen Flüssigkeit aufgereinigt ist.

24. Pharmazeutische Zusammensetzung oder Impfstoffzusammensetzung gemäß Anspruch 23, wobei besagte biologische Flüssigkeit Urin ist.

25. Pharmazeutische Zusammensetzung oder Impfstoffzusammensetzung gemäß Anspruch 24, wobei besagter Urin von einer Person stammt, die an Pankreaskrebs leidet.

26. Pharmazeutische Zusammensetzung oder Impfstoffzusammensetzung gemäß einem der Ansprüche 16 bis 25, wobei besagtes Glycopeptid auf Antigen-präsentierenden Zellen, vorzugsweise dendritischen Zellen, geladen wird.

27. Pharmazeutische Zusammensetzung, umfassend einen Antikörper, der in der Lage ist, ein Glycopeptid spezifisch zu erkennen, umfassend 1 bis 40 Wiederholungen der Peptidsequenz, die in SEQ ID NR: 14 beschrieben ist und von einem oder mehreren Enzymen mit Transferaseaktivität glycosyliert ist, die aus der Gruppe ausgewählt sind, bestehend aus der Core 2 β(1-6) N-Acetylglucosaminyltransferase (C2GnT), der Fucosyltransferase FUT3, die eine α(1-3) und α(1-4) Fucosyltransferaseaktivität besitzt, oder der Fucosyltransferase FUT7, die eine α(1-3) Fucosyltransferaseaktivität besitzt, zur Verwendung in der präventiven oder kurativen Behandlung eines Pankreaskrebses.

28. Pharmazeutische Zusammensetzung gemäß Anspruch 27, wobei besagtes Glycopeptid von den Enzymen C2GnT und FUT3 glycosyliert ist.

29. Pharmazeutische Zusammensetzung gemäß Anspruch 27, wobei besagtes Glycopeptid von den Enzymen C2GnT und FUT7 glycosyliert ist.

30. Pharmazeutische Zusammensetzung gemäß Anspruch 27, wobei besagtes Glycopeptid von den Enzymen C2GnT, FUT3 und FUT7 glycosyliert ist.

31. Pharmazeutische Zusammensetzung gemäß einem der Ansprüche 27 bis 30, wobei besagtes Glycopeptid zwischen 1 und 15 besagter Wiederholungen umfasst.

32. Pharmazeutische Zusammensetzung gemäß einem der Ansprüche 27 bis 31, wobei besagter Antikörper der J28-Antikörper, ein Fragment davon, ein davon abstammender einzelkettiger, humanisierter oder chimärer Antikörper, welche die gleiche Spezifität beibehalten wie der Antikörper, oder ein Antikörper ist, der mit dem monoklonalen J28-Antikörper um das BSDL- und/oder FAPP-Molekül konkurriert, an das besagter monoklonaler J28-Antikörper spezifisch bindet.

33. Pharmazeutische Zusammensetzung gemäß einem der Ansprüche 27 bis 31, wobei besagter Antikörper der 16D10-Antikörper, der von dem bei der Collection Nationale de Culture de Microorganismes (CNCM) in Paris am 16. März 2004 unter der Nr. I-3188 hinterlegten Hybridom produziert wird, ein Fragment davon, ein davon abstammender chimärer, humanisierter oder einzelkettiger Antikörper, welche die gleiche Spezifität beibehalten wie der Antikörper, oder ein Antikörper ist, der mit dem monoklonalen 16D10-Antikörper um das BSDL- und/oder FAPP-Molekül konkurriert, an das besagter monoklonaler 16D10-Antikörper spezifisch bindet.

## Claims

1. Monoclonal antibody selected from monoclonal antibody 16D10 produced by the hybridoma deposited with the Collection Nationale de Culture de Microorganismes (CNCM) in Paris on 16 March 2004 under the number I-3188, a fragment thereof, a chimeric, humanized or single chain antibody derived thereof which conserves the same specificity as the antibody, and an antibody which can compete with monoclonal antibody 16D10 for any BSDL and/or FAPP molecule to which said monoclonal antibody 16D10 specifically binds.

2. Antibody according to claim 1, wherein said antibody is humanized, chimeric or human.

3. Antibody according to claim 1 or 2, wherein the antibody is of the IgG type.

4. Antibody according to claim 1 or 2, wherein the antibody is a single chain antibody.

5. Use of an antibody according to any one of claims 1 to 4 for preparing a diagnostic composition which can be used for detecting pancreatic cancer or diabetes, *in vivo* or *in vitro.*

6. Use according to claim 5, wherein the pancreatic pathology is pancreatic cancer.

7. Method of detection *in vitro* of a subject suffering from pancreatic cancer or diabetes, comprising contacting a biological sample from the subject with an antibody according to any one of claims 1 to 4 and detecting the formation of immunological complexes resulting from the immunological reaction between said antibody and said biological sample.

8. Method according to claim 7, wherein said biological sample is a sample of pancreatic tissue.

9. Method according to claim 7, wherein said biological sample is a biological fluid preferably selected from among pancreatic juices, serum and urine.

10. Method according to any one of claims 7 to 9, wherein the method enables the detection of a subject suffering from pancreatic cancer.

11. Kit for diagnosis of a pancreatic pathology, comprising an antibody according to any one of claims 1 to 4, and optionally the means for detecting the immunological complex resulting from the immunological reaction between the biological sample and said antibody.

12. Method of detection *in vitro* of a subject suffering from pancreatic cancer or diabetes, comprising contacting the urine of the subject with an antibody which can specifically recognize a glycopeptide comprising 1 to 40 repetitions of the peptide sequence described in SEQ ID No 14 and glycosylated by one or more enzymes having glycosyltransferase activity selected in the group consisting of Core 2 β(1-6) N-acetylglucosaminyltransferase (C2GnT), fucosyltransferase FUT3 which has α(1-3) and α(1-4) fucosyltransferase activity, or fucosyltransferase FUT7 which has α(1-3) fucosyltransferase activity, and detecting the formation of immunological complexes resulting from the immunological reaction between said antibody and said urine.

13. Method according to claim 12, wherein said antibody is the antibody J28, a fragment thereof, a chimeric, humanized or single chain antibody derived thereof which conserves the same specificity as the antibody, or an antibody which can compete with monoclonal antibody J28 for any BSDL and/or FAPP molecule to which said monoclonal antibody J28 specifically binds.

14. Method according to claim 12, wherein said antibody is the antibody 16D10 produced by the hybridoma deposited with the Collection Nationale de Culture de Microorganismes (CNCM) in Paris on 16 March 2004 under the number I-3188, a fragment thereof, a chimeric, humanized or single chain antibody derived thereof which conserves the same specificity as the antibody, or an antibody which can compete with monoclonal antibody 16D10 for any BSDL and/or FAPP molecule to which said monoclonal antibody 16D10 specifically binds.

15. Method according to any one of claims 12 to 14, wherein the method enables the detection of a subject suffering from pancreatic cancer.

16. Pharmaceutical or vaccine composition comprising a glycopeptide comprising 1 to 40 repetitions of the peptide sequence described in SEQ ID No 14 and glycosylated by one or more enzymes having glycosyltransferase activity selected in the group consisting of Core 2 β(1-6) N-acetylglucosaminyltransferase (C2GnT), fucosyltransferase FUT3 which has α(1-3) and α(1-4) fucosyltransferase activity, or fucosyltransferase FUT7 which has α(1-3) fucosyltransferase activity, for its use in the preventive or curative treatment of pancreatic cancer.

17. Pharmaceutical or vaccine composition according to claim 16, wherein said glycopeptide is glycosylated by the enzymes C2GnT and FUT3.

18. Pharmaceutical or vaccine composition according to claim 16, wherein said glycopeptide is glycosylated by the enzymes C2GnT and FUT7.

19. Pharmaceutical or vaccine composition according to claim 16, wherein said glycopeptide is glycosylated by the enzymes C2GnT, FUT3 and FUT7.

20. Pharmaceutical or vaccine composition according to any one of claims 16 to 19, wherein said glycopeptide is further glycosylated by α(1-3)galactosyltransferase (GT).

21. Pharmaceutical or vaccine composition according to any one of claims 16 to 20, wherein said glycopeptide comprises between 1 and 15 of said repetitions.

22. Pharmaceutical or vaccine composition according to any one of claims 16 to 21, wherein said glycopeptide is recombinant.

23. Pharmaceutical or vaccine composition according to any one of claims 16 to 22, wherein said glycopeptide is purified from a biological fluid.

24. Pharmaceutical or vaccine composition according to claim 23, wherein said biological fluid is urine.

25. Pharmaceutical or vaccine composition according to claim 24, wherein said urine originates from a subject suffering from pancreatic cancer.

26. Pharmaceutical or vaccine composition according to any one of claims 16 to 25, wherein said glycopeptide is loaded on antigen-presenting cells, preferably dendritic cells.

27. Pharmaceutical composition comprising an antibody which can specifically recognize a glycopeptide comprising 1 to 40 repetitions of the peptide sequence described in SEQ ID No 14 and glycosylated by one or more enzymes having transferase activity selected in the group consisting of Core 2 β(1-6) N-acetylglucosaminyltransferase (C2GnT), fucosyltransferase FUT3 which has α(1-3) and α(1-4) fucosyltransferase activity, or fucosyltransferase FUT7 which has α(1-3) fucosyltransferase activity, for its use in the preventive or curative treatment of pancreatic cancer.

28. Pharmaceutical composition according to claim 27, wherein said glycopeptide is glycosylated by the enzymes C2GnT and FUT3.

29. Pharmaceutical composition according to claim 27, wherein said glycopeptide is glycosylated by the enzymes C2GnT and FUT7.

30. Pharmaceutical composition according to claim 27, wherein said glycopeptide is glycosylated by the enzymes C2GnT, FUT3 and FUT7.

31. Pharmaceutical composition according to any one of claims 27 to 30, wherein said glycopeptide comprises between 1 and 15 of said repetitions.

32. Pharmaceutical composition according to any one of claims 27 to 31, wherein said antibody is the antibody J28, a fragment thereof, a chimeric, humanized or single chain antibody derived thereof which conserves the same specificity as the antibody, or an antibody which can compete with monoclonal antibody J28 for any BSDL and/or FAPP molecule to which said monoclonal antibody J28 specifically binds.

33. Pharmaceutical composition according to any one of claims 27 to 31, wherein said antibody is the antibody 16D10 produced by the hybridoma deposited with the Collection Nationale de Culture de Microorganismes (CNCM) in Paris on 16 March 2004 under the number I-3188, a fragment thereof, a chimeric, humanized or single chain antibody derived thereof which conserves the same specificity as the antibody, or an antibody which can compete with monoclonal antibody 16D10 for any BSDL and/or FAPP molecule to which said monoclonal antibody 16D10 specifically binds.
